(19) Europäisches Patentamt
European Patent Office
Office européen des brevets

(11) EP 1 445 260 A1

(12) **EUROPÄISCHE PATENTANMELDUNG**

(43) Veröffentlichungstag:
**11.08.2004 Patentblatt 2004/33**

(51) Int Cl.[7]: **C07K 1/04**, B01J 19/00, C07B 61/00

(21) Anmeldenummer: **03002404.6**

(22) Anmeldetag: **04.02.2003**

(84) Benannte Vertragsstaaten:
**AT BE BG CH CY CZ DE DK EE ES FI FR GB GR HU IE IT LI LU MC NL PT SE SI SK TR**
Benannte Erstreckungsstaaten:
**AL LT LV MK RO**

(71) Anmelder: **Jerini AG**
**10115 Berlin (DE)**

(72) Erfinder:
- **Schnatbaum, Karsten, Dr.**
  **10245 Berlin (DE)**
- **Reimer, Ulf, Dr.**
  **10405 Berlin (DE)**
- **Scharn, Dirk, Dr.**
  **13507 Berlin (DE)**
- **Schutkowski, Mike, Dr.**
  **06268 Ziegelroda (DE)**

(74) Vertreter: **Bohmann, Armin K., Dr.**
**Bohmann & Loosen,**
**Anwaltssozietät,**
**Sonnenstrasse 8**
**80331 München (DE)**

(54) **Verfahren zur Immobilisierung von chemischen Verbindungen an Festphasen**

(57) Die vorliegende Erfindung betrifft ein Verfahren zur Ausbildung einer chemischen Bindung zwischen einer ersten, auf einer insbesondere planaren Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung und einem Kupplungsreagenz und optional einem Zuschlagstoff sowie optional mit einer Base kontaktiert und umgesetzt wird, dadurch gekennzeichnet, dass die Schritte

- Kontaktieren der Oberfläche mit dem Kupplungsreagenz und
- Kontaktieren der Oberfläche mit der zu immobilisierenden Verbindung

getrennt, insbesondere zeitlich voneinander getrennt erfolgen.

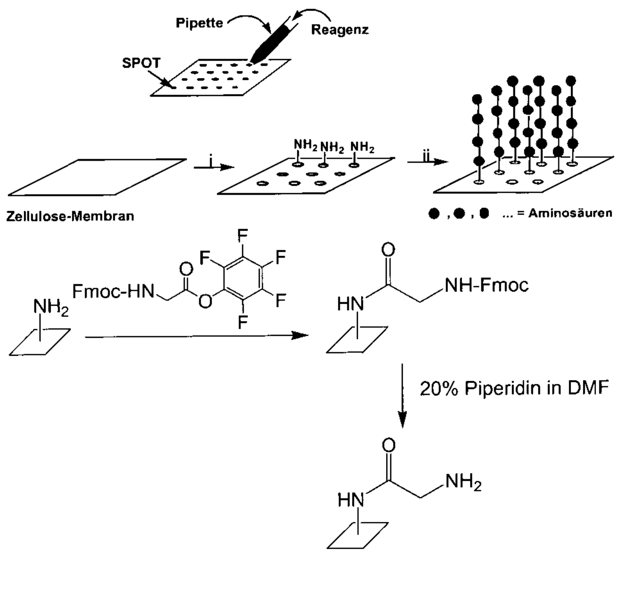


Fig. 10

EP 1 445 260 A1

Printed by Jouve, 75001 PARIS (FR)

## Beschreibung

Die vorliegende Erfindung betrifft Verfahren zur Ausbildung einer chemischen Bindung zwischen einer ersten auf einer Oberfläche immobilisierten funktionellen Gruppe und einer zweiten funktionellen Gruppe, die Teil einer zu immobilisierenden Verbindung ist.

Proteine spielen eine wichtige Rolle in nahezu allen biologischen Prozessen, wie beispielsweise enzymatischer Katalyse, Transport und Speicherung, mechanische Stützfunktion, Immunschutz, Nervenreizleitung und Kontrolle von Wachstum und Differenzierung. Dabei sind Proteine aus einer oder mehreren linearen Polypeptidketten aufgebaut. Ihre Faltung und dreidimensionale Struktur ist bestimmt durch die Primärstruktur oder Aminosäuresequenz der Ketten. Die Vielfalt an Proteinstrukturen in der Natur wird erzeugt durch Kombination von zwanzig Aminosäurebausteinen, die im Rahmen der Proteinbiosynthese zusammengeführt werden. Kurze Partialsequenzen (Oligopeptide) können auch durch chemische Synthese hergestellt werden und im Rahmen verschiedenster Anwendungen verwendet werden, was unter anderem darin begründet ist, dass diese zum gewissen Teil die lokale Struktur und Funktion dieser Sequenz im Gesamtproteinverband imitieren können. Synthetische Oligopeptide sind somit unter anderem ein wichtiges Hilfsmittel bei der Strukturfunktionsanalyse von Proteinen. Aufgrund der Größe und Komplexität der Proteine werden für systematische Studien sehr viele verschiedene Peptide benötigt.

Biologische Testreaktionen wie enzymatische Umsetzung oder Antikörperbindung sind sehr empfindlich, so dass geringe Mengen (µg bis ng) Peptidsubstrate dafür benötigt werden. Für eine schnelle, einfache Durchführung solcher Tests ist es vorteilhaft, wenn das Peptidsubstrat an einem festen Trägermaterial immobilisiert ist und leicht aus der Reaktionslösung entfernt werden kann. Die Reaktion mit dem Peptid kann dann entweder in der verbleibenden Reaktionslösung oder durch nachfolgende Analyse des trägergebundenen Materials quantitativ vermessen werden.

Der Umfang, mit dem systematische Untersuchungen mit Peptiden oder ähnlichen Verbindungen durchgeführt werden können, hängt im wesentlichen von der Schnelligkeit der Synthese und der Zahl der gleichzeitig synthetisierbaren Verbindungen ab.

Zur hochparallelen, schnellen Synthese kleiner Mengen von Peptiden oder anderer kleiner Moleküle [N. Heine et al., *Tetrahedron Letters* **2001,** *42*, 227-230] wird derzeit insbesondere die sogenannte SPOT-Synthese verwendet [R. Frank, *Tetrahedron* **1992,** *48*, 9217-9232], [A.Kramer et al., *J. Pept. Res.* **1999,** *54*, 319-327], [H. Wenschuh et al. in *Combinatorial Chemistry*, H. Fenniri, Ed., Oxford University Press, **2000,** S. 95-116]. Dabei werden Aliquots einer Lösung einer zu kuppelnden Verbindung wie einer Aminosäure, die in Form eines aktivierten Derivats vorliegt, auf einem Flachmaterial aufgetragen. Auf dem von der Lösung benetzten Bereich des Flachmaterials findet dann eine chemische Reaktion zwischen der zu kuppelnden Verbindung und einer auf dem Flachmaterial immobilisierten funktionellen Gruppe statt. Nach Beendigung der Reaktion werden überschüssige Reagenzien und lösliche Reaktionsprodukte weggewaschen. Da in der Regel sehr viele verschiedene Reaktionen auf räumlich getrennten Bereichen eines polymeren Flachmaterials durchgeführt werden, lässt sich in einem Schritt eine Vielzahl paralleler Reaktionen gleichzeitig durchführen.

Bezüglich des aktivierten Derivats einer zu kuppelnden Verbindung sind im Stand der Technik zwei unterschiedliche Verfahren beschrieben worden. Im ersten Verfahren verwendet man vorher synthetisierte und isolierte Aktivderivate, die in einem geeigneten Lösungsmittel aufgelöst und dann für die geplante Kupplungsreaktion eingesetzt werden. Dabei sind insbesondere Pentafluorphenylester von Aminosäuren [R. Frank, *Tetrahedron* **1992,** *48*, 9217-9232] [U. Reineke et al., *Curr. Topics Microbiol. Immunol.* **1999,** *243*, 23-36], aber auch vorsynthetisierte Aminosäure-Dhbt-Ester [A. Kramer et al., *Methods: A Companion to Methods in Enzymology,* **1994,** *6*, 388-395], Fmoc-Aminosäure-N-Carboxyanhydride [R. Frank, *Tetrahedron* **1992,** *48*, 9217-9232] und Carbonsäure-N-hydroxysuccinimid-Ester [K. Licha et al., *Tetrahedron Lett.* **2000,** *41*, 1711-1715] sind verwendet worden.

Bei dem zweiten Verfahren wird die nichtaktivierte Form der zu kuppelnden Verbindung durch ein geeignetes Reagenz in einer Lösung in die entsprechende aktivierte Form überführt und diese ohne Isolierung des Aktivderivats *in situ* für die gewünschte Kupplungsreaktion verwendet. Beispiele für Reagenzien, die zur *in situ*-Synthese aktivierter Derivate verwendet wurden, sind TBTU mit der Base DIPEA [R. Volkmer-Engert et al., *Tetrahedron Lett.* **1997 ,** *38* , 1029-1032], PyBOP mit der Base DIPEA [A. Kramer et al., *Methods: A Companion to Methods in Enzymology,* **1994,** *6*, 388-395], DIC mit der Base NMI [U. Hoffmüller et al., *Angew. Chem.* **1999,** *38*, 2000-2004], DIC mit dem Zuschlagstoff HOBt [R. Frank et al., DE4027675, Patent, **1990**] [F. Molina et al., *Pept. Res.* **1996,** *9*, 151-155] [M. Ho et al., *J. Chin. Chem. Soc.* **1999,** *46*, 735-743] [Bracci et al., *Biochemistry* **2001,** *40,* 6611-6619] und DIC mit dem Zuschlagstoff HOAt [Matysiak et al., *Bio Technigues* **2001,** *31*, 896-904].

Trotz der unbestreitbaren Vorteile, die mit der SPOT-Synthese verbunden sind, weist diese jedoch auch einige Limitierungen, insbesondere bei der parallelen Synthese mehrere Peptide an fester Phase, auf.

Diese Nachteile liegen im wesentlichen darin begründet, dass Lösungen von aktivierten Verbindungen eine geringe Stabilität aufweisen. Besonders die häufig verwendeten *in situ*hergestellten Aktivderivate, wie z.B. OBt- und OAt-Ester sind dabei empfindlich. OBt- und OAt-Ester zerfallen in Lösung relativ schnell [Albericio et al, *Methods Enzymol.* **1997,** *289,* 104-126] und viele Versuche zur Isolierung scheiterten deshalb [J. Coste et al., *J. Org. Chem.*

**1994,** *59*, 2437-2446] [J. Coste et al., *Tetrahedron Lett.* **1995,** *36*, 4253-4256].

Bei der Verwendung von vorsynthetisierten und isolierten Aktivderivaten sind die Stabilitätsprobleme in der Regel nicht so gravierend wie bei der *in situ*-Aktivierung. Trotzdem treten auch hier häufig Probleme auf, wie z.B. bei dem Arg-Derivat Fmoc-Arg(Pmc)-OPfp, das in Lösung zum entsprechenden β-Lactam abreagiert und deshalb bei der SPOT-Synthese vieler Peptide jeweils frisch in NMP angelöst werden muss [R. Frank, *Tetrahedron* **1992,** *48*, 9217-9232]. Weitere Nachteile von isolierten Aktivderivaten sind die Zeit- und Kostenintensität der Herstellung und Reinigung, wobei manchmal sogar nur verunreinigte Produkte erhalten werden können (z.B. wurde Fmoc-His(1-Trt) -OPfp lediglich verunreinigt mit HOPfp und Cyclohexan erhalten [P. Sieber et al., *Tetrahedron Lett.* **1987,** *28,* 6031-6034]). Außerdem sind OPfp-Ester und viele andere vorsynthetisierte und isolierte Aktivderivate relativ schwache Acylierungsmittel [Albericio et al, *Methods Enzymol.* **1997,** *289*, 104-126], was besonders bei schweren Peptidkupplungen zu schlechten Produktreinheiten führt.

Auch bei der vollautomatischen SPOT-Synthese weisen die nach dem Stand der Technik verwendeten voraktivierten Aminosäure-Lösungen große Nachteile auf. Bei diesem Verfahren ist es zu einem durchgängigen Betrieb über Nacht außerhalb der üblichen Arbeitszeiten unbedingt notwendig, dass alle aktivierten Derivate der zu kuppelnden Verbindungen in Lösung für mindestens 12 Stunden stabil sind, vorausgesetzt, man möchte auf eine Zeit- und Material-intensive Voraktivierung vor jedem Kupplungscyclus verzichten. Das ist aber weder bei HBTU/DIPEA-aktivierten Aminosäuren noch beim Fmoc-Arg(Pmc)-OPfp-Ester der Fall. Außerdem fallen z.B. bei OPfp-Estern von Fmoc-Aminosäuren nach längerem Stehen an der Luft häufig kristalline Niederschläge aus, was ein effektives Pipettieren dieser Lösungen verhindert. Eine effektive vollautomatische Synthese ist deshalb mit den nach dem Stand der Technik vorhandenen Methoden nicht möglich.

Ein weiterer Nachteil der Verwendung bereits in Lösung aktivierten Verbindungen ist die Möglichkeit, dass besonders reaktive Aktivderivate bei längerem Stehenlassen zu weniger aktiven Derivaten abreagieren, was zu geringeren Ausbeuten bei der Kupplungsreaktion führen kann.

Der vorliegenden Erfindung lag somit die Aufgabe zugrunde, ein Verfahren zur Ausbildung einer chemischen Bindung, insbesondere ein Verfahren zur Herstellung von Polypeptiden, bereitzustellen, welches die Nachteile der im Stand der Technik bekannten Verfahren überwindet. Dabei war es insbesondere Aufgabe der vorliegenden Erfindung, ein Verfahren bereitzustellen, welches eine vollautomatische Synthese von Peptiden auf einer insbesondere planaren Oberfläche erlaubt.

Erfindungsgemäß wird die Aufgabe in einem ersten Aspekt gelöst durch ein Verfahren zur Ausbildung einer chemischen Bindung zwischen einer ersten, auf einer insbesondere planaren Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung und einem Kupplungsreagenz und optional einem Zuschlagstoff sowie optional mit einer Base kontaktiert und umgesetzt wird, dadurch gekennzeichnet, dass

die Schritte **-**Kontaktieren der Oberfläche mit dem Kupplungsreagenz und
**-**Kontaktieren der Oberfläche mit der zu immobilisierenden Verbindung getrennt, insbesondere zeitlich voneinander getrennt erfolgen.

In einer Ausführungsform ist vorgesehen, dass dem Kupplungsreagenz und/oder der Verbindung mindestens ein Zuschlagstoff zugesetzt wird.

In einer Ausführungsform ist vorgesehen, dass der Verbindung und/oder dem Kupplungsreagenz die Base zugesetzt ist.

In einer weiteren Ausführungsform ist vorgesehen, dass die Base dargestellt wird durch die immobilisierte funktionelle Gruppe.

In einer weiteren Ausführungsform ist vorgesehen, dass das erfindungsgemäße Verfahren weiter den Schritt umfasst:

Kontaktieren der Oberfläche mit einem Zuschlagstoff, wobei dieser Schritt getrennt, insbesondere zeitlich getrennt von den Schritten Kontaktieren der Oberfläche mit dem Kopplungsreagenz und Kontaktieren der Oberfläche mit der zu immobilisierenden Verbindung erfolgt.

In einer noch weiteren Ausführungsform ist vorgesehen, dass nach zumindest einem der Schritte

- Kontaktieren der Oberfläche mit dem Kupplungsreagenz,
- Kontaktieren der Oberfläche mit der zu immobilisierenden Verbindung,
- Kontaktieren der Oberfläche mit dem Zuschlagstoff, oder
- Kontaktieren der Oberfläche mit der Base

die so behandelte Oberfläche getrocknet wird.

In einer noch weiteren Ausführungsform ist vorgesehen, dass die Ausbildung der chemischen Bindung bei Anwesenheit sowohl der Verbindung als auch des Kupplungsreagenz und der Base sowie optional des Zuschlagstoffes erfolgt.

In einer noch weiteren Ausführungsform ist vorgesehen, dass nach der Ausbildung der chemischen Bindung die Oberfläche mit einem Lösungsmittel gewaschen wird.

Erfindungsgemäß wird die Aufgabe in einem zweiten Aspekt gelöst durch ein Verfahren zur Ausbildung einer chemischen Bindung zwischen einer ersten, auf einer insbesondere planaren Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung, einem Kupplungsreagenz und optional einem Zuschlagstoff kontaktiert und umgesetzt wird, dadurch gekennzeichnet, dass die folgenden Schritte zeitlich getrennt voneinander durchgeführt werden:

a) Kontaktieren der Oberfläche mit dem Kupplungsreagenz; und
b) Kontaktieren der Oberfläche mit der zu immobilisierenden Verbindung,

wobei die immobilisierte funktionelle Gruppe eine Base ist.

Erfindungsgemäß wird die Aufgabe in einem dritten Aspekt gelöst durch ein Verfahren zur Ausbildung einer chemischen Bindung zwischen einer ersten, auf einer insbesondere planaren Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung, einem Kupplungsreagenz und einer Base und optional einem Zuschlagstoff kontaktiert und umgesetzt wird, dadurch gekennzeichnet, dass die folgenden Schritte zeitlich getrennt voneinander durchgeführt werden:

a) Kontaktieren der Oberfläche mit dem Kupplungsreagenz; und
b) Kontaktieren der Oberfläche mit der zu immobilisierenden Verbindung und der Base.

Erfindungsgemäß wird die Aufgabe in einem vierten Aspekt gelöst durch ein Verfahren zur Ausbildung einer chemischen Bindung zwischen einer ersten, auf einer insbesondere planaren Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung, einem Kupplungsreagenz und einer Base sowie optional einem Zuschlagstoff kontaktiert und umgesetzt wird, dadurch gekennzeichnet, dass die folgenden Schritte zeitlich getrennt voneinander durchgeführt werden:

a) Kontaktieren der Oberfläche mit dem Kupplungsreagenz und der zu immobilisierenden Verbindung und
b) Kontaktieren der Oberfläche mit der Base.

In einer Ausführungsform gemäß dem zweiten bis vierten Aspekt ist vorgesehen, dass das Kontaktieren der Oberfläche an einer definierten Stelle der Oberfläche erfolgt.

In einer weiteren Ausführungsform gemäß dem zweiten bis vierten Aspekt ist vorgesehen, dass das Kontaktieren gemäß Schritt a) und Schritt b) an der im Wesentlichen gleichen Stelle erfolgt.

In einer noch weiteren Ausführungsform gemäß dem zweiten bis vierten Aspekt ist vorgesehen, dass das Kontaktieren gemäß Schritt a) auf einem größeren Teil der Oberfläche erfolgt als das Kontaktieren gemäß Schritt b).

Erfindungsgemäß wird die Aufgabe in einem fünften Aspekt gelöst durch ein Verfahren zur parallelen Ausbildung einer chemischen Bindung an einer Mehrzahl von Stellen auf einer Oberfläche, bevorzugterweise einer planaren Oberfläche, zwischen einer ersten, auf den Stellen der Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung, einem Kupplungsreagenz und einer Base und optional einem Zuschlagstoff kontaktiert und umgesetzt wird, dadurch gekennzeichnet, dass die folgenden Schritte zeitlich getrennt voneinander durchgeführt werden:

a) Kontaktieren der Oberfläche mit dem Kupplungsreagenz und der zu immobilisierenden Verbindung an mindestens einer definierten Stelle der Oberfläche,
b) Mindestens einmaliges Wiederholen von Schritt a), wobei bei der Wiederholung von Schritt a) das Kontaktieren an mindestens einer weiteren definierten Stelle der Oberfläche erfolgt, und
c) Kontaktieren der Oberfläche mit der Base, bevorzugterweise an den definierten Stellen der Oberfläche.

Erfindungsgemäß wird die Aufgabe in einem sechsten Aspekt gelöst durch ein Verfahren zur parallelen Ausbildung einer chemischen Bindung an einer Mehrzahl von Stellen auf einer Oberfläche, bevorzugterweise einer planaren Oberfläche, zwischen einer ersten, auf den Stellen der Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung, einem Kupplungsreagenz und einer Base und optional einem Zuschlagstoff kontaktiert und umgesetzt wird, dadurch gekennzeichnet, dass die folgenden Schritte zeitlich getrennt voneinander durchgeführt werden:

a) Kontaktieren der Oberfläche mit dem Kupplungsreagenz und der zu immobilisierenden Verbindung an mindestens einer definierten Stelle der Oberfläche,
b) Kontaktieren der Oberfläche mit der Base, bevorzugterweise an der mindestens einen definierten Stelle der Oberfläche gemäß Schritt a);
c) Mindestens einmaliges Wiederholen von Schritt a) und Schritt b), wobei bei der Wiederholung von Schritt a) das Kontaktieren an einer weiteren definierten Stelle der Oberfläche erfolgt.

In einer Ausführungsform gemäß dem fünften und sechsten Aspekt ist vorgesehen, dass das Kontaktieren der Oberfläche mit der Base an der im Wesentlichen gleichen Stelle erfolgt wie das Kontaktieren der Oberfläche mit dem Kupplungsreagenz und der zu immobilisierenden Verbindung.
In einer noch weiteren Ausführungsform gemäß dem fünften und sechsten Aspekt ist vorgesehen, dass bei der Wiederholung von Schritt a) die zu immobilisierende Verbindung die gleiche oder eine andere zu immobilisierende Verbindung ist verglichen mit derjenigen des vorhergehenden Schrittes. In einer Ausführungsform gemäß dem zweiten bis sechsten Aspekt ist vorgesehen, dass dem Kupplungsreagenz und/oder der Verbindung mindestens ein Zuschlagstoff zugesetzt ist/wird.
In einer weiteren Ausführungsform ist vorgesehen, dass das Verfahren weiter den Schritt umfasst:
Kontaktieren der Oberfläche mit einem Zuschlagstoff, wobei dieser Schritt getrennt, insbesondere zeitlich getrennt von dem Schritt Kontaktieren der Oberfläche mit dem Kupplungsreagenz und/oder dem Schritt Kontaktieren der Oberfläche mit dem Kupplungsreagenz und der zu immobilisierenden Verbindung erfolgt.
In einer Ausführungsform gemäß dem zweiten bis sechsten Aspekt ist vorgesehen, dass nach zumindest einem der Schritte

- Kontaktieren der Oberfläche mit dem Kupplungsreagenz,
- Kontaktieren der Oberfläche mit dem Kupplungsreagenz und der zu immobilisierenden Verbindung,
- Kontaktieren der Oberfläche mit der Base,
- Kontaktieren der Oberfläche mit der zu immobilisierenden Verbindung und der Base oder
- Kontaktieren der Oberfläche mit einem Zuschlagstoff die so behandelte Oberfläche getrocknet wird.

In einer weiteren Ausführungsform gemäß dem zweiten bis sechsten Aspekt ist vorgesehen, dass die Ausbildung der chemischen Bindung bei Anwesenheit sowohl der Verbindung als auch des Kupplungsreagenz und optional des Zuschlagstoffes und optional der Base erfolgt.
In einer noch weiteren Ausführungsform gemäß dem zweiten bis sechsten Aspekt ist vorgesehen, dass nach der Ausbildung der chemischen Bindung die Oberfläche mit einem Lösungsmittel gewaschen wird.
In einer noch weiteren Ausführungsform gemäß dem zweiten bis sechsten Aspekt ist vorgesehen, dass das Kupplungsreagenz, der Zuschlagstoff, die Base und/oder die Verbindung jeweils in einer Lösung vorliegt.
In einer Ausführungsform der erfindungsgemäßen Verfahren ist vorgesehen, dass es sich bei der ausgebildeten Bindung um eine Bindung handelt, die ausgewählt ist aus der Gruppe, die Carbonsäureamid-Bindung, Carbonsäureester-Bindung, Carbonsäureanhydrid-Bindung, Sulfonsäureamid-Bindung, Phosphorsäureester-Bindung, Phosphonsäureester-Bindung, Phosphorigsäureester-Bindung und Phosphorsäureamid-Bindung umfasst.
In einer Ausführungsform der erfindungsgemäßen Verfahren ist vorgesehen, dass das Kupplungsreagenz ausgewählt ist aus der Gruppe, die Carbodiimide, Aminium- und Phosphoniumsalze umfasst, bevorzugterweise ausgewählt ist aus der Gruppe, die HBTU, TBTU und PyBOP umfasst.
In einer Ausführungsform der erfindungsgemäßen Verfahren ist vorgesehen, dass das Kupplungsreagenz in einem Lösungsmittel vorliegt und das Lösungsmittel bevorzugterweise ausgewählt ist aus der Gruppe, die N,N-Dimethylformamid, 1-Methyl-2-pyrrolidinon, N,N-Dimethylacetamid, Dimethylsulfoxid, Tetrahydrofuran, Dioxan, Acetonitril, Methanol, Ethylacetat, 2,2,2-Trifluorethanol, 1,1,1,3,3,3-Hexafluor-2-Propanol, 1,2-Dibromethan, Dichlormethan, Diethylether und Aceton umfasst.
In einer weiteren Ausführungsform der erfindungsgemäßen Verfahren ist vorgesehen, dass der Zuschlagstoff ausgewählt ist aus der Gruppe, die Benzotriazole, Benzotriazine, Phenole und organische und anorganische Salze umfasst, bevorzugterweise der Zuschlagstoff ausgewählt ist aus der Gruppe, die HOBt, HOAt, HOOBt, HOPfp, $NaClO_4$ und KSCN umfasst.
In eine weiteren Ausführungsform der erfindungsgemäßen Verfahren ist vorgesehen, dass der Zuschlagstoff

in einem Lösungsmittel vorliegt und das Lösungsmittel bevorzugterweise ausgewählt ist aus der Gruppe, die N,N-Dimethylformamid, 1-Methyl-2-pyrrolidinon, N,N-Dimethylacetamid, Dimethylsulfoxid, Tetrahydrofuran, Dioxan, Acetonitril, Methanol, Ethylacetat, 2,2,2-Trifluorethanol, 1,1,1,3,3,3-Hexafluor-2-Propanol, 1,2-Dibromethan, Dichlormethan, Diethylether und Aceton umfasst.

In einer weiteren Ausführungsform der erfindungsgemäßen Verfahren ist vorgesehen, dass die zu immobilisierende Verbindung ausgewählt ist aus der Gruppe, die Carbonsäuren, Carbonsäureester, Sulfonsäuren, Phosphorsäureester, Phosphonsäureester, Phosphorigsäureester, Phosphoramidite, primäre Amine, sekundäre Amine und Alkohole umfasst, und die Verbindung bevorzugterweise ausgewählt ist aus der Gruppe, die Carbonsäuren, primäre Amine und sekundäre Amine umfasst.

In einer weiteren Ausführungsform der erfindungsgemäßen Verfahren ist vorgesehen, dass die Verbindung in einem Lösungsmittel vorliegt und das Lösungsmittel bevorzugterweise ausgewählt ist aus der Gruppe, die N,N-Dimethylformamid, 1-Methyl-2-pyrrolidinon, N,N-Dimethylacetamid, Dimethylsulfoxid, Tetrahydrofuran, Dioxan, Acetonitril, Methanol, Ethylacetat, 2,2,2-Trifluorethanol, 1,1,1,3,3,3-Hexafluor-2-Propanol, 1,2-Dibromethan, Dichlormethan, Diethylether und Aceton umfasst.

In einer weiteren Ausführungsform der erfindungsgemäßen Verfahren ist vorgesehen, dass die Base ausgewählt ist aus der Gruppe, die tertiäre Amine, Pyridine, Imidazole und organische Salze umfasst, bevorzugterweise die Base ausgewählt ist aus der Gruppe, die DIPEA, Collidin, DMAP, NMI, NMM, Pyridin und KOBt umfasst.

In einer weiteren Ausführungsform der erfindungsgemäßen Verfahren ist vorgesehen, dass die Base in einem Lösungsmittel vorliegt, wobei bevorzugterweise das Lösungsmittel ausgewählt ist aus der Gruppe, die N,N-Dimethylformamid, 1-Methyl-2-pyrrolidinon, N,N-Dimethylacetamid, Dimethylsulfoxid, Tetrahydrofuran, Dioxan, Acetonitril, Methanol, Ethylacetat, 2,2,2-Trifluorethanol, 1,1,1,3,3,3-Hexafluor-2-Propanol, 1,2-Dibromethan, Dichlormethan, Diethylether und Aceton umfasst.

In einer weiteren Ausführungsform der erfindungsgemäßen Verfahren ist vorgesehen, dass die immobilisierte funktionelle Gruppe ausgewählt ist aus der Gruppe, die Carbonsäuren, Carbonsäureester, Sulfonsäure, Phosphorsäureester, Phosphonsäureester, Phosphorigsäureester, Phosphoramidite, primäre Amine, sekundäre Amine und Alkohole umfasst und die bevorzugterweise die immobilisierte funktionelle Gruppe ausgewählt ist aus der Gruppe, die Carbonsäuren, primäre Amine und sekundäre Amine umfasst.

In einer weiteren Ausführungsform der erfindungsgemäßen Verfahren ist vorgesehen, dass das Kontaktieren mittels Kontaktieren mit einer Kapillare erfolgt.

In einer noch weiteren Ausführungsform der erfindungsgemäßen Verfahren ist vorgesehen, dass das Kontaktieren durch Baden der Oberfläche in einer der Lösungen erfolgt.

In einer noch weiteren Ausführungsform der erfindungsgemäßen Verfahren ist vorgesehen, dass das Kontaktieren durch Aufsprühen, bevorzugterweise einer das jeweilige Reagenz enthaltenden Lösung erfolgt.

In einer noch weiteren Ausführungsform der erfindungsgemäßen Verfahren ist vorgesehen, dass das Kontaktieren durch Aufstempeln erfolgt.

Erfindungsgemäß wird die Aufgabe in einem siebten Aspekt gelöst durch eine Oberfläche mit einer chemischen Bindung zwischen einer ersten funktionellen Gruppe und einer zweiten funktionellen Gruppe, wobei die zweite funktionelle Gruppe Teil einer auf der Oberfläche immobilisierten Verbindung ist, herstellbar durch ein Verfahren gemäß der vorliegenden Erfindung.

Der vorliegenden Erfindung liegt die überraschende Erkenntnis zugrunde, dass es durch die spezifische Abfolge und zeitliche Trennung der Schritte des Kontaktierens der Oberfläche mit dem Kupplungsreagens und Kontaktieren der Oberfläche mit der zu immobilisierenden Verbindung bzw. dadurch dass die reaktive Species der zu immobilisierenden Verbindung erst auf der Oberfläche ausgebildet wird, möglich ist, die im Stand der Technik bekannten und vorstehend beschriebenen Nachteile eines Verfahrens zur Ausbildung einer chemischen Bindung zu umgehen, bei dem die Kupplung von typischerweise derivatisierten Aminosäuren erfolgt, wobei die Aminosäuren vor der Kupplung als aktivierte derivatisierte Aminosäuren vorliegen. Ein derartiges Verfahren ist das im Stand der Technik bekannte SPOT-Verfahren. Insbesondere für den Fall, dass es sich bei den erfindungsgemäßen Verfahren um solche handelt, bei denen die Synthese eines Polypeptids erfolgt und die zu immobilisierende Verbindung insoweit eine Aminosäure ist, werden die bei der Verwendung von aktivierten Aminosäurederivaten beobachteten Stabilitätsprobleme, insbesondere für den Fall, dass die aktivierten Derivate in Gegenwart von Basen aufbewahrt werden, vermieden. So führte längeres Stehenlassen von Aminosäuren, die mit dem Kupplungsreagenz HATU und der Base Collidin voraktiviert wurden, zu erhöhter Racemisierung der Aminosäure [L. A. Carpino et al., *Tetrahedron* **1999,** *55*, 6813-6830] und zu schneller Zersetzung. Lösungen reiner Kupplungsreagenzien oder Lösungen von Kupplungsreagenzien mit Basen sind dagegen häufig für Stunden stabil [F. Albericio et al., *J. Org. Chem.* **1998,** *63*, 9678-9683] [F. Albericio et al., *Tetrahedron Lett.* **1997,** *38*, 4853-4856].

Darüber hinaus erlauben die erfindungsgemäßen Verfahren, soweit es sich bei der zu immobilisierenden Verbindung um eine Aminosäure handelt, dass die als Ausgangsprodukte für die Synthese verwendeten Aminosäuren in einer Form vorliegen können, die eine langfristige Lagerung erlauben, so dass bei automatisierten Ausführungsformen der erfindungsgemäßen Verfahren eine zeit- und materialintensive Voraktivierung vor jedem Kupplungszyklus nicht erforderlich ist. Weiterhin wird mit dem erfindungsgemäßen Verfahren, soweit es sich bei der zu immobilisierenden Verbindung um eine Aminosäure oder ein Peptid mit einer Carboxyl- und einer Aminogruppe handelt, gewährleistet, dass die Anzahl der unerwünschten Nebenreaktionen infolge einer gleichzeitigen Präsenz von sowohl Carboxyl- als auch Aminogruppen verringert wird.

Das Prinzip der Festphasensynthese von Peptiden (Solid Phase Peptide Synthesis, SPPS) ist den Fachleuten grundsätzlich bekannt und schematisch nochmals in Fig. 1 beschrieben.

Bei dem erfindungsgemäßen Verfahren zur Ausbildung einer chemischen Bindung zwischen einer ersten, auf einer Oberfläche, insbesondere planaren Oberfläche, immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung und einem Kupplungsreagenz und optional einem Zuschlagsstoff sowie ebenfalls optional mit einer Base kontaktiert und umgesetzt wird, ist vorgesehen, dass die Schritte Kontaktieren der Oberfläche mit dem Kupplungsreagenz und Kontaktieren der Oberfläche mit der zu immobilisierenden Verbindung getrennt erfolgen. Dabei ist insbesondere vorgesehen, dass die Durchführung der vorstehend genannten beiden Schritte zeitlich voneinander getrennt erfolgt. Es ist dabei im Rahmen der vorliegenden Erfindung, dass zuerst das Kupplungsreagenz mit der Oberfläche kontaktiert wird und anschließend die Oberfläche mit der zu immobilisierenden Verbindung. Alternativ ist es möglich, dass die Oberfläche zuerst mit der zu immobilisierenden Verbindung und anschließend mit dem Kupplungsreagenz kontaktiert wird.

Im Rahmen der vorliegenden Erfindung kann dabei der Zusatz bzw. das Vorhandensein einer Base die Ausbildung der Bindung zwischen der immobilisierten funktionellen Gruppe und der zweiten funktionellen Gruppe positiv beeinflussen. Das Vorhandensein der Base kann entweder durch Zugabe einer entsprechenden Base, bevorzugterweise in einem geeigneten Lösungsmittel, erfolgen, oder aber durch die einzelnen, miteinander in Reaktion zu bringenden Komponenten oder Agenzien, d. h. zu immobilisierender Verbindung, Kupplungsreagenz und optional Zuschlagsstoff, durch eine derselben alleine oder in Kombination mit einer oder mehreren der miteinander in Reaktion zu bringenden Komponenten dargestellt oder ausgebildet werden. Beispielsweise kann infolge der Ausbildung einer chemischen Bindung zwischen einer Aminosäure, die mit ihrer Aminogruppe die immobilisierte funktionelle Gruppe darstellt und bei Verwendung von Carbodiimid als Kupplungsreagenz das solchermaßen immobilisierte Amin bereits als Base wirken, so dass die Zugabe einer weiteren Base nicht unbedingt erforderlich ist. Weitere Ausführungsformen des erfindungsgemäßen Verfahrens ergeben sich für den Fall, dass als Base eine Komponente verwendet wird, die verschieden ist von der immobilisierten Verbindung, die die immobilisierte funktionelle Gruppe im Sinne der vorliegenden Erfindung bereitstellt. Vielmehr wird in diesen Ausführungsformen die Base als eigenständige Verbindung in dem Verfahren zugegeben bzw. verwendet. Dabei kann in einer Ausführungsform vorgesehen sein, dass Kupplungsreagenz und Base gemeinsam, bevorzugterweise in einem Lösungsmittel auf die Oberfläche aufgebracht werden und anschließend daran die zu immobilisierende Verbindung. Alternativ ist es möglich, die zu immobilisierende Verbindung mit der Oberfläche zu kontaktieren und anschließend das Kupplungsreagenz und die Base, wiederum bevorzugterweise in einer beide umfassenden Lösung.

In einer weiteren Ausführungsform ist es vorgesehen, dass das Kupplungsreagenz zuerst auf die Oberfläche durch Kontaktieren gegeben wird und sodann eine Mischung aus zu immobilisierender Verbindung und Base. Alternativ kann vorgesehen sein, dass zuerst die Mischung aus zu immobilisierender Verbindung und Base und sodann das Kupplungsreagenz auf die Oberfläche aufgegeben wird. In einer weiteren Ausführungsform erfolgt die Zugabe der einzelnen Komponenten, d. h. der zu immobilisierenden Verbindung, des Kupplungsreagenzes sowie der Base, zeitlich aufeinander, wobei grundsätzlich alle Permutationen der Reihenfolge der Aufbringung der Komponenten auf die Oberfläche möglich sind. Die zu immobilisierende Verbindung, das Kupplungsreagenz sowie die Base werden dabei bevorzugterweise in separaten Arbeitsschritten auf die Oberfläche aufgebracht. Entsprechend kann die Oberfläche zuerst mit dem Kupplungsreagenz, gefolgt von der Base und anschließend mit der zu immobilisierenden Verbindung kontaktiert werden. Alternativ kann zuerst ein Kontaktieren der Oberfläche mit dem Kupplungsreagenz erfolgen, gefolgt von Kontaktieren der Oberfläche mit der zu immobilisierenden Verbindung und schließlich Kontaktieren der Oberfläche mit der Base. In einer weiteren alternativen Ausführungsform ist vorgesehen, dass die Oberfläche zuerst mit der Base kontaktiert wird, anschließend mit der zu immobilisierenden Verbindung und schließlich mit dem Kupplungsreagenz. In einer weiteren Ausführungsform ist vorgesehen, dass die Oberfläche zuerst mit der Base, dann mit dem Kupplungsreagenz und schließlich mit der zu immobilisierenden Verbindung kontaktiert wird. In einer weiteren alternativen Ausführungsform ist vorgesehen, dass die Oberfläche zuerst mit der zu immobilisierenden Verbindung kontaktiert wird, sodann mit dem Kupplungsreagenz und schließlich mit der Base. In einer noch weiteren alternativen Ausführungsform ist vorgesehen, dass die Oberfläche zuerst mit der zu immobilisierenden Verbindung, sodann mit der Base und schließlich mit dem Kupplungsreagenz kontaktiert wird. Bevorzugterweise erfolgt nach einem jeden Auftrag eines oder mehrerer der vorstehend genannten Agenzien ein Trocknungsschritt, zumindest werden jedoch die einzelnen Agenzien bzw. Mischungen, wie vorstehend dargelegt, separat mit der Oberfläche kontaktiert. Dabei ist es im Rahmen der vorliegenden Erfindung, dass das Kontaktieren der Oberfläche mit den verschiedenen Agenzien an der im wesentlichen

gleichen Stelle erfolgt. Es ist dabei auch im Rahmen der vorliegenden Erfindung, dass das Kontaktieren einzelner Agenzien auf einem größeren Teil der Oberfläche erfolgt als das Kontaktieren mit einem oder mehreren der anderen Agenzien. Mit Agenzien werden hierin allgemein insbesondere die zu immobilisierende Verbindung, das Kupplungsreagenz, die Base und der Zuschlagsstoff bezeichnet. Dabei ist es imRahmen der vorliegenden Erfindung, dass der Zuschlagstoff grundsätzlich zu einem jeden Zeitpunkt im Reaktionsgeschehen zugegeben werden kann.

Bei dem erfindungsgemäßen Verfahren zur Ausbildung einer chemischen Bindung zwischen einer ersten, auf einer insbesondere planaren Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung, einem Kupplungsreagenz und einer Base und optional einem Zuschlagstoff kontaktiert und umgesetzt wird, ist vorgesehen, dass die folgenden Schritte zeitlich getrennt voneinander durchgeführt werden:

a) Kontaktieren der Oberfläche mit dem Kupplungsreagenz und;
b) Kontaktieren der Oberfläche mit der zu immobilisierenden Verbindung und der Base,

wobei die vorstehend beschriebenen Vorteile ebenfalls auftreten.

Dies gilt auch bei dem weiteren erfindungsgemäßen Verfahren zur Ausbildung einer chemischen Verbindung zwischen einer ersten, auf einer insbesondere planaren Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung, einem Kupplungsreagenz und einer Base und optional einem Zuschlagstoff kontaktiert und umgesetzt wird, wobei vorgesehen ist, dass die folgenden Schritte zeitlich getrennt voneinander durchgeführt werden:

a) Kontaktieren der Oberfläche mit dem Kupplungsreagenz und der zu immobilisierenden Verbindung und
b) Kontaktieren der Oberfläche mit der Base.

Darüber hinaus tritt hier noch insbesondere ein weiterer Vorteil dadurch auf, daß parallele Synthesen, wie auch hierin offenbart, einfach und schnell durchzuführen sind aufgrund der Tatsache, daß für alle Synthesen i.d.R. das gleiche Kupplungsreagenz und die gleiche Base verwendet wird und es apparativ einfacher ist, eine Lösung auf verschiedenen Stellen aufzubringen als verschiedene Lösungen.

In dem erstgenannten der beiden vorstehenden Verfahren erfolgt dabei das Kontaktieren der Oberfläche mit dem Kupplungsreagens zeitlich getrennt von dem Kontaktieren der Oberfläche mit der zu immobilisierenden Verbindung und der Base. Die beiden Schritte sind dabei bevorzugterweise in dieser Reihenfolge durchzuführen. Der zeitliche Abstand zwischen den einzelnen Kontaktierungsschritten kann dabei sowenig wie 0.01 sek und soviel wie 5 Stunden betragen. Das Kontaktieren der Oberfläche mit der zu immobilisierenden Verbindung und der Base kann dabei so ausgebildet sein, dass die zu immobilisierende Verbindung und die Base in einer Lösung oder Lösungsmittel gemeinsam vorliegen und die beide enthaltende Lösung oder das die beide enthaltende Lösungsmittel durch Kontaktieren der Lösung oder des Lösungsmittels mit der Oberfläche auf der Oberfläche abgesetzt werden. Es ist jedoch auch im Rahmen der vorliegenden Erfindung, dass die zu immobilisierende Verbindung mit der Oberfläche kontaktiert wird und zeitlich getrennt davon die Base mit der Oberfläche kontaktiert wird. Dabei ist bevorzugt, dass die Kontaktierung der Oberfläche mit der zu immobilisierenden Verbindung und der Base sehr schnell hintereinander erfolgt, wobei es sowohl möglich ist, zuerst die Base abzusetzen und dann die zu immobilisierende Verbindung als auch zuerst die zu immobilisierende Verbindung und dann die Base. Die zeitlichen Rahmenbedingungen sind bevorzugterweise so, dass die bevorzugterweise in Lösung abgesetzte zu immobilisierende Verbindung noch nicht eingetrocknet ist zu dem Zeitpunkt, da die Base hinzugegeben wird. Gleiches gilt auch für den Fall, dass die Base zuerst abgesetzt wird. Typische Zeitabstände hängen von den jeweiligen Versuchsbedingungen ab, so beispielsweise von der Luftfeuchtigkeit und der Temperatur. Bei Raumtemperatur und normaler Luftfeuchtigkeit beträgt der zeitliche Abstand für diese Form der Ausführung des Kontaktierens der Oberfläche mit der zu immobilisierenden Verbindung und der Base sowenig wie 0.01 sek und soviel wie 1 Stunde.

Die vorstehend beschriebenen Überlegungen hinsichtlich der Reihenfolge und der Art und Weise des Kontaktierens der Oberfläche mit der zu immobilisierenden Verbindung und der Base gelten sinngemäß auch für das zweite vorstehend beschriebene erfindungsgemäße Verfahren, bei dem ein Kontaktieren der Oberfläche mit dem Kupplungsreagens und der zu immobilisierenden Verbindung erfolgt in einem ersten Schritt erfolgt und das Kontaktieren der Oberfläche mit der Base in einem zweiten Schritt. Auch hier ist eine gemeinsame sowie getrennte Kontaktierung der Oberfläche des Kupplungsreagens und der zu immobilisierenden Verbindung grundsätzlich möglich, ausgeprägt in Analogie zu dem Kontaktieren der zu immobilisierenden Verbindung und der Base in dem ersten der vorstehend beschriebenen erfindungsgemäßen Verfahren.

Bevorzugten Ausführungsformen der erfindungsgemäßen Verfahren ist gemeinsam, dass die Aktivierung der

zu immobilisierenden Verbindung erst dann erfolgt, wenn diese auf der Oberfläche vorhanden ist oder auf dieser abgesetzt ist oder auf dieser abgesetzt wird.

Wie hierein verwendet bezeichnet der Begriff der Oberfläche bevorzugterweise die Oberfläche eines Trägermaterials.

In einer Ausführungsform der vorliegenden Erfindung handelt es sich bei der Ausbildung einer chemischen Bindung bevorzugterweise um eine Peptidbindung. In diesem Falle stammt in einer Ausführungsform die immobilisierte funktionelle Gruppe von einer bereits immobilisierten Aminosäure. Die funktionelle Gruppe kann dabei bevorzugterweise entweder eine Aminogruppe oder eine Carboxylgruppe sein. Darüber hinaus ist in diesem Falle die zweite funktionelle Gruppe eine Carboxylgruppe bzw. Aminogruppe, die von der weiteren zu immobilisierenden Verbindung, im vorliegenden Falle bevorzugterweise ebenfalls einer Aminosäure stammt, die an die erste bereits immobilisierte Aminosäure durch Ausbildung einer Peptidbindung gebunden und damit immobilisiert werden soll. Die erfindungemäßen Verfahren können grundsätzlich mehrfach hintereinander zur Ausführung gelangen, so dass ein Peptid mit einer erwünschten Länge und Sequenz auf der jeweiligen Oberfläche synthetisiert werden kann.

Zwischen der Oberfläche und dem synthetisierten Peptid befindet sich häufig ein Linker, welcher sowohl mit den erfindungsgemäßen Verfahren als auch mit herkömmlichen Methoden an die Oberfläche angeknüpft werden kann. Dem Fachmann ist eine Vielzahl verschiedener Linker bekannt [F. Guillier et al., *Chem*. *Rev*. **2000,** *100*, 2091-2157]. Das Peptid kann aber auch ohne Linker direkt auf der Oberfläche synthetisiert werden, wenn diese geeignet funktionalisiert ist , wie beispielsweise mit OH-Gruppen, auf denen das Peptid über eine Esterbindung immobilisiert ist.

Im Rahmen der erfindungsgemäßen Verfahren ist besonders bevorzugt, wenn ein jeder Kontaktierungsschritt an einer definierten Stelle der Oberfläche erfolgt. Bevorzugterweise ist eine derartige definierte Stelle der Oberfläche ein Teilbereich der Oberfläche. So ist in einer Ausführungsform der erfindungsgemäßen Verfahren vorgesehen, dass das Kontaktieren auf einer Oberfläche erfolgt und die Stelle zuvor bestimmt oder festgelegt worden ist. Dies ist besonders dann erforderlich, wenn auf der Oberfläche mehrere Stellen vorhanden sind, auf denen eine Verbindung immobilisiert wird bzw. immobilisiert ist und an denen bevorzugterweise das erfindungsgemäße Verfahren parallel oder sequenziell durchgeführt wird. Eine derartige definierte Stelle wird hierin auch als Reaktionsstelle bezeichnet. Eine typische Anwendung des Verfahrens ist insoweit bei der Herstellung von Bio-Chips zu sehen, die an definierten Stellen Polymere, insbesondere Peptide, die hierin allgemein auch als Polypeptide bezeichnet werden, oder Polynucleotide mit einer definierten Sequenz aufweisen.

Es ist ebenso im Rahmen der erfindungsgemäßen Verfahren, dass das Kupplungsreagens entweder selektiv an der Reaktionsstelle, an der Reaktionsstelle und einen diesen umgebenden Bereich, oder aber insgesamt auf der Oberfläche aufgebracht wird. Bevorzugterweise werden in einem derartigen Fall dann die zu immobilisierende Verbindung und die Base an entweder dieser Reaktionsstelle, oder in einem Bereich, der mit dem Kupplungsreagens infolge Kontaktieren der Oberfläche mit dem Kupplungsreagens ausgestattet ist, abgesetzt oder aufgetragen. So ist in einer Ausführungsform der erfindungsgemäßen Verfahren vorgesehen, dass ein größerer Bereich der Oberfläche als die Reaktionsstelle mit Kupplungsreagens ausgestattet ist und in diesen Bereich, bevorzugterweise wieder an einer spezifischen Stelle davon, die zu immobilisierende Verbindung und die Base durch Kontaktieren der Oberfläche bereitgestellt werden.

Es ist auch im Rahmen der erfindungsgemäßen Verfahren, dass die Oberfläche mit dem Kupplungsreagens und der zu immobilisierenden Verbindung kontaktiert wird und dabei diese darauf abgesetzt werden. Bevorzugterweise werden dabei das Kupplungsreagens und die zu immobilisierende Verbindung an der definierten Stelle der Oberfläche mit dieser kontaktiert bzw. dort abgesetzt werden. In einem nächsten Schritt erfolgt dann das Kontaktieren der Oberfläche mit der Base. Dabei kann vorgesehen sein, dass das Kontaktieren der Oberfläche mit der Base der Stelle entspricht, an der gemäß Schritt a) das Kupplungsreagens und die zu immobilisierende Verbindung die Oberfläche kontaktieren bzw. darauf abgesetzt wurden. Es ist jedoch auch im Rahmen der vorliegenden Erfindung, dass sich der Bereich der Oberfläche, der mit der Base kontaktiert wird, über den Bereich oder die Stelle der Oberfläche, die mit dem Kupplungsreagens und der zu immobilisierenden Verbindung kontaktiert wird, erstreckt. Es ist auch im Rahmen der erfindungsgemäßen Verfahren, dass das Kontaktieren der Oberfläche mit der Base so ausgebildet ist, dass die gesamte Oberfläche mit der Base kontaktiert wird.

Das vorstehend beschriebene Prinzip kann auch bei einer Ausgestaltung der Verfahren angewandt werden, bei der chemische Bindungen an einer Mehrzahl von Stellen auf einer Oberfläche, bevorzugterweise einer planaren Oberfläche, wie hierin definiert, ausgebildet werden sollen. Dabei erfolgen die vorstehend beschriebenen Verfahren für eine jede der Mehrzahl der Stellen der Oberfläche grundsätzlich gleich.

Insbesondere ist es im Rahmen der vorliegenden Erfindung, dass für den Fall, dass an einer Mehrzahl von Stellen einer Oberfläche eine Ausbildung einer chemischen Bindung zwischen einer ersten, auf einer insbesondere planaren Oberfläche immobilisierten funktionellen Gruppe und einer zweiten funktionellen Gruppe erfolgen soll, wobei die zweite funktionelle Gruppe Teil einer zu immobilisierenden Verbindung ist, ein jeder der Schritte, wie sie im Zusammenhang mit den vorstehenden Verfahren beschrieben wurden, und bei denen ein Agens oder eine Mischung aus mehreren Agenzien mit der Oberfläche kontaktiert wird, zuerst an einer jeden der Mehrzahl der Stellen durchgeführt,

bevor der jeweils weitere Schritt an der Mehrzahl der Stellen durchgeführt wird. Es ist auch im Rahmen der vorliegenden Erfindung, dass von den einzelnen Kontaktierungsschritten mehr als einer der jeweils einzelnen Schritte, bei dem ein Agens oder eine Mischung aus Agenzien mit jeweils einer der Mehrzahl der Stellen kontaktiert wird, an der einzelnen Stelle durchgeführt wird und sodann die gleiche oder abgewandelte Abfolge von Schritten an einer oder mehreren der weiteren Stellen der Mehrzahl der Schritte durchgeführt wird. Das erfindungsgemäße Verfahren ist dabei auf eine konkrete Abfolge der einzelnen Schritte an der Mehrzahl der verschiedenen Stellen nicht beschränkt. Vielmehr ist eine jegliche Kombination aus Einzelschritten bzw. Abfolge von mehreren Schritten an einer oder mehreren der Mehrzahl der Stellen der Oberfläche möglich.

Bei einer parallelen Ausführung der erfindungsgemäßen Verfahren ist in einem Aspekt insbesondere vorgesehen, dass die folgenden Schritte des erfindungsgemäßen Verfahrens zur parallelen Ausbildung einer chemischen Bindung an einer Mehrzahl von Stellen auf einer Oberfläche, bevorzugterweise einer planaren Oberfläche, zwischen einer ersten, auf den Stellen der Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung, einem Kupplungsreagens, einer Base und optional einem Zuschlagsstoff kontaktiert und umgesetzt wird, zeitlich getrennt voneinander durchgeführt werden:

a) Kontaktieren der Oberfläche mit dem Kupplungsreagenz und der zu immobilisierenden Verbindung an mindestens einer definierten Stelle der Oberfläche,
b) Mindestens einmaliges Wiederholen von Schritt a), wobei bei der Wiederholung von Schritt a) das Kontaktieren an mindestens einer weiteren definierten Stelle der Oberfläche erfolgt, und
c) Kontaktieren der Oberfläche mit der Base bevorzugterweise an den definierten Stellen der Oberfläche.

Bei diesem Verfahren erfolgt das Kontaktieren der Oberfläche mit dem Kupplungsreagens und der zu immobilisierenden Verbindung grundsätzlich in gleicher Weise wie bei dem erfindungsgemäßen Verfahren zur Ausbildung einer chemischen Bindung beschrieben. Als nächster Schritt ist vorgesehen, dass an den verschiedenen Stellen, an denen eine chemische Bindung ausgebildet werden soll, beispielsweise im Rahmen einer Synthese eines Polymers, insbesondere eines Polypeptids, der Schritt a) mindestens einmal wiederholt wird. Bevorzugterweise erfolgt die Wiederholung an einer anderen Stelle als im jeweils vorhergehenden Schritt. Die Wiederholung kann jedoch auch an der gleichen Stelle oder Reaktionsstelle erfolgen. Das Kontaktieren der Oberfläche mit der Base gemäß Schritt c) kann dabei so erfolgen, dass die einzelnen Stellen oder Reaktionsstellen der Oberfläche, an denen ein Kontaktieren mit dem Kupplungsreagens und der zu immobilisierenden Verbindung erfolgt ist, einzeln mit der Base kontaktiert werden. Es ist jedoch auch im Rahmen der vorliegenden Erfindung, dass mehrere der Stellen gleichzeitig und ggf. auch die gesamte Oberfläche mit der Base kontaktiert wird.

Die Alternative des erfindungsgemäßen Verfahrens zur parallelen Ausbildung, welche eine parallele Ausbildung einer chemischen Bindung an einer Mehrzahl von Stellen auf einer Oberfläche, bevorzugterweise einer planaren Oberfläche, zwischen einer ersten, auf den Stellen der Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung, einem Kupplungsreagens, einer Base und optional einem Zuschlagsstoff kontaktiert und umgesetzt wird, und bei dem vorgesehen ist, dass die folgenden Schritte zeitlich getrennt voneinander durchgeführt werden:

a) Kontaktieren der Oberfläche mit dem Kupplungsreagenz und der zu immobilisierenden Verbindung an mindestens einer definierten Stelle der Oberfläche,
b) Kontaktieren der Oberfläche mit der Base, bevorzugterweise an der mindestens einen definierten Stelle der Oberfläche gemäß Schritt a)
c) Mindestens einmaliges Wiederholen von Schritt a) und Schritt b), wobei bei der Wiederholung von Schritt a) das Kontaktieren an einer weiteren definierten Stelle der Oberfläche erfolgt.

Dieses Verfahren unterscheidet sich von dem zuvor beschriebenen erfindungsgemäßen Verfahren zur parallelen Ausbildung einer chemischen Bindung dadurch, dass für eine jede einzelne Reaktionsstelle nach dem Kontaktieren der Oberfläche bzw. der Reaktionsstelle darauf, mit dem Kupplungsreagens und der zu immobilisierenden Verbindung ein Kontaktieren der Oberfläche mit der Base erfolgt. Diese Abfolge von Schritten wird sodann für eine jede der definierten Stellen wiederholt. Dabei ist es im Rahmen der vorliegenden Erfindung, dass Schritt a) an der gleichen Stelle mehrfach wiederholt wird, bevor Schritt b) durchgeführt wird. Es ist weiterhin im Rahmen der vorliegenden Erfindung, dass der Schritt a) an mehreren verschiedenen definierten Stellen durchgeführt wird, bevor das Kontaktieren der Oberfläche mit der Base erfolgt. Besonders bevorzugt ist dabei, dass das Kontaktieren der Base an der gleichen oder in etwa an der gleichen definierten Stelle oder den gleichen definierten Stellen erfolgt, wie in Schritt a). Diese Abfolge aus mehrfachem Wiederholen des solchermaßen ausgebildeten Schrittes a) und des solchermaßen ausgebildeten Schrittes b) kann sodann selbst wiederum mindestens einmalig wiederholt werden. Bei einem jeden der erfindungsgemäßen Verfahren zur parallelen Ausbildung einer chemischen Bindung gelten dabei hinsichtlich der Ausgestaltung der einzelnen Verfahrensschritte und der Auswahl der funktionellen Gruppen grundsätzlich die im Zusammenhang mit den erfindungsgemäßen Verfahren zur Ausbildung einer chemischen Bindung hierein offenbarten Merkmale und Ausführungsformen.

Bei den erfindungsgemäßen Verfahren zur parallelen Ausbildung einer chemischen Bindung kann in einer Ausführungsform vorgesehen sein, dass die zu immobilisierende Verbindung die gleiche oder eine andere Verbindung ist verglichen mit derjenigen Verbindung, die im jeweils vorhergehenden Schritt an dieser oder einer anderen Reaktionsstelle verwendet wird. Bei den besagten erfindungsgemäßen Verfahren ist es möglich, dass ein Wiederholen von Schritt a) oder Schritt b) in praktisch einem jeden Umfang, an der gleichen definierten Stelle oder an verschiedenen definierten Stellen der Oberfläche, jeweils unabhängig voneinander erfolgt. In der Regel wird die Wiederholung sooft erfolgen, bis an der jeweils definierten Stelle der Oberfläche die konkret herzustellende Verbindung, insbesondere das jeweilige Polymer, hergestellt ist. Insoweit handelt es sich bei den verschiedenen erfindungsgemäßen Verfahren in bevorzugten Ausführungen um Verfahren zur Synthese von Polymeren, insbesondere von Polypeptiden, wobei die zu immobilisierenden Verbindungen sodann Aminosäuren sind.

Im Rahmen der erfindungsgemäßen Verfahren werden unter anderem Kupplungsreagenzien verwendet. Kupplungsreagenzien, wie hierin verwendet, sind dabei solche Substanzen, welche die hierin offenbarten Reaktionen, insbesondere Ausbildung der verschiedenen Bindungstypen, ermöglichen.

Besonders bevorzugt sind dabei solche Kupplungsreagenzien, die zur Bindung einer Amidbindung führen, wie diese beispielsweise in Fig. 3 dargestellt ist.

Beispiele für die Vielzahl heute bekannter Kupplungsreagenzien sind:

*O*-(Benzotriazol-1-yl)-1,1,3,3-tetramethyluronium-hexafluorophosphat bzw. *N*-[(1*H*-Benzotriazol-1-yloxy)(dimethylamino)methylen]-*N*-methylmethanaminiumhexafluorophosphat bzw. *N*-[(1*H*-Benzotriazol-1-yl)-(dimethylamino)methylen]-*N*-methylmethanaminium-hexafluorophosphat-*N*-oxid bzw. 1-[Bis(dimethylamino)-methylen]-1*H*-benzotriazolium-hexafluorophosphat-3-oxid **(HBTU),** *O*-(Benzotriazol-1-yl)-1,1,3,3-tetramethyluronium-tetrafluoroborat bzw. *N*-[(1*H*-Benzotriazol-1-yloxy)(dimethylamino)methylen]-*N*-methylmethanaminium-tetrafluoroborat bzw. *N*-[(1*H*-Benzotriazol-1-yl)-(dimethylamino)methylen]-*N*-methylmethanaminium-tetrafluoroborat-*N*-oxid bzw. 1-[Bis(dimethylamino)-methylen]-1*H*-benzotriazolium-tetrafluoroborat-3-oxid **(TBTU),** 1-Benzotriazolyloxytris(pyrrolidino)phosphonium-hexafluorophosphat **(PyBOP),** 1-Benzotriazol-1-yloxy-bis(pyrrolidino)uronium-hexafluorophosphat **(BBC),** 5-(1*H*-Benzotriazol-1-yloxy)-3,4-dihydro-1-methyl-2*H*-pyrrolium-hexachloroanitimonat **(BDMP),** Benzotriazol-1-yloxy-*N*,*N*-dimethylmethaniminium-hexachloroantimonat **(BOMI),** *O*-(7-Azabenzotriazol-1-yl)-1,1,3,3-tetramethyluronium-hexafluorophosphat bzw. *N*-[(1*H*-1,2,3-Triazolo-[4,5-*b*]pyridin-1-yloxy)(dimethylamino)methylen]-*N*-methylmethanaminiumhexafluorophosphat bzw. *N*-[(dimethylamino)-1*H*-triazolo-[4,5-*b*]pyridin-1-yl-methylen]-*N*-methylmethanaminium-hexafluorophosphat-*N*-oxid bzw. 1-[Bis(dimethylamino)methylen]-1*H*-1,2,3-triazolo-[4,5-*b*]pyridinium-hexafluorophosphat-3-oxid **(HATU),** *O*-(7-Azabenzotriazol-1-yl)-1,1,3,3-bis(tetramethylen)uronium-hexafluorophosphat **(HAPyU),** *O*-(7-Azabenzotriazol-1-yl)-1,1,3,3-bis(pentamethylen)uronium-tetrafluoroborat **(TAPipU),** *O*-(7-Azabenzotriazol-1-yl)-tris(dimethylamino)phosphonium-hexafluorophosphat **(AOP),** Benzotriazol-1-yl-diethylphosphat **(BDP),** 1-Benzotriazolyoxytris(dimethylamino)phosphonium-hexafluorophosphat (Castro's Reagenz, **BOP),** 7-Azobenzotriazolyoxytris(pyrrolidino)phosphonium-hexafluorophosphat **(PyAOP),** 2-(3,4-Dihydro-4-oxo-1,2,3-benzotriazin-3-yl)-1,1,3,3-tetramethyluronium-tetrafluoroborat **(TDBTU),** 2-(5-Norbornen-2,3-dicarboximido)-1,1,3,3-tetramethyluronium-tetrafluoroborat **(TNTU),** 2-(2-Oxo-1(2*H*)-pyridyl-1,1,3,3-tetramethyluronium-tetrafluoroborat **(TPTU),** 2-Succinimido-1,1,3,3-tetramethyluronium-tetrafluoroborat **(TSTU),** 2-Brom-3-ethyl-4-methyl thiazolium-tetrafluoroborat **(BEMT),** Bis(2-oxo-3-oxazolidinyl)phosphionchlorid **(BOP-Cl),** Bromtris(dimethylamino)phosphonium-hexafluorophosphat **(BroP),** Bis(tetramethylenfluoroformamidinium)-hexafluorophosphat **(BTFFH),** 2-Chlor-1,3-dimethylimidazolidinium-hexafluorophosphat **(CIP),** 3-(Diethoxyphosphoryloxy)-1,2,3-benzotriazin-4(3*H*)-on **(DEPBT),** Diphenylphosphionchlorid **(Dpp-Cl),** Diphenylphosphorsäure-Azid **(DPPA),** 2-Ethoxy-1-ethoxycarbonyl-1,2-dihydroquinolin **(EEDQ),** Pentafluorphenyl-diphenylphosphinat **(FDPP),** S-(1-Oxido-2-pyridinyl)-1,1,3,3-tetramethylthiouronium-hexafluorophosphat **(HOTT),** Bromtris(pyrrolydino)phosphoniumhexafluorophosphat **(PyBroP),** Chlortris(pyrrolydino)phosphonium-hexafluorophosphat **(PyCloP),** Tetramethylfluoroformamidinium-hexafluorophosphat **(TFFH),** S-(1-Oxido-2-pyridinyl)-1,1,3,3-tetramethylthiouronium-tetrafluoroborat **(TOTT),** 1,1'-Carbonyldiimidazol **(CDI),** 1-(Mesitylen-2-sulfonyl)-3-nitro-1H-1,2,4-triazol **(MSNT),** Di-(N-Succinimidyl)carbonate bzw. N,N'-Disuccinimidyl-carbonat, **(DSC),** Chlor-N,N,N',N'tetramethylformamidinium-hexafluorophosphat, 1-Isobutoxycarbonyl-2-isobutoxy-1,2-dihydroquinolin **(IIDQ;** N-Isobutoxycarbonyl-2-isobutoxy-1,2-dihydroquinon), 2-Chlor-1,3-dimethylimidazolidinium-hexafluorophosphat **(CIP),** 2-Chlor-1,3-dimethylimidazolidiniumtetrafluoroborat **(CIB),** 2-Chlor-1-methylpyridinium-iodid, 2-Ethyl-1,2-benzisoxazoliumtetrafluoroborat, 2-Morpholinoethyl-isocyanid (**MEI**, 4-(2-Isocyanoethyl)morpholin), Chlor-dipiperidinocarbenium-hexafluorophosphat (**PipClU**, Chlor-N,N,N',N'bis(pentamethylen)formamidinium-hexafluorophosphat), Diethylcyanophosphonat (DEPC, Diethylphosphorocyanidat; Diethylphosphorylcyanid), Diphenyl-N-succinimidylphosphat **(SDPP),** O-(1,2-Dihydro-2-oxo-1-pyridyl)-N,N,N',N'-bis(tetramethylen)uroniumhexafluorophosphat (N,N,N',N'-Bis(tetramethylen)-O-(1,2-dihydro-2-oxo-1-pyridyl)uroniumhexafluorophosphat), O-[(Ethoxycarbonyl)cyanomethylenamino]-N,N,N',N'tetramethyluronium-tetrafluoroborat (TOTU), Propylphosphonanhydrid-Lösung (in DMF: ~50 %, 1-Propanphosphon-Anhydrid; 2,4,6-Tripropyl-1,3,5,2,4,6-trioxatriphosphorinan-2,4,6-trioxid), 2-Ethyl-5-phenylisoxazolium-3'-sulfonat **(Woodwards-Reagens),** Benzyltriphenylphosphonium-dihydrogen-trifluorid **(BTF)**, Propylphosphonsäureanhydrid **(PPAA),** Diphenylphosphinsäureanhydrid **(Dpp, Dppa),** 4-(4,6-Dimethoxy[1.3.5]triazin-2-yl)-4-methylmorpholiniumchlorid-hydrat **(DMTMM),** 5-(1*H*-7-azabenzotriazol-1-yloxy)-3,4-dihydro-1-methyl 2*H*-pyrrolium-hexachloroantimonat **(AOMP),** 2-Brom-1-ethyl-pyridiniumtetrafluoroborat **(BEP),** 2-Brom-1-ethyl-pyridinium-hexachloroantimonat **(BEPH),** 1-(1*H*-Benzotriazol-1-yloxy)phenylmethylen-pyrrolidinium-hexachloroantimonat **(BPMP),** Chlor (4-morpholino)methylen-morpholinium-hexafluorophosphat **(CMMM),** 2-Fluor-1-ethylpyridinium-tetrafluoroborat **(FEP),** 2-Fluor-1-ethyl-pyridinium-hexachloroantimonat **(FEPH),** *O*-(1*H*-Benzotriazol-1-yl)-*N*,*N*,*N*',*N*'-bis(pentamethylen)uronium-hexafluorophosphat **(HBPipU),** *O*-(1*H*-Benzotriazol-1-yl)-*N*,*N*,*N*',*N*'-bis(tetramethylen)uroniumhexafluorophosphat **(HBPyU),** 5-(3',4'-Dihydro-4'-oxo-1',2',3'-benzotriazin-3'-yloxy)-3,4-dihydro-1-methyl-2*H*-pyrrolium-hexachloroantimonat **(DOMP),** 5-(Pentafluorphenyloxy)-3,4-dihydro-1-methyl-2*H*-pyrrolium-hexachloroantimonat **(FOMP),** (1*H*-Benzotriazol-1-yloxy)-1,3-dimethyl-1,3-dimethylenuronium-hexafluorophosphat **(HBMDU),** 5-(Succinimiddyloxy)-3,4-dihydro-1-methyl-2*H*-pyrrolium-hexachloroantimonat **(SOMP),** α-Chlorvinyl-ethylether, α,α-Dichlor-diethylether, Ethoxyacetylen, Diphenylketen-p-Tolylimin, Diphenylketen, 2-Methyl-4,5,6,7-tetrahydrobenzo[*d*]-1,2-oxazolium-tetrafluoroborat, 2-Ethyl-4,5,6,7-tetrahydrobenzo[*d*]-1,2-oxazolium-tetrafluoroborat, N,N'-Dicyclohexylcarbodiimid **(DCC, DCCI),** 1-Ethyl-3-(3'-dimethylaminopropyl)carbodiimid·HCl **(EDC·HCl, WSC·HCl),** Diisopropylcarbodiimid (**DIC**) und Thionylchlorid.

Die verschiedenen, erfindungsgemäß verwendbaren Kupplungsreagenzien lassen sich zu bestimmten Untergruppen zuordnen. Eine wichtige Gruppe stellt dabei die der Carbodiimide **(X3)** dar, welche bei Peptidkupplungen unter $H_2O$-Aufnahme zu Harnstoffen reagieren. Die Formel **X3** zeigt ein derartiges Carbodiimid.

$$R^1\text{-N=C=N-}R^2 \qquad \textbf{X3}$$

$R^1$, $R^2$ = Alkyl, Cycloalkyl, substituierte Alkyl, substituierte Cycloalkyl. Wie hierin allgemein verwendet, bezeichnet dabei Alkyl ein jedes geradkettiges oder verzweigtkettiges Alkan mit einer Kettenlänge von C1 bis C12. Dabei sind Kettenlängen von C1 bis C6 bevorzugt.

Weiterhin ist es im Rahmen der vorliegenden Erfindung, dass der Alkylrest ein Cycloalkyl ist. Bevorzugterweise besteht das Cycloalkyl drei bis 12 Kohlenstoffatomen (C3 bis C12), bevorzugtererweise aus fünf bis sieben Kohlenstoffatomen (C5 bis C7). Wie hierin verwendet bezeichnet C1 bis C12 eine jede Anzahl von Kohlenstoffatomen von 1 bis 12, d.h. 1, 2, 3, 4, 5, 6, 7, 8, 9, 10, 11 und 12. Das gleiche gilt sinngemäß für die entsprechenden anderen Zahlenbereiche.

Beispiele für erfindungsgemäß verwendbare Carbodiimide sind:

N,N'-Dicyclohexylcarbodiimid **(DCC, DCCI),** 1-Ethyl-3-(3'dimethylaminopropyl)carbodiimid·HCl **(EDC·HCl, WSC·HCl)** und Diisopropylcarbodiimid (**DIC**).

An Stelle der Carbodiimide werden bevorzugterweise Aminiumsalze **(X1)** und Phosphoniumsalze **(X2)** verwendet.

**Aminium-Salze** **Phosphonium-Salze**

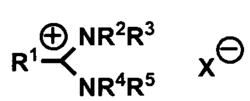

**X1**

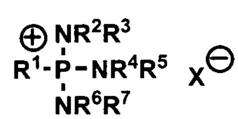

**X2**

**$R^1$ = OAlkyl, OAryl, OHeteroaryl, Halogenyl, Alkyl, Aryl, Heteroaryl, SHeteroaryl**

**$R^2$ - $R^7$ = Alkyl, Aryl, Heteroaryl**

**$X^-$ = anorg. oder org. Anion**

(anorg. = anorganisch; org. = organisch)

In Ausführungsformen der verschiedenen Salze ist vorgesehen, dass die Reste R2 und R4 und/oder R3 und R5 zur Ausbildung einer Ringbildung befähigt sind und insoweit ein heteroaromatischer Ring ausgebildet wird. Ebenfalls ist es möglich, daß R2 und R3 und/oder R4 und R5 einen Ring bilden können, wodurch ebenfalls ein heteroaromatischer Ring ausgebildet wird.

Ein wichtiger Grund dafür, dass sich Aminium- und Phosphoniumsalze als Kupplungsreagenzien in der Peptidsynthese weitgehend durchgesetzt haben, ist das relativ ausgewogene Verhältnis zwischen Aktivität und Stabilität - zwei Eigenschaften, die in der Regel gegenläufig sind.

So sind die Lösungen vieler Aminium- und Phosphoniumreagenzien in Lösung für mehrere Stunden bis Tage stabil [F. Albericio et al., *J. Org. Chem*. **1998,** *63*, 9678-9683]. Gleichzeitig führen sie aber auch zu guten Ausbeuten bei Kupplungen [L. A: Carpino et al., *J. Chem. Soc. Chem. Commun*. **1994**, 201-203]. Besonders bevorzugs bei den Aminium- und Phosphoniumreagenzien sind HBTU **(X3),** TBTU **(X4)** und PyBOP **(X5),** weil diese relativ preiswert kommerziell erhältlich sind. Desweiteren enthalten diese Verbindungen als Strukturelement ein Oxy-Benzotriazol, so dass bei deren Reaktion mit Carbonsäuren oder Wasser der Zuschlagstoff HOBt entsteht, welcher sich z.B. als Racemisierungssuppressierender Stoff bewährt hat.

Anhand des Beispiels HBTU lassen sich sehr positive Ergebnisse erklären. Der allgemein akzeptierte Mechanismus der Peptid-Bindungs-Bildung ist dargestellt in Fig. 4. Bei extrem kurzen Voraktivierungszeiten, wie sie im Fall der vorliegenden Erfindung vorliegen, kann ein größerer Teil der Peptidbindung über das Intermediat **X6** von Fig. 4 gebildet werden. Im Falle der Mesitylsäure ist **X6** reaktiver als **X7** (OAt-Ester) [L. A. Carpino et al., *Angew. Chem.* **2002,** *114*, 458-461]. Dieser Unterschied in den Aktivspecies gegenüber den bisher bekannten Verfahren kann eine Reihe von positiven Effekten auf den Kupplungsprozeß haben: Racemisierung kann ausgehend von X6 geringer sein als ausgehend von X7 von Fig. 4, die Reaktion kann schneller verlaufen und die Ausbeuten können erhöht sein.

Eine ähnliche Erklärung des Phänomens, dass durch Aktivierung auf der Membran bessere Ausbeuten erzielt werden können als durch Voraktivierung in Lösung, liegt in den zwei möglichen Isomeren von X7 begründet. Man vermutet, daß bei Aktivierungen mit HBTU zunächst das Intermediat **X7a** entsteht, das anschließend zu **X7b** isomerisiert [L. A. Carpino et al., *Angew. Chem.* **2002,** *114*, 458-461]. Da **X7a** reaktiver ist als **X7b** [K. Barlos et al., *Int. J. Pept. Prot. Res.* **1984,** *23*, 300], können bei kurzen im Vergleich zu langen Voraktivierungszeiten höhere Kupplungsausbeuten, schnellere Reaktionen und geringere Ausmaße an Racemisierung erzielt werden. Die geringste prinzipiell mögliche Voraktivierungszeit wird durch das erfindungsgemäße Verfahren der Aktivierung auf der Membran erreicht.

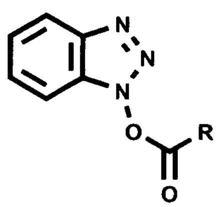

**X7a**

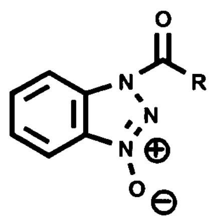

**X7b**

Ohne die Einwirkung von Basen findet mit HBTU keine Aktivierung einer Carbonsäure statt, wie anhand der NMR-Spektren in Fig. 7 deutlich wird. Das erklärt die hohe Stabilität von Lösungen aus HBTU und Fmoc-AS-OH in DMF, die selbst bei eintägigem Stehenlassen an der Luft noch durchschnittlich ca. 70% ihrer Ursprungsaktivität besitzen (vgl. Fig. 8).

Phosphonium-Reagenzien reagieren nach einem sehr ähnlichen Mechanismus wie Aminium-Reagenzien, wobei der Mechanismus in Fig. 5 beschrieben ist.

Bei extrem kurzen Voraktivierungszeiten, wie sie im Fall der beschriebenen Erfindung vorliegen, kann ein größerer Teil der Peptidbindung über das Intermediat X8 gebildet werden. Dieser Unterschied in den Aktivspecies gegenüber den bisher bekannten Verfahren weist eine Reihe von positiven Effekten auf den Kupplungsprozess auf: die Racemisierung ist ausgehend von X8 geringer als ausgehend von **X9**, die Reaktion verläuft schneller und die Ausbeuten sind erhöht.

Beispiele für Aminium- und Phosphonium-Kupplungsreagenzien, wie sie in den erfindungsgemäßen Verfahren verwendet werden können, sind:

*O*-(Benzotriazol-1-yl)-1,1,3,3-tetramethyluronium-hexafluorophosphat bzw. *N*-[(1*H*-Benzotriazol-1-yloxy)(dimethylamino)methylen]-*N*-methylmethanaminiumhexafluorophosphat bzw. *N*-[(1*H*-Benzotriazol-1-yl)-(dimethylamino)methylen]-*N*-methylmethanaminium-hexafluorophosphat-*N*-oxid bzw. 1-[Bis(dimethylamino)-methylen]-1*H*-benzotriazolium-hexafluorophosphat-3-oxid **(HBTU),** *O*-(Benzotriazol-1-yl)-1,1,3,3-tetramethyluronium-tetrafluoroborat bzw. *N*-[(1*H*-Benzotriazol-1-yloxy)(dimethylamino)methylen]-*N*-methylmethanaminium-tetrafluoroborat bzw. *N*-[(1*H*-Benzotriazol-1-yl)-(dimethylamino)methylen]-*N*-methylmethanaminium-tetrafluoroborat-*N* oxid bzw. 1-[Bis(dimethylamino)-methylen]-1*H*-benzotriazolium-tetrafluoroborat-3-oxid **(TBTU),** 1-Benzotriazolyloxytris(pyrrolidino)phosphonium-hexafluorophosphat **(PyBOP),** 1-Benzotriazol-1-yloxy-bis(pyrrolidino)uronium-hexafluorophosphat **(BBC),** *O*-(7-Azabenzotriazol-1-yl)-1,1,3,3-tetramethyluronium-hexafluorophosphat bzw. *N*-[(1*H*-1,2,3-Triazolo-[4,5-*b*]pyridin-1-yloxy)(dimethylamino)methylen]-*N*-methylmethanaminiumhexafluorophosphat bzw. *N*-[(dimethylamino)-1*H*-triazolo-[4,5-*b*]pyridin-1-yl-methylen]-*N*-methylmethanaminium-hexafluorophosphat-*N*-oxid bzw. 1-[Bis(dimethylamino)methylen]-1*H*-1,2,3-triazolo-[4,5-*b*]pyridinium-hexafluorophosphat-3-oxid **(HATU),** *O*-(7-Azabenzotriazol-1-yl)-1,1,3,3-bis (tetramethylen)uronium-hexafluorophosphat **(HAPyU),** *O*-(7-Azabenzotriazol-1-yl)-1,1,3,3-bis(pentamethylen)uronium-tetrafluoroborat **(TAPipU),** *O*-(7-Azabenzotriazol-1-yl)-tris(dimethylamino)phosphonium-hexafluorophosphat **(AOP),** 1-Benzotriazolyoxytris(dimethylamino)phosphonium-hexafluorophosphat (Castro's Reagenz, **BOP),** 7-Azobenzotriazolyoxytris(pyrrolidino)phosphonium-hexafluorophosphat **(PyAOP),** 2-(3,4-Dihydro-4-oxo-1,2,3-benzotriazin-3-yl)-1,1,3,3-tetramethyluronium-tetrafluoroborat **(TDBTU),** 2-(5-Norbornen-2,3-dicarboximido)-1,1,3,3-tetramethyluronium-tetrafluoroborat **(TNTU),** 2-(2-Oxo-1(2*H*)-pyridyl-1,1,3,3-tetramethyluronium-tetrafluoroborat **(TPTU),** 2-Succinimido-1,1,3,3-tetramethyluronium-tetrafluoroborat **(TSTU),** Bromtris(dimethylamino)phosphonium-hexafluorophosphat **(BroP),** Bis(tetramethylenfluorformamidinium)-hexafluorophosphat **(BTFFH),** *S*-(1-Oxido-2-pyridinyl)-1,1,3,3-tetramethylthiouronium-hexafluorophosphat **(HOTT),** Bromtris(pyrrolydino)phosphonium-hexafluorophosphat **(PyBroP),** Chlortris(pyrrolydino)phosphonium-hexafluorophosphat **(PyCloP),** Tetramethylfluorformamidinium-hexafluorophosphat **(TFFH),** Chlor-N,N,N',N'tetramethylformamidinium-hexafluorophosphat, 2-Chlor-1,3-dimethylimidazolidiniumhexafluorophosphat **(CIP),** 2-Chlor-1,3-dimethylimidazolidinium-tetrafluoroborat **(CIB),** Chlor-dipiperidinocarbenium-hexafluorophosphat **(PipCIU,** Chlor-N,N,N',N'bis(pentamethylen)formamidinium-hexafluorophosphat), O-(1,2-Dihydro-2-oxo-1-pyridyl)-N,N,N',N'-bis(tetramethylen)uronium-hexafluorophosphat (N,N,N',N'-Bis(tetramethylen)-O-(1,2-dihydro-2-oxo-1-pyridyl)uronium-hexafluorophosphat), Chlor(4-morpholino)methylenmorpholinium-hexafluorophosphat **(CMMM),** *O*-(1*H*-Benzotriazol-1-yl)-*N*,*N*,*N'*,*N'*bis(pentamethylen)uronium-hexafluorophosphat **(HBPipU)** und *O*-(1*H*-Benzotriazol-1-yl)-*N*,*N*,*N'*,*N'*-bis(tetramethylen)uronium-hexafluorophosphat **(HBPyU).**

Von den Aminium- und Phosphonium-Kupplungsreagenzien werden in den erfindungsgemäßen Verfahren besonders bevorzugt jene Reagenzien verwendet, die auf Hydroxybenzotriazolen wie HOBt **(X6)** und HOAt (**X7**) basieren.

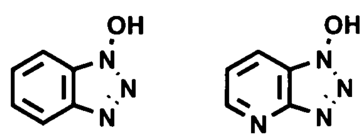

**HOBt (X6)** **HOAt (X7)**

Beispiele hierfür sind:

*O*-(Benzotriazol-1-yl)-1,1,3,3-tetramethyluronium-hexafluorophosphat bzw. *N*-[(1*H*-Benzotriazol-1-yloxy)(dimethylamino)methylen]-*N*-methylmethanaminiumhexafluorophosphat bzw. *N*-[(1*H*-Benzotriazol-1-yl)-(dimethylamino)methylen]-*N*-methylmethanaminium-hexafluorophosphat *N*-oxid bzw. 1-[Bis(dimethylamino)-methylen]-1*H*-benzotriazolium-hexafluorophosphat-3-oxid (**HBTU**), *O*-(Benzotriazol-1-yl)-1,1,3,3-tetramethyluronium-tetrafluoroborat bzw. *N*-[(1*H*-Benzotriazol-1-yloxy)(dimethylamino)methylen]-*N*-methylmethanaminium-tetrafluoroborat bzw. *N*-[(1*H*-Benzotriazol-1-yl)-(dimethylamino)methylen]-*N*-methylmethanaminium-tetrafluoroborat-*N*-oxid bzw. 1-[Bis(dimethylamino)-methylen]-1*H*-benzotriazolium—tetrafluoroborat-3-oxid **(TBTU),** 1-Benzotriazolyloxytris(pyrrolidino)phosphonium-hexafluorophosphat **(PyBOP),** 1-Benzotriazol-1-yloxy-bis(pyrrolidino)uronium-hexafluorophosphat **(BBC),** *O*-(7-Azabenzotriazol-1-yl)-1,1,3,3-tetramethyluronium-hexafluorophosphat bzw. *N*-[(1*H*-1,2,3-Triazolo-[4,5-*b*]pyridin-1-yloxy)(dimethylamino)methylen]-*N*-methylmethanaminiumhexafluorophosphat bzw. *N*-[(dimethylamino)-1*H*-triazolo-[4,5-*b*]pyridin-1-yl-methylen]-*N*-methylmethanaminium-hexafluorophosphat-*N*-oxid bzw. 1-[Bis(dimethylamino)methylen]-1*H*-1,2,3-triazolo-[4,5-*b*]pyridinium-hexafluorophosphat-3-oxid **(HATU),** *O*-(7-Azabenzotriazol-1-yl)-1,1,3,3-bis (tetramethylen)uronium hexafluorophosphat **(HAPyU),** *O*-(7-Azabenzotriazol-1-yl)-1,1,3,3-bis(pentamethylene)uronium-tetrafluoroborat **(TAPipU),** *O*-(7-Azabenzotriazol-1-yl)-tris(dimethylamino)phosphonium-hexafluorophosphat **(AOP),** 1-Benzotriazolyoxytris(dimethylamino)phosphonium-hexafluorophosphat (Castro's Reagenz, **BOP),** 7-Azobenzotriazolyoxytris(pyrrolidino)phosphonium-hexafluorophosphat **(PyAOP),** *O*-(1*H*-Benzotriazol-1-yl)-*N*,*N*,*N'*,*N'*-bis (pentamethylene)uronium-hexafluorophosphat **(HBPipU)** und *O*-(1*H*-Benzotriazol-1-yl)-*N*,*N*,*N'*,*N'*-bis(tetramethylene) uroniumhexafluorophosphat **(HBPyU).**

Im Rahmen der vorliegenden Erfindung ganz besonders bevorzugt sind HBTU **(X3),** TBTU **(X4)** und PyBOP

**(X5).** Für HBTU sind in der Vergangenheit zwei chemische Strukturen beschrieben worden. Anfänglich wurde Struktur B vorgeschlagen [V. Dourtoglou et al., *Synthesis* **1984,** 572], später Struktur A [I. Abdelmoty et al., *Lett. Pept. Sci.* **1994,** *1*, 52], und seit kurzem ist bekannt, dass beide Strukturen existieren [L. A. Carpino et al., *Angew. Chem.* **2002,** *114*, 458-461]. Im Rahmen der vorliegenden Erfindung schließt der Name HBTU ausdrücklich beide möglichen Isomere ein. Das gleiche gilt für dem HBTU strukturverwandte Verbindungen.

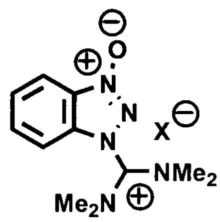

**A**

**bzw.**

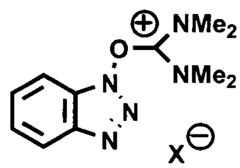

**B**

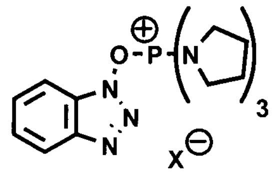

**$X^-$ = $PF6^-$: HBTU (X3)**
**$X^-$ = $BF4^-$: TBTU (X4)**

**PyBOP (X5)**

Der Kupplungsprozess wird häufig durch den Zusatz von Basen positiv beeinflusst. Basen, wie sie gemäß der erfindungsgemäßen Verfahren verwendet werden können, sind auch allgemein solche gemäß der Definition von Brönsted-Verbindungen, die als Protonen-Akzeptor wirken können [A. F. Hollemann, N. Wiberg, *Lehrbuch der Anorganischen Chemie*, Walter de Gruyter, New York, **1985**, S.236]. Diese Eigenschaft ist insbesondere bei der Bildung von Amidbindungen günstig bzw. von Vorteil, da der gewünschte Kupplungsschritt häufig durch die Abstraktion eines Protons von der Carboxylgruppe der Carbonsäure initiiert wird (vgl. Fig. 3).

Beispiele für in den erfindungsgemäßen Verfahren verwendbare Basen sind:

Tertiäre Amine wie z.B. Diisopropylethylamin (DIEA, DIPEA), Trimethylamin, Triethylamin (TEA), Triisopropylamin, N-Methyl-piperidin, N-Methyl-morpholin (NMM), N-Ethylmorpholin, N-tBu-morpholin;

Pyridin-Derivate wie z.B. Pyridin, Dimethylaminopyridin (DMAP), 2,4,6-Trimethylpyridin (TMP, Collidin), 2,6-Dimethylpyridin (2,6-Lutidin), 2,6-Di-*tert*-butyl-4-(dimethylamino)pyridin (DB(DMAP)), 2,3,5,6-Tetramethylpyridin (TEMP), Octahydroacridin (OHA), 2,6-Dimethyl-4-(dimethylamino)pyridin (2,6-diMe-DMAP), 2,3,4,5,6-Pentamethylpyridin, 2,6-Diethyl-4-methylpyridin, 2,6-Diisopropyl-4-methylpyridin;

Imidazol-Derivate wie z.B. N-Methyl-Imidazol (NMI),

oder organische oder anorganische Salze wie z.B. 1-Oxy-benzotrizolyl-kalium (KOBt), Natriumhydroxid (NaOH), Kaliumhydroxid (KOH).

Bei der Durchführung der erfindungsgemäßen Verfahren, bei der es zur Ausbildung von Amidbindungen kommt, können als Basen verwendet werden:

Tertiäre Amine wie z.B. Diisopropylethylamin (DIEA, DIPEA), Trimethylamin, Triethylamin (TEA), Triisopropylamin, N-Methyl-piperidin, N-Methyl-morpholin (NMM), N-Ethylmorpholin, N-tBu-morpholin;

Pyridin-Derivate wie z.B. Pyridin, Dimethylaminopyridin (DMAP), 2,4,6-Trimethylpyridin (TMP, Collidin), 2,6-Dimethylpyridin (2,6-Lutidin), 2,6-Di-*tert*-butyl-4-(dimethylamino)pyridin (DB(DMAP)), 2,3,5,6-Tetramethylpyridin (TEMP), Octahydroacridin (OHA), 2,6-Dimethyl-4-(dimethylamino)pyridin (2,6-diMe-DMAP), 2,3,4,5,6-Pentamethylpyridin, 2,6-Diethyl-4-methylpyridin, 2,6-Diisopropyl-4-methylpyridin;

Imidazol-Derivate wie z.B. N-Methyl-Imidazol (NMI).

Im Rahmen der vorliegenden Erfindung werden als Basen besonders bevorzugt verwendet:

Diisopropylethylamine (DIEA, DIPEA), N-Methyl-morpholin (NMM), Pyridin, Dimethylaminopyridin (DMAP), N-Methyl-Imidazol (NMI).

Im Rahmen der verschiedenen erfindungsgemäßen Verfahren und deren Ausführungsformen können ggf. Zuschlagsstoffe verwendet werden, insbesondere können Zuschlagsstoffe zusätzlich zum Kupplungsreagens bzw. zur immobilisierenden Verbindung zugesetzt werden.

Zuschlagstoffe, wie hierin verwendet, sind Verbindungen, die geeignet sind, den gewünschten Kupplungsprozess positiv zu beeinflussen. Geeignete Zuschlagstoffe können von den Fachleuten auf dem Gebiet auch im Rahmen von Routinetests hinsichtlich ihrer Eignung zur Verwendung in einem der erfindungsgemäßen Verfahren ermittelt bzw. bestimmt werden.

So ist z.B. bekannt, dass der Zuschlagstoff HOAt, der zur Gruppe der Benzotriazole gehört, bei Aminosäurekupplungen mit dem Kupplungsreagenz EDC zur Verringerung unerwünschter Racemisierung der Aminosäure führt [Carpino et al., *J. Am. Chem. Soc.* **1993,** *115*, 4397-4398]. Der gleiche Effekt ist auch mit einer Reihe anderer Kupplungsreagenzien beobachtet worden; z.B. bei HAPyU mit HOAt [F. Albericio et al. in *Peptides 2000*, J. Martinez, J.-A. Fehrenz, Eds., EDK, Paris, **2000,** S. 23-24] oder DIC mit 6-Cl-HOBt [F. Albericio et al. in *Peptides 2000*, J. Martinez, J.-A. Fehrenz, Eds., EDK, Paris, **2000,** S. 287-288].

Der Mechanismus, der den positiven Effekten von Zuschlagstoffen zugrunde liegt, ist nicht immer bekannt. Bei dem Zuschlagstoff HOAt mit Carbodiimiden als Kupplungsreagenzien geht man aber davon aus, daß das HOAt zunächst einen Komplex **X10** mit der Aminosäure eingeht, der durch Wasserstoffbrückenbindungen mit einem Amin (=Base) stabilisiert wird, und dadurch zu verminderter Racemisierung führt.

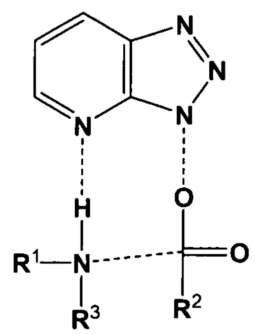

**X10**

Neben HOAt sind auch andere Zuschlagstoffe beschrieben worden, die im Rahmen der vorliegenden Erfindung verwendet werden können. So ist beispielsweise bekannt, dass Kupplungen mit dem Kupplungsreagenz DCC durch den Zusatz des Zuschlagstoffes HOBt zu verminderter Racemisierung und höheren Ausbeuten an gewünschtem Peptid führen [W.

König et al., *Chem. Ber.* **1970,** 103, 788]. Insoweit ist die Verwendung von DCC und HOBt im Rahmen der vorliegenden Erfindung besonders bevorzugt.

Ein weiteres Beispiel für den positiven Einfluss von Zuschlagstoffen auf Kupplungen ist die Verbesserung der Reinheit bei der PyBOP-vermittelten Synthese eines Decapeptides durch den Zuschlagstoff HOAt [F. Albericio et al., *Tetrahedron Lett.* **1997,** *38*, 4853-4856]. Beim gleichen Peptid führte der Zuschlagstoff HOBt zu einer verbesserten Ausbeute bei der Synthese mit dem Kupplungsreagenz HPyOPfp [A. El-Faham, *Lett. Pept. Sci.* **2000,** *7*, 113-121]. Insoweit ist die Verwendung von HPyOPfp und HOAt bzw. HOBt im Rahmen der vorliegenden Erfindung besonders bevorzugt.

Die Zuschlagstoffe HOBt, Pentachlorphenol und 2,4-Dinitrophenol sind geeignet, bei Peptidkupplungen die unerwünschte basenkatalysierte Bildung von Aminosuccinyl-Peptiden aus Aspartyl-Peptiden zu verringern. Der zugrundeliegende Mechanismus ist hier nicht bekannt, es wird aber vermutet, dass die schwach sauren Verbindungen verwendete Base abpuffern [J. Martinez et al., *Int. J. Pept. Prot. Res.* **1978,** 277].

Auch einfache anorganische Salze wie LiCl, $NaClO_4$ oder KSCN können als Zuschlagstoffe positive Effekte auf Peptidkupplungen haben. Man geht hier davon aus, dass diese Zuschlagstoffe zu einer verminderten unerwünschten Aggregation von Peptidketten führt. Das führt im Endeffekt zu erhöhten Reinheiten der synthetischen Peptide. Man nennt die hier verwendeten Salze auch chaotrope Salze [Albericio et al, *Methods Enzymol.* **1997,** *289*, 104-126].

Zuschlagstoffe, die im Rahmen der vorliegenden Erfindung beispielhaft verwendet werden können, sind unter anderem 1-Hydroxy-7-azabenzotriazol **(HOAt),** 1-Hydroxybenzotriazol **(HOBt),** 1-Hydroxybenzotriazol·$H_2O$ **(HOBt·$H_2O$),** 6-Chlor-1-hydroxybenzotriazol **(6-Cl-HOBt),** Ethyl-1-hydroxy-1H-1,2,3-triazol-4-carboxylat **(HOCt),** N-Hydroxy-5-norbornenendo-2,3-dicarboximid **(HONB),** N-Hydroxysuccinimid **(NHS, HOSu),** 3-Hydroxy-3,4-dihydro-4-oxo-1,2,3-benzotriazin **(HODhbt, HOOBt),** Pentafluorphenol **(HOPfp),** Lithiumchlorid **(LiCl),** Natriumperchlorat **(NaClO4),**

Kaliumrhodanid **(KSCN),** Kupfer(II)chlorid **($CuCl_2$)** oder andere organische oder anorganische Salze oder andere Reagenzien, die geeignet sind, den Kupplungsprozess positiv zu beeinflussen.

Die Zuordnung eines Stoffes zu den Gruppen Zuschlagstoff und Base ist manchmal ambivalent. So kann z. B. die Base KOBt [K. M. Sivanandaiah et al., *Int. J. Pept. Prot. Res.* **1994,** *44*, 24-30] auch als Zuschlagstoff angesehen werden, da bei Kupplungen mit KOBt, bei denen Säuren freigesetzt werden, der Zuschlagstoff HOBt entsteht. Insoweit können die hierin als Zuschlagstoff angeführten Verbindungen die Funktion einer Base wahrnehmen und somit als Base im Rahmen der erfindungsgemäßen Verfahren verwendet werden und Basen als Zuschlagstoffe, zumindest jeweils in bestimmtem Umfang. Es ist somit auch im Rahmen der vorliegenden Erfindung, dass die verschiedenen Stoffe, wie Kupplungsreagens, Zuschlagstoff und Base, erst durch Reaktion anderer Verbindungen, insbesondere anderer der vorstehend genannten Gruppen vor oder während der eigentlichen Reaktion entstehen und dann die entsprechenden Eigenschaften oder Funktionen oder Wirkungen, die der jeweiligen Gruppe von Verbindungen zugeschrieben wird, zeigen.

Im Rahmen der erfindungsgemäßen Verfahren ist ein Trocknungsschritt vorgesehen, wobei es bei der Trocknung der behandelten Oberfläche oder eines Teils davon generell günstig ist, zwischen zwei oder mehreren der Schritte zu trocknen. Bevorzugterweise erfolgt eine Trocknung zumindest vor dem zeitlich jeweils letzten Schritt. Das hat den Vorteil, dass nach dem letzten Schritt, im Anschluss dessen die Aktivierung und Kupplung der zu immobilisierenden Verbindung an die immobilisierte Gruppe erfolgt, eine möglichst hohe Konzentration an aktiviertem Derivat erreicht werden kann. Höhere Konzentrationen an Reaktionspartnern führen in der Regel zu höheren Reaktionsgeschwindigkeiten. Wird dagegen nicht getrocknet, so führt das in den ersten Schritten verwendete Lösungsmittel zu kleineren Konzentrationen an letztendlich benötigtem Aktivderivat.

Bevorzugte Ausführungsformen der erfindungsgemäßen Verfahren sehen vor, dass das Trocknen zwischen der Auftragung von Kupplungsreagenz und einem Verbindungs-Basen-Gemisch und/oder zwischen der Auftragung eines Verbindungs-Kupplungsreagenz-Gemisches und einer Base erfolgt.

Im Rahmen der erfindungsgemäßen Verfahren ist auch ein Waschschritt vorgesehen, bei dem die Oberfläche von Agenzien befreit wird bzw. deren Umfang auf der Oberfläche verringert wird. Dabei wird ein Lösungsmittel verwendet.

Bevorzugterweise erfolgt ein Waschschritt nach Beendigung der gewünschten chemischen Reaktion vor der Durchführung einer neuen Reaktion. Damit wird verhindert, dass während der zweiten Reaktion Reagenzien von der ersten Reaktion stören. Zur praktischen Ausführung kann die Oberfläche in einer Schale mit Lösungsmittel überschüttet und dieses geschüttelt werden.

Grundsätzlich sind alle Lösungsmittel zum Waschen der Oberfläche geeignet. Bevorzugt werden jedoch die folgenden Lösungsmittel in den erfindungsgemäßen Verfahren verwendet:

N,N-Dimethylformamid (DMF), 1-Methyl-2-pyrrolidinon (NMP), N,N-Dimethylacetamid (DMA), Dimethylsulfoxid (DMSO), Tetrahydrofuran (THF), Dioxan, Acetonitril (MeCN, ACN), Methanol (MeOH), Ethylacetat (EE), 2,2,2-Trifluorethanol (TFE), 1,1,1,3,3,3-Hexafluor-2-propanol (Hexafluoroisopropanol, HFIP), 1,2-Dibromethan, Dichlormethan (DCM), Diethylether, Aceton, 1,2-Dimethoxyethan (DME), Bis(2-Methoxyethyl)ether (Diglyme), 1,3-Dimethyl-2-imidazolidinon, 1,3-Dimethyl-3,4,5,6-tetrahydro-2(1H)-pyrimidinon, Hexamethylphosphorsäureamid (HMPA), Dimethoxyethan, Nitromethan, Nitrobenzol, Propylacetat, Wasser, Chloroform, 1,2-Dichlorethan, 1,3-Dichlorpropan, Toluol, Benzol, Xylol, 1-Butanol, 2-Butanol, Petrolether, Cyclohexan, Cyclopentan, tert-Butylmethylether, Ethoxyethanol, Pentan, Hexan, Heptan, Octan, Methylethylketon, Diethylketon, 1,2-Dichlorbenzol, Kohlenstofftetrachlorid, 1-Chlorbutan, Diisopropylether, 1,1,1-Trichlorethan, 1,1,2-Trichlortrifluorethan, 1-Butylacetat, Tetrachlorethylen, Trichlorethylen, Trimethylpentan, 2-Methylbutan, Methylcyclohexan, Chlorbenzol, Cyclohexanon und 2,2,4-Trimethylpentan.

Besonders günstige Lösungsmittel zum Waschen haben die Eigenschaft, die bei der chemischen Reaktion eingesetzten und entstandenen Verbindungen zu lösen, so dass sie diese beim Waschen effektiv entfernen. Dadurch verbleiben keine Substanzen auf der Oberfläche, die bei einer möglichen darauffolgenden Ausbildung einer chemischen Bindung stören könnten.

Bevorzugt ist das aufeinanderfolgende Waschen der Oberfläche mit verschiedenen Lösungsmitteln, wobei das Waschen nach Abschluss der Reaktion nach einem jeden Reaktionsschritt erfolgt. Bei den verschiedenen Waschschritten werden dabei verschiedene Lösungsmittel verwendet. Insbesondere die Verwendung von Lösungsmitteln unterschiedlicher Polarität hat hier Vorteile, da so eine noch größere Bandbreite unterschiedlicher auf der Oberfläche befindlicher nicht kovalent gebundener Substanzen entfernt werden kann, als dies mit einem einzigen Lösungsmittel der Fall wäre. Ein Beispiel ist das aufeinanderfolgende Waschen mit DMF, MeOH und DCM nach jedem Schritt.

Im Rahmen der erfindungsgemäßen Verfahren werden die verschiedenen Verbindungen, d. h. die zu kuppelnde Verbindung bzw. die zu immobilisierende Verbindung, das Kupplungsreagenz, der Zuschlagstoff und/oder die Base in Lösungsmitteln vorliegend eingesetzt. Dabei ist es auch im Rahmen der Erfindung, dass zwei oder mehrere

der vorstehend genannten Verbindungen gleichzeitig in einem Lösungsmittel bzw. im gleichen Lösungsmittel enthalten sind.

Beispiele für Lösungsmittel, in denen die zu immobilisierende oder zu kuppelnde Verbindung, das Kupplungsreagenz oder der Zuschlagstoff vorliegen können, sind
N,N-Dimethylformamid (DMF), 1-Methyl-2-pyrrolidinon (NMP), N,N-Dimethylacetamid (DMA), Dimethylsulfoxid (DMSO), Tetrahydrofuran (THF), Dioxan, Acetonitril (MeCN, ACN), Methanol (MeOH), Ethylacetat (EE), 2,2,2-Trifluorethanol (TFE), 1,1,1,3,3,3-Hexafluor-2-propanol (Hexafluoroisopropanol, HFIP), 1,2-Dibromethan, Dichlormethan (DCM), Diethylether, Aceton, 1,2-Dimethoxyethan (DME), Bis(2-Methoxyethyl)ether (Diglyme), 1,3-Dimethyl-2-imidazolidinon, 1,3-Dimethyl-3,4,5,6-tetrahydro-2(1H)-pyrimidinon, Hexamethylphosphorsäureamid (HMPA), Dimethoxyethan, Nitromethan, Nitrobenzol, Propylacetat, Wasser, Chloroform, 1,2-Dichlorethan, 1,3-Dichlorpropan, Toluol, Benzol, Xylol, 1-Butanol, 2-Butanol, Petrolether, Cyclohexan, Cyclopentan, tert-Butyl-methylether, Ethoxyethanol, Pentan, Hexan, Heptan, Octan, Methylethylketon, Diethylketon, 1,2-Dichlorbenzol, Kohlenstofftetrachlorid, 1-Chlorbutan, Diisopropylether, 1,1,1-Trichlorethan, 1,1,2-Trichlortrifluorethan, 1-Butylacetat, Tetrachlorethylen, Trichlorethylen, Trimethylpentan, 2-Methylbutan, Methylcyclohexan, Chlorbenzol, Cyclohexanon, 2,2,4-, Trimethylpentan.

Bevorzugterweise werden in den erfindungsgemäßen Verfahren die folgenden Lösungsmittel verwendet.

N,N-Dimethylformamid (DMF), 1-Methyl-2-pyrrolidinon (NMP), N,N-Dimethylacetamid (DMA), Dimethylsulfoxid (DMSO), Tetrahydrofuran (THF), Dioxan, Acetonitril (MeCN, ACN), Methanol (MeOH), Ethylacetat (EE), 2,2,2-Trifluorethanol (TFE), 1,1,1,3,3,3-Hexafluor-2-propanol (Hexafluoroisopropanol, HFIP), 1,2-Dibromethan, Dichlormethan (DCM), Diethylether, Aceton, 1,2-Dimethoxyethan (DME), Bis(2-Methoxyethyl)ether (Diglyme), 1,3-Dimethyl-2-imidazolidinon, 1,3-Dimethyl-3,4,5,6-tetrahydro-2(1H)-pyrimidinon, Hexamethylphosphorsäureamid (HMPA), Dimethoxyethan, Nitromethan, Nitrobenzol, Propylacetat, Wasser, Chloroform, 1,2-Dichlorethan, 1,3-Dichlorpropan, Toluol, Benzol, Xylol, 1-Butanol, 2-Butanol, Petrolether, Cyclohexan, Cyclopentan.

Ganz besonders bevorzugt werden in den erfindungsgemäßen Verfahren die folgenden Lösungsmittel verwendet:

> N,N-Dimethylformamid (DMF), 1-Methyl-2-pyrrolidinon (NMP), N,N-Dimethylacetamid (DMA), Dimethylsulfoxid (DMSO), Tetrahydrofuran (THF), Dioxan, Acetonitril (MeCN, ACN), Methanol (MeOH), Ethylacetat (EE), 2,2,2-Trifluorethanol (TFE), 1,1,1,3,3,3-Hexafluor-2-propanol (Hexafluoroisopropanol, HFIP), 1,2-Dibromethan, Dichlormethan (DCM), Diethylether, Aceton,

insbesondere
N,N-Dimethylformamid (DMF), 1-Methyl-2-pyrrolidinon (NMP) und N,N-Dimethylacetamid (DMA).

Neben einzelnen Lösungsmitteln können auch Mischungen derselben verwendet werden.

Zur allgemeinen Auswahl des jeweils geeigneten Lösungsmittels kann der Fachmann auf dem Gebiet verschiedene Kriterien heranziehen. Ein wichtiges Kriterium des zu verwendenden Lösungsmittels ist dessen Eigenschaft, die jeweilige Substanz bzw. die jeweiligen Substanzen in hohen Konzentrationen zu lösen. Das hat die Vorteile, dass durch hohe Konzentrationen schnelle Reaktionsgeschwindigkeiten erreicht werden können und die Menge an teuren und umweltbelastenden Lösungsmitteln reduziert werden kann. Dies hat aber auch den Effekt, dass das Lösungsmittel und die darin enthaltene Verbindung(en) mit höherer Geschwindigkeit durch ein Pipettiergerät verteilt werden können, was insbesondere bei der parallelen Synthese sehr vieler verschiedener Verbindungen und im Rahmen eines automatisierten erfindungsgemäßen Verfahrens nicht unerheblich ist.

Weitere wichtige Kriterien zur Auswahl eines geeigneten Lösungsmittels sind die Flüchtigkeit desselben und der Zeitpunkt, an dem dieses verwendet wird. Im Anschluß an die ersten Schritte des Verfahrens, in denen noch keine oder nur eine geringe Ausbildung der gewünschten chemischen Bindung erfolgt, ist eine Trocknung der Oberfläche günstig (siehe oben). Dies ist einfach zu bewerkstelligen bei Verwendung eines leicht flüchtigen Lösungsmittels, was aber nicht bedeutet, dass höhersiedende Lösungsmittel wie z.B. DMF, NMP, DMA oder DMSO nicht geeignet sind.

Beispiele für leicht flüchtige Lösungsmittel, die im Rahmen der erfindungsgemäßen Verfahren verwendet werden können, sind:

> Tetrahydrofuran (THF), Dioxan, Acetonitril (MeCN, ACN), Methanol (MeOH), Ethylacetat (EE), 2,2,2-Trifluorethanol (TFE), 1,1,1,3,3,3-Hexafluor-2-propanol (Hexafluoroisopropanol, HFIP), Dichlormethan (DCM), Diethylether, Aceton, Chloroform, Petrolether, Cyclohexan, Cyclopentan, tert-Butyl-methylether, Pentan, Hexan, Heptan, Kohlenstofftetrachlorid und 2-Methylbutan.

Beim letzten Schritt des Verfahrens, im Anschluß dessen die Aktivierung und Kupplung der zu immobilisierenden Verbindung erfolgt, sind schwerflüchtige Lösungsmittel bevorzugt. Diese verdunsten langsamer und führen dadurch zu verlängerten Reaktionszeiten und somit zu besseren Ausbeuten. Außerdem sind hier Lösungsmittel günstig, die geeignet sind, die Aggregation von immobilisierten Substanzen auf der Oberfläche zu minimieren, da erhöhte

Aggregation häufig zu verminderter Zugänglichkeit reaktiver Gruppen und damit zu geringeren Reaktionsausbeuten führt. In der Peptidsynthese wurde dieser Effekt vielfach beschrieben [C. Hyde et al., *Int. J. Pept. Prot. Res.* **1994,** *43*, 431-440].

Lösungsmittel, die besonders gut geeignet sind, die Aggregation wachsender Peptidketten zu verringern und damit im Rahmen der erfindungsgemäßen Verfahren verwendet zu werden, sind z.B. DMF, NMP, DMSO, TFE mit DCM oder HFIP mit DCM [Albericio et al, *Methods Enzymol.* **1997,** *289*, 104-126].

Die chemische Bindung, die im Rahmen der erfindungsgemäßen Verfahren zwischen einer ersten auf einer Oberfläche, insbesondere planaren Oberfläche, immobilisierten funktionellen Gruppe und einer funktionellen Gruppe ausgebildet wird, wobei die zweite funktionelle Gruppe Teil einer zu immobilisierenden Verbindung ist, ist bevorzugterweise ausgewählt aus der Gruppe, die Carbonsäureamid-Bindungen, Carbonsäureester-Bindungen, Carbonsäureanhydrid-Bindungen, Sulfonsäureamid-Bindungen, Phosphorsäureester-Bindung, Phosphonsäureester-Bindung, Phosphorigsäureester-Bindung und Phosphorsäureamid-Bindungen umfasst. Bevorzugt ist dabei die Ausbildung von Carbonsäureamid-Bindungen oder sogenannten Peptidbindungen.

Im Rahmen der vorliegenden Erfindung sind die zu immobilisierenden Verbindungen insbesondere solche Verbindungen, die eine funktionelle Gruppe enthalten, welche geeignet ist, eine der vorstehend genannten chemischen Verbindungen auszubilden. Beispiele hierfür sind Carbonsäuren, Carbonsäureester, Sulfonsäuren, Phosphorsäureester, Phosphonsäureester, Phosphorigsäureester, Phosphoramidite, primäre Amine, sekundäre Amine und Alkohole. Dabei sind Carbonsäuren, primäre Amine und sekundäre Amine bevorzugt, und besonders bevorzugt Carbonsäuren.

Carbonsäuren, wie sie im Rahmen des erfindungsgemäßen Verfahrens als immobilisierte Gruppe bzw. Verbindung oder zu immobilisierende Gruppe oder Verbindung eingesetzt bzw. verwendet werden, sind bevorzugterweise N-terminal geschützte natürliche oder nicht-natürliche Aminosäuren, besonders bevorzugt N-terminal geschützte $\alpha$-Aminosäuren und ganz besonderes bevorzugt N-terminal geschützte $\alpha$-L-Aminosäuren.

Die immobilisierte funktionelle Gruppe, wie im Rahmen der erfindungsgemäßen Verfahren verwendet, ist eine funktionelle Gruppe, welche geeignet ist, eine der vorstehend genannten chemischen Bindungen auszubilden. Beispiele hierfür sind Carbonsäuren, Carbonsäureester, Sulfonsäuren, Phosphorsäureester, Phosphonsäureester, Phosphorigsäureester, Phosphoramidite, primäre Amine, sekundäre Amine und Alkohole. Bevorzugterweise ist die immobilisierte funktionelle Gruppe aus der Gruppe ausgewählt, die Carbonsäuren, primäre Amine und sekundäre Amine umfasst. Bevorzugterweise ist die immobilisierte funktionelle Gruppe ein primäres Amin oder ein sekundäres Amin. Primäre und sekundäre Amine sind bevorzugterweise C-terminal geschützte natürliche oder nicht-natürliche Aminosäuren, besonders bevorzugt C-terminal geschützte $\alpha$-Aminosäuren, bevorzugterweise C-terminal geschützte $\alpha$-L-Aminosäuren.

Im Rahmen der erfindungsgemäßen Verfahren ist vorgesehen, dass die Oberfläche mit einer Lösung kontaktiert wird. Verschiedene Methoden hierfür werden in Fig. 7+8 erläutert. Eine prinzipielle Möglichkeit des Aufbringens von Flüssigkeit ist das Kontaktieren mit einer Kapillare. Bei vollständig manuellen Verfahren kann dabei eine Pipette verwendet werden. Bei automatisierter Verteilung einer Vielzahl verschiedener Lösungen wird bevorzugterweise ein Dispensionsapparat im weitesten Sinne verwendet. Dabei kann auch der *Auto-Spot-Robot ASP 222* der Fa. Abimed Analysentechnik GmbH, Raiffeisenstraße 3, D-40764 Langenfeld [R. Frank et al. in *Combinatorial Peptide and Nonpeptide Libraries,* Ed. G. Jung, VCH, Weinheim, **1996,** S. 363-386] verwendet werden. Dieser besteht im wesentlichen aus einem *Gilson Model XL 222 Sample Changer* und einem *Gilson Model 401 Dilutor,* die von einem PC mit entsprechender Software gesteuert werden [R. Frank et al. in *Combinatorial Peptide and Nonpeptide Libraries,* Ed. G. Jung, VCH, Weinheim, **1996,** S. 363-386]. Das Aufbringen einer Lösung auf eine Oberfläche erfolgt hier i.d.R. mit einer Stahlnadel mit einer Länge von ca. 5-15 cm und einem Innendurchmesser von 0.2 bis 2.0 mm, deren Innendurchmesser an der Spitze gegebenenfalls durch eine spezielle Nadelspitze ("Needle Tip") [Auto-Spot Robot ASP 222, Handbuch, Abimed, Langenfeld, S. 7-1] verringert wird (vgl. Fig. 6).

Neben der Kombination einer Kapillare mit einer Spritze oder Pumpe, wie es z.B. beim *Auto-Spot-Robot ASP 222* der Fall ist, können Kapillaren auch ohne mechanische Unterstützung zum Pumpen von Flüssigkeiten eingesetzt werden, wobei Kapillarkräfte das Aufsaugen und Abgeben von Flüssigkeit bestimmen. Geeignete Kapillaren können dabei jene sein, wie sie in Fig. 7B+C dargestellt sind.

Speziellere Ausführungsformen zum Kontaktieren einer Oberfläche mit einer Flüssigkeit durch Kontaktieren mit einer Kapillare sind z.B. das $\mu$FN-Verfahren (hier benutzt man ein Netzwerk vieler Mikrokapillaren) und das $\mu$WP-Verfahren (Micro-Wet-Printing der Fa. Clondiag [E. Erdmanntraut et al., *Proceedings of* $\mu$*TAS'98* **1998,** 217-221, Kluwer Scientific Publishing, Utrecht], bei der die Verwendung von Masken mit Löchern, die mit Flüssigkeit gefüllt sind, vorgesehen ist ($\mu$WP-Methode), die beide auch als Verfahren unter Einsatz einer Kapillare angesehen werden können. Mit der $\mu$WP-Methode lassen sich theoretisch Dichten von bis zu 1 Mio. Spots pro $cm^2$ erreichen.

Neben dem Aufbringen von Lösungen mittels Kontaktieren mit einer Kapillare ist auch das Aufsprühen von Flüssigkeiten ein mögliches Verfahren. Verfahren zum Aufsprühen von Flüssigkeiten auf Oberflächen, wie sie im Rahmen der erfindungsgemäßen Verfahren verwendet werden können, sind den Fachleuten bekannt.

Das allgemeine Prinzip dieses Verfahrens besteht darin, dass Tropfen der aufzubringenden Flüssigkeit erzeugt und auf die Oberfläche gesprüht werden, ohne dass die Oberfläche und die Anordnung, an der die Tropfen generiert werden, sich berühren. Diese Vorgehensweise weist einige Vorteile auf: So kann z.B. keine mechanische Zerstörung von Dispenser oder Oberfläche durch Berührung auftreten. Außerdem sind Nicht-Kontakt-Verfahren i.d.R. schneller, da keine vertikale Bewegung z.B. einer Nadel notwendig ist, während der die horizontale Bewegung des Tropfen-Generators gestoppt werden müsste.

Zur Generierung von Tropfen können z.B. Schläuche oder Nadeln in Kombination mit einer Pumpe oder Spritze verwendet werden. Ein apparativ besonders einfaches Verfahren ist hier die Verwendung einer Spritzflasche [VWR International, Laborkatalog, 2002, S. 215.32]. Andere Möglichkeiten sind z.B. die Verwendung von Düsen mit einem thermischen Druck-Überträger, dem "nozzleness acoustic jet" (nozzleness = engl. düsenfrei), "Bubble-Jet-Devices" sowie piezoelektrischen Kapillaren und Hohlräumen [T. P. Theriault et al. in *DNA Microarrays: A Practical Approach,* M. Schena, Ed., Oxford University Press, New York, **1999,** pp. 101-120] [A. C. Tam, *Appl. Opt.* **1982,** *21*, 1891-1895].

Der Prototyp eines piezoelektrischen Gerätes ist eine von einem cylindrischen piezoelektrischen Element umgebene Glaskapillare [T. W. Shield, *IBM J. Res. Dev.* **1987,** *31*, 96-100]. Ein kurzer elektrischer Puls (typischerweise 100 V, 5 µs) am piezoelektrischen Element führt zu einer Kompression desselben, was wiederum Schock-Pulse in der Flüssigkeitskammer erzeugt. Dadurch werden Tropfen aus dem Gerät geschleudert, wie dies beispielsweise in Fig. 8A und 8B dargestellt ist. Piezoelektrische Geräte benötigen eine relativ große Menge an verwendeter Flüssigkeit (typischerweise 1-4 µl); dafür lassen sich Tropfen mit sehr definierter Größe generieren.

Ein besonderer Fall des Aufsprühens von Lösungen ist das sogenannte Top-Spot-Printen der Fa. GeneScanEurope GmbH, wie dies beispielsweise in Fig. 8C dargestellt ist. Hier erzeugt ein pneumatischer Puls an einer speziellen Mikrotiterplatte bis zu 96 verschiedene Lösungstropfen parallel innerhalb einer Sekunde. Der wesentliche Vorteil dieses Verfahrens ist die extrem hohe Geschwindigkeit, mit der die Tropfen auf die Oberfläche aufgebracht werden können.

Zum simultanen Besprühen sehr vieler unterschiedlicher Bereiche einer Oberfläche mit einer Lösung, wie es im Rahmen der erfindungsgemäßen Verfahren ebenfalls in Ausführungsformen vorkommen kann, bietet sich die Verwendung spezieller Sprühdüsen, die z.B. in handelsüblichen Sprühflaschen, Druckpump-Zerstäubern oder Pumpzerstäuberflaschen eingebaut sind [VWR International, Laborkatalog, **2002,** S. 215.33], an. Die wichtigsten Vorteile dieser Geräte sind die einfache Handhabung, der geringe Preis sowie die extrem hohe Geschwindigkeit, mit der große Bereiche einer Oberfläche simultan besprüht werden können. So werden z.B. bei der SPOT-Synthese von Peptiden bzw. im Rahmen der vorliegenden Erfindung häufig mehrere Tausend unterschiedliche Peptide parallel auf einer Oberfläche (z.B. Cellulose-Membran, typischerweise mit eine Größe von 28x39 cm) synthetisiert.

Bei den erfindungsgemäßen Verfahren gilt es in verschiedenen Ausführungsformen die gleiche Lösung, z.B. eines Kupplungsreagenzes oder einer Base, mit allen wachsenden Peptidketten in Kontakt zu bringen. Ein Beispiel ist die parallele Aminosäure-Kupplung durch das simultane Inkontaktbringen aller membrangebundener Amine mit einer Lösung des Kupplungsreagenzes HBTU (z.B. 0.3 mol/l in Acetonitril gelöst), Trocknen, und anschließendem Aufbringen einer Lösung einer Aminosäure als der zu immobilisierenden Verbindung und einer Base (z.B. Fmoc-Gly-OH und DIPEA, 0.6 M und 1.2 M in NMP). Die parallele Auftragung der HBTU-Lösung auf z.B. 2000 Spots gelingt z.B. mit einer geeigneten Sprühflasche innerhalb weniger Sekunden.

Neben dem Aufbringen von Lösungen mittels Kontaktieren mit einer Kapillare ist auch das Aufstempeln von Flüssigkeiten ein mögliches Verfahren. Ein Beispiel für mögliche Ausführungsformen ist das Contact Tip Deposition Printing (Fig. 7-A). Bei diesem Verfahren taucht die Spitze eines festen Pins in eine Lösung, wodurch eine definierte Menge der Lösung am Pin adsorbiert wird. Indem die Pin-Spitze dann mit der Oberfläche in Kontakt gebracht wird, wird die Flüssigkeit auf die Oberfläche übertragen. Dadurch lassen sich Spot-Größen bis hinunter zu 50 µm herstellen. Zum simultanen Aufstempeln mehrerer Flüssigkeiten sind Geräte mit mehreren (z.B. 96) parallel angeordneten Pins kommerziell erhältlich.

Ein sehr ähnliches Verfahren ist die µCP-Technologie, bei der die festen Pins aus sogenannten "inked stamps" (d.h. mit Tinte beladenen Stempeln) aus strukturierten Elastomeren bestehen. Die erreichbare Präzision liegt hier im kleinen µm-Bereich [Y. Xia et al., *Angew. Chem.* **1998,** *110*, 568-594].

Eine andere mögliche Ausführungsform ist das "Pin- and Ring-Printing" (Fig. 7-D). Hier ist ein kleiner Ring mit der zu übertragenden Flüssigkeit gefüllt und ein fester Pin überträgt einen Teil dieser Lösung auf die Oberfläche, indem die Ppin-Spitze durch die Flüssigkeit auf die Oberfläche bewegt wird. So lässt sich eine Flüssigkeit schnell mehrmals auf eine Oberfläche aufbringen.

Als Trägermaterial oder Oberfläche, beide Begriffe werden hierin als gleichbedeutend verwendet, das bzw. die im Rahmen der erfindungsgemäßen Verfahren verwendet wird, wird ein mechanisch stabiles, gegenüber den eingesetzten Reagenzien und Lösungsmitteln chemisch inertes Flachmaterial eingesetzt. Unter Flachmaterial wird ein Material verstanden, bei dem eine der drei zueinander senkrechten räumlichen Dimensionen wesentlich kleiner als die beiden anderen Dimensionen ist. Dieses Flachmaterial oder Träger daraus wird/werden im Rahmen der erfindungsgemäßen Verfahren mit beliebiger räumlich begrenzter Form als Segmente verwendet, beispielsweise in Form von

Blättern oder Streifen. Das Flachmaterial kann porös oder nicht porös und nach Belieben auf einer Unterlage aufgebracht oder selbsttragend sein. Einsetzbar als Flachmaterial sind z.B. Folien, Platten, Membranen oder Papiere. Wesentlich ist, daß das Flachmaterial chemisch reaktionsfähige Gruppen (Funktionen) trägt, die bevorzugterweise Teil einer immobilisierten Verbindung sind, an die geeignete Verbindungen kovalent geknüpft werden können. Vorteilhafterweise ist das Trägermaterial oder die Oberfläche, welches für die chemische Synthese eingesetzt wird, auch für den Einsatz in - bevorzugt biologischen - Testreaktionen geeignet. Biologische Testreaktionen werden eingesetzt, um die Struktur oder Funktion einer Verbindung (z.B. ein an ein Flachmaterial gebundenes Peptid) zu untersuchen. Ein Beispiel ist die Umsetzung mit einem fluoreszenzmarkierten Antikörper, der spezifisch an bestimmte Bereiche eines Peptids bindet. Die unter Anwendung der erfindungsgemäßen Verfahren hergestellten Verbindungen können so ohne Isolierung und nachfolgender Immobilisierung direkt in den besagten Testreaktionen eingesetzt werden, wenn die Verknüpfung zwischen zu immobilisierender Verbindung und Trägermaterial, so gewählt wurde, dass sie stabil während der biologischen Testreaktion ist. Wenn das Flachmaterial porös ist, steht auch dessen innere Oberfläche für die Synthese zur Verfügung. Waschoperationen können dann auch durch Saugen von Lösungen durch das Material erfolgen. Nichtporöses Flachmaterial hat eine sehr viel geringere Oberfläche. Waschoperationen können daran durch einfaches Abspülen erfolgen.

Im Rahmen der erfindungsgemäßen Verfahren können Flachmaterialien aus verschiedenen Materialien verwendet werden, so z. B. die Hydroxylfunktionen tragende und damit direkt einsetzbare Cellulose, Glas oder Kunststoffe wie Polyethylen, Polypropylen, oder Teflon, an deren Oberfläche geeignete chemische Funktionen eingeführt wurden, z.B. durch Pfropfpolymerisation von Acrylsäure oder deren Derivaten. Je nach Ausführungsform und chemischer Vorbehandlung des Flachmaterials kann die flächenspezifische Beladung mit reaktiven Funktionen oder immobilisierten Verbindungen in einem weiten Bereich variiert werden (z.B. 1 pmol bis 1µmol pro $cm^2$).

Beispiele für im Rahmen der erfindungsgemäßen Verfahren verwendbare planare Oberflächen sind, unter anderem, Cellulose [R. Frank et al., *Tetrahedron* **1988,** *44*, 6031-6040] [J. Eichler et al., *Collect. Czech. Chem. Commun.* **1989**, *54,* 1746-1751] [R. Frank, *Tetrahedron* **1992**, *48*, 9217-9232], Baumwolle [J. Eichler et al., *Peptide Res.* **1991,** *4*, 296-307] [M. Schmidt et al., *Bioorg. Med. Chem. Lett*. **1993,** *3*, 441-446] oder polymere Filme [R. H. Berg et al., *J. Am. Chem. Soc.* **1989,** *111*, 8024-8026], Scheiben [K. Lou et al., *Proc. Natl. Acad. Sci. USA* **1995,** *92*, 11761-11765] [N. Hird, *Tetrahedron* **1999**, *55*, 9575-9584] und Membranen [S. B. Daniels et al. *Tetrahedron Lett.* **1989,** *30*, 4345-4348] [Z. Wang et al., *Peptide Res.* **1992,** *5*, 275-280] oder rigide, nicht-poröse Materialien wie Glas, Goldüberzogene Oberflächen, Titan, Aluminiumoxid, Silizium und modifizierte Polymere wie Polypropylen, Polyethylen, Polytetrafluorethylen oder Polyvinylidendifluorid-Oberflächen.

**Figurenbeschreibung**

Die vorliegende Erfindung wird nun anhand der Figuren sowie Beispiele näher erläutert, aus denen sich weitere Vorteile sowie Merkmale und Ausführungsformen der vorliegenden Erfindung ergeben. Dabei zeigt

Fig. 1 die Prinzipien der Festphasensynthese von Peptiden;

Fig. 2 eine Übersicht über die Fmoc/$^t$Bu-Strategie;

Fig. 3 den allgemeinen Mechanismus einer Amidbildungs-Reaktion;

Fig. 4 den Mechanismus der Peptid-Bindungs-Bildung durch Aminiumsalze;

Fig. 5 den Mechanismus der Peptid-Bindungs-Bildung durch Phosphonium-Reagenzien;

Fig. 6 eine schematische Darstellung des *Auto-Spot-Robot ASP 222*, einschließlich einer Detaildarstellung der verwendeten Nadel;

Fig. 7 u. 8 eine Übersicht über verschiedene Aufbringungsverfahren von Flüssigkeiten auf Oberflächen und der dabei verwendeten Kapillaren;

Fig. 9 das Messprinzip zur Bestimmung des Derivatisierungsgrades einer Oberfläche, auf der im Rahmen der erfindungsgemäßen Verfahren eine Verbindung immobilisiert ist;

Fig. 10 den prinzipiellen Reaktionsablauf einer Peptidsynthese an planaren Oberflächen unter SPOT-Bedingungen nach dem Verfahren nach dem Stand der Technik und Verwendung von OPfp-Estern;

Fig. 11A-E das Ergebnis von Beispiel 2 in Form von HPLC-Chromatogrammen, wobei in a) jeweils die Ergebnisse unter Anwendung des erfindungsgemäßen Verfahrens und in b) jeweils die Ergebnisse unter Anwendung des Verfahrens nach dem Stand der Technik dargestellt sind;

Fig. 12A-C das Ergebnis von Beispiel 3 in Form von HPLC-Chromatogrammen, wobei in a) jeweils die Ergebnisse unter Anwendung des erfindungsgemäßen Verfahrens und in b) jeweils die Ergebnisse unter Anwendung des Verfahrens nach dem Stand der Technik dargestellt sind;

Fig. 13 das Ergebnis von Beispiel 4 in Form des Anteils an gewünschtem Hauptprodukt bei der Kopplung von R auf RFG bei verschiedenen Voraktivierungszeiten und Anwendung des erfindungsgemäßen Verfahrens und eines Verfahrens nach dem Stand der Technik;

Fig. 14 Ausschnitte von $^{13}C$-NMR-Spektren von Fmoc-Ala-OH und HBTU (je 0.45 M in DMF-d7) vor Zugabe von DIPEA (a und b) und nach Zugabe von DIPEA (c und d); und

Fig. 15A u. B die Aktivität von Lösungen (gemäß der Definition in Beispiel 9) verschiedener Aminosäuren mit dem Kupplungsreagens HBTU bei verschiedenen Lagerungsbedingungen.

**Figurendetailbeschreibung**

Fig. 1 zeigt das Prinzip der Festphasensynthese von Peptiden.

Die C-terminale Aminosäure des Zielpeptides, d. h. des durch mehrfaches Ausführen der erfindungsgemäßen Verfahren herzustellenden Polypeptides, wird zunächst über die Carbonylgruppe am unlöslichen Trägermaterial angebunden. Dabei kann eine direkte Anknüpfung an die Oberfläche erfolgen oder die Aminosäure wird an einen Linker angeknüpft, der seinerseits an die Oberfläche gebunden ist. Linker werden häufig in der Festphasensynthese eingesetzt mit dem Zweck, eine während einer Synthese stabile Anknüpfung einer Verbindung an eine Festphase zu gewährleisten, die nach der Synthese wieder gespalten werden kann. Beispiele für Linker sind in vielen Übersichtsartikeln aufgeführt [F. Guillier et al., *Chem. Rev.* 2000, *100*, 2091-2157]. Funktionelle Gruppen in der Seitenkette der Aminosäure(n), die bereits auf der Oberfläche immobilisiert ist, müssen mit permanenten Schutzgruppen maskiert werden, die durch die Bedingungen der Peptipsynthese nicht beeinträchtigt werden. Die temporäre Schutzgruppe für die Aminogruppe, die die besagte bereits immobilisiert vorliegende Aminosäure während der Anbindung an die Festphase schützt, wird entfernt. Ein Überschuss der zweiten Aminosäure, d. h. der an die immobilisierte Aminosäure zu immobilisierenden bzw. chemisch bindenden Aminosäure, wird eingeführt, wobei die Carboxygruppe der Aminosäure für die Amidbindungs-Bildung aktiviert wird. Nach der Kupplung werden überschüssige Reagenzien durch Waschen entfernt und die Schutzgruppe vom N-Terminus der nun als Dipeptid vorliegenden immobilisierten Verbindung entfernt, bevor die dritte Aminosäure addiert wird. Dieser Prozess wird wiederholt, bis die gewünschte Peptidsequenz synthetisiert ist. Im letzten Schritt wird das Peptid von der Oberfläche abgespalten und die Seitenketten-Schutzgruppen entfernt. Häufig werden Scheitenketten-Schutzgruppen und der Linker so gewählt, dass beide unter denselben Bedingungen gespalten werden.

Weitere Aspekte der Peptidsynthese wurden von Williams, Alberico und Giralt zusammengefasst [Lloyd-Williams et al., *Chemical Approaches to the Synthesis of Peptides and Proteins*, CRC Press, **1997**], auf die der Fachmann ansonsten bei der Durchführung der erfindungsgemäßen Verfahren zurückgreift.

Verglichen mit der Peptid-Synthese in homogener Lösung sind mit dem Festphasenverfahren deutlich längere Oligomere in kürzerer Zeit synthetisierbar. Die sonst nach jedem Syntheseschritt erforderliche - meist chromatographische - Aufreinigung wird bei der Festphasensynthese durch einfaches Waschen der Festphase ersetzt. Die Zeit für einen Kupplungszyklus wird so erheblich verkürzt. Zwar können Nebenprodukte, wie sie etwa durch unvollständige Reaktionen entstehen, nicht abgetrennt werden, dieser Nachteil kann jedoch durch die Anwendung hoher Reagenzienüberschüsse sowie Mehrfachkopplungen minimiert werden. Reaktionen lassen sich auf diese Weise nahezu quantitativ durchführen, in einem jeden Fall jedoch mit einer verglichen mit der Synthese in homogener Lösung erhöhten Ausbeute. Die so für jeden Reaktionsschritt erreichbaren hohen Ausbeuten sind eine notwendige Voraussetzung zur Synthese längerer Oligomere, da anderenfalls keine zufriedenstellenden Produktausbeuten erhalten werden können.

Neben der Synthese vom C-Terminus aus kann das Peptid unter Anwendung der erfindungsgemäßen Verfahren auch beginnend am N-Terminus schrittweise aufgebaut werden. Die Aktivierung von Festphasen-gebundenen Carbonsäuren führt aber häufig zu Racemisierung, weshalb dieser Weg nur selten beschritten wird, gleichwohl er eine für manche Anwendungen sinnvolle Alternative zur Synthese vom C-Terminus ausgehend ist.

Die wichtigste Schutzgruppenstrategie zur SPPS ist die sogenannte Fmoc/$^{t}$Bu-Strategie [L. A. Carpino et al., *J. Org. Chem.* **1972,** *37*, 3404-3409], welche die früher häufig verwendete Boc/Bzl-Strategie zunehmend verdrängt.

Das Verfahren basiert auf einer orthogonalen Schutzgruppenstrategie, wobei die basenlabile N-Fmoc-Gruppe

für den Schutz der alpha-Aminogruppe und säurelabile Scheitenketten-Schutzgruppen und Linker verwendet werden. In der Praxis werden $^{t}$Butyl- und Trityl-basierte Seitenketten-Schutzgruppen und Alkoxy-benzyl-basierte Linker verwendet, da sie mit TFA entfernt werden können. TFA ist ein excellentes Lösungsmittel für Peptide, kann in Standard-Glasapparaturen verwendet werden und ist flüchtig genug, um im Vakuum entfernt werden zu können.

Fig. 2 zeigt eine Übersicht über die Fmoc/$^{t}$Bu-Strategie. Die C-terminale Aminosäure wird zunächst an einen TFA-labilen Linker angebunden. Die Seitenketten sind dabei mit TFA-labilen Schutzgruppen geschützt. Mit 20% Piperidin in DMF wird dann die temporäre N-alpha-Fmoc-Schutzgruppe entfernt. Anschließend gelingt die Kupplung der nächsten Aminosäure üblicherweise in DMF oder NMP mit aktivierten Aminosäuren. Mit 95 % TFA werden dann das Peptid vom Linker gespalten und zugleich die Seitenketten-Schutzgruppen entfernt. Der Fmoc-Zugang zu SPPS wurde schon in vielen Übersichtsartikeln zusammengefasst [Fields et al., *Int. J. Pept. Protein Res.* **1990,** *35*, 161] [Chan, White, Ed., *Fmoc Solid Phase Peptide Synthesis*, Oxford University Press, **2000**].

Fig. 3 zeigt den allgemeinen Mechanismus einer Amidbildungs-Reaktion.

Wegen der relativ geringen Reaktivität freier Carbonsäuren gegenüber Aminogruppen gelingt die Bildung einer Amidbindung unter milden Bedingungen nur durch den Zusatz sogenannter Kupplungsreagenzien [Jakubke, *Peptide: Chemie und Biologie*, Spektrum Akademischer Verlag, **1996,** S. 99], wie sie hierin auch beschrieben sind. Dabei wird in der Regel die Carbonylkomponente aktiviert. Das geschieht meist durch Einführung von elektroaffinen -I- bzw. -M-Substituenten (XR') durch ein Kupplungsreagenz. Diese Reaktion wird in der Regel durch den Zusatz von Basen begünstigt, da der initiierende Schritt häufig die Abstraktion (=Abspaltung) des aciden COOH-Protons darstellt.

Durch XR' wird die Elektronendichte am Carbonyl-C-Atom verringert, so daß der nucleophile Angriff der Aminokomponente begünstigt wird. Die nucleophile Aminokomponente **XXb** greift mit dem freien Elektronenpaar das Carbonyl-C-Atom an und verdrängt das Bindungselektronenpaar, das vom Carbonyl-O-Atom aufgenommen wird. Die elektrophile Stabilisierung dieser negativen Ladung erfolgt innerhalb des Intermediats **XXc,** das unter Bildung der Amidbindung zerfällt.

Fig. 4 zeigt den Mechanismus der Peptid-Bindungs-Bildung durch Aminiumsalze [V. Dourtoglou et al., *Synthesis* **1984,** 572]. Das stabilere der beiden Intermediate **X6** und **X7** ist die ungeladene Verbindung **X7.** Bei längeren Voraktivierungszeiten liegt deshalb überwiegend **X7** in Lösung vor und die Kupplungen im Rahmen der erfindungsgemäßen Verfahren erfolgen ausgehend von dieser Aktivspecies [Albericio et al, *Methods Enzymol*. **1997**, *289*, 104-126].

Fig. 5 zeigt den Reaktionsmechanismus der Peptidbindungsbildung durch Phosphonium-Reagenzien.

Das stabilere der beiden Intermediate **X8** und **X9** ist auch hier die ungeladene Verbindung **X9.** Bei längeren Voraktivierungszeiten liegt deshalb überwiegend **X9** in Lösung vor und die Kupplungen erfolgen ausgehend von dieser Aktivspecies [Albericio et al, *Methods Enzymol.* **1997**, *289*, 104-126] und weniger ausgehend von **X8.**

Die geringe Lebenszeit des Intermediats **X8** ist in einigen Untersuchungen bestätigt worden: Kim und Patel [M. H. Kim et al., *Tetrahedron Lett*. **1994,** *35*, 5603-5606] berichteten, dass solche Intermediate nur bei -20 °C in Abwesenheit eines Überschusses an HOBt existieren können. Coste und Campagne [J. Coste et al., *Tetrahedron Lett*. **1995,** *36,* 4253-4256] beschrieben diese sogar als noch labilere Species, die sogar bei niedrigen Temperaturen zum Aktivester umgesetzt werden.

Bei extrem kurzen Voraktivierungszeiten, wie sie im Fall der beschriebenen Erfindung vorliegen, kann aber ein größerer Teil der Peptidbindung über das Intermediat **X8** gebildet werden. Dieser Unterschied in den Aktivspecies gegenüber den bisher bekannten Verfahren kann eine Reihe von positiven Effekten auf den Kupplungsprozeß haben: Racemisierung kann ausgehend von **X8** geringer sein als ausgehend von **X9,** die Reaktion kann schneller verlaufen und die Ausbeuten können erhöht sein.

Fig. 6 zeigt eine schematische Darstellung des *Auto-Spot-Robot ASP 222*, einschließlich einer Detaildarstellung der verwendeten Nadel.

Mit dem *Auto-Spot-Robot ASP 222* lassen sich Volumina von ca. 0.05 µl bis zu mehreren ml exakt aufbringen; in der Regel werden je nach Größe des verwendeten Spots Volumina von 0.1 µl bis 2.0 µl verwendet. Diese Volumina führen in der Regel zu Spots mit einem Durchmesser von ca. 1-10 mm, so dass sich Dichten von bis zu 25 Spots pro $cm^2$ erreichen lassen. Da die Arbeitsfläche des ASP ca. 28x39 cm beträgt, ist das Gerät prinzipiell geeignet, um 27.000 und mehr Peptide parallel zu synthetisieren.

Fig. 7 zeigt die verschiedenen Aufbringungsverfahren von oder Kontaktierverfahren für Flüssigkeiten auf Oberflächen und die dabei verwendeten Absetzwerkzeuge. Fig. 7B und 7C zeigen Methoden zum Aufbringen von Lösungen durch Kontaktieren mit einer Kapillare; Fig. 7A und 7D zeigen Methoden zum Aufbringen von Lösungen durch Aufstempeln. Dabei können, wie im Falle der Figur 7 B und 7C die verwendeten Kapillaren am oberen Rand sowohl geschlossen als auch geöffnet sein. Geschlitzte Nadeln, wie beispielsweise in Fig. 7 B dargestellt, werden auch als "Split-Pins" bezeichnet. Neben einer einzelnen Nadel können auch Arrays von mehreren Nadeln eingesetzt werden. Bei dem Verfahren, wie es in Fig. 7 A schematisch dargestellt ist, wird ein an einem Absetzwerkzeug infolge ihrer Kapillarkräfte bzw. Oberflächenspannungen gehaltener Flüssigkeitstropfen durch Kontakt des Tropfens mit der

Oberfläche abgesetzt. Im Falle des Verfahrens gemäß Fig. 7 B wird durch das Kontaktieren der geschlitzten Nadel, die im Schlitz eine bestimmte Flüssigkeitsmenge enthält, diese Flüssigkeitsmenge auf der Oberfläche abgesetzt. Bei den in Fig. 7 C dargestellten Verfahren wird die beidseitig offene Kapillare mit einem Ende in Kontakt gebracht mit der Oberfläche, auf der die Flüssigkeit abgesetzt werden soll, durch Kapillarkräfte bzw. Oberflächenspannungen und weitere Kräfte (z.B. Gravitationskräfte) wird ein bestimmtes Flüssigkeitsvolumen sodann abgesetzt. Bei dem in Fig. 7 D dargestellten Verfahren wird eine Flüssigkeitsmenge dadurch abgesetzt, dass im weitesten Sinne ein Loch, welches mit einer Flüssigkeit gefüllt ist, bereitgestellt wird, durch das ein Übertragungswerkzeug gestoßen wird, wobei durch das Durchdringen des Absetzwerkzeuges durch die Flüssigkeit ein bestimmtes Flüssigkeitsvolumen aus dem Loch auf die Oberfläche transferiert wird

Dieses Verfahren wird auch als "Pin-and-Ring-Printing" bezeichnet

Fig. 8 zeigt die verschiedenen Aufbringungsverfahren von oder Kontaktierverfahren für Flüssigkeiten auf Oberflächen und die dabei verwendeten Absetzwerkzeuge für den Fall, daß das Aufbringen mittels Aufsprühen erfolgt. In Fig. 8A und 8B sind Ausführungsformen eines piezoelektrischen Gerätes zum Aufsprühen von Lösungen dargestellt, bei dem ein elektrischer Puls zu einer Kompression und letzlich der Generierung eines Tropfens einer Flüssigkeit führt. Fig. 8C beschriebt das Top-Spot-Printen der Firma GeneScanEurope GmbH, das sich dadurch auszeichnet, daß durch einen pneumatischen Puls an einer Mikrotiterplatte parallel bis zu 96 Flüssigkeitstropfen erzeugt werden.

**Beispiel 1: Material und Methoden**

Die im Folgenden beschriebenen Materialien und Methoden sowie allgemeinen Arbeitsvorschriften wurden im Rahmen der hierin beschriebenen weiteren Beispiele durchgeführt.

Lösungsmittel:

Alle verwendeten Lösungsmittel wurden in der angegebenen Qualität ohne weitere Reinigung eingesetzt:

Acetonitril (Gradient grade, J.T. Baker); Dichlormethan (zur Synthese, Merck Eurolab); Diethylether (zur Synthese, Merck Eurolab); *N*,*N*-Dimethylformamid (LAB, Merck Eurolab); Dioxan (zur Synthese, Aldrich); Methanol (zur Synthese, Merck Eurolab).

Wasser wurde unter Verwendung einer Vollentsalzungsanlage (Milli-Q Plus, Millipore) entmineralisiert.
Die Lösungsmittel für Reagenzien, die zur SPOT-Synthese (vergleiche hierzu allgemeine Arbeitsvorschrift 6) verwendet wurden, wurden über Molsieb gelagert: *N*-Methylpyrrolidon und Dimethylsulfoxid (beide: Fluka).

Reagenzien:

Die verwendeten Reagenzien wurden von den Firmen Advanced ChemTech (Bamberg, Deutschland), Sigma-Aldrich-Fluka (Deisenhofen, Deutschland), Bachem (Heidelberg, Deutschland), J.T. Baker (Phillipsburg, USA), Lancaster (Mühlheim/Main, Deutschland), Merck Eurolab (Darmstadt, Deutschland), Neosystem (Strassburg, Frankreich) oder Novabiochem (Bad Soden, Deutschland) bezogen und ohne weitere Aufreinigung verwendet. Zur SPOT-Synthese wurden Whatman 50 Zellulosemembranen (Whatman Maidstone, UK) verwendet, die auf die benötigte Grösse zurechtgeschnitten wurden.
Bei den Konzentrationen der Reagenzien in Prozent handelt es sich, sofern nicht anders angegebenen, um Volumenprozent (v/v).

RP-HPLC-MS-Analysen:

Chromatographie erfolgte unter Verwendung eines Hewlett Packard Serie 1100-Systems (Entgaser G1322A, Quaternäre Pumpe G1311A, Automatischer Probengeber G1313A, thermostatiertes Säulenfach G 1316A, Variabler UV-Detektor G1314A) und gekoppelter ESI-MS (Finnigan LCQ Ion-Trap-Massenspektrometer). Dazu wurde eine Steuersoftware der Firma Finnigan verwendet (Navigator Ver 1.1 sp1). Als Stossgas in der Ionenfalle diente Helium. Die Trennung erfolgte an RP-18-Säulenmaterial (Vydac 218 TP5215, 2,1 x 150 mm, 5 µm, C18, 300 A mit Vorsäule (Merk)) bei 30°C und einem Fluss von 0,3 ml/min unter Anwendung eines linearen Gradienten für alle Chromatogramme (5-95 % B innerhalb von 25 min, wobei A: 0,05 % TFA in Wasser und B: 0,05 % TFA in $CH_3CN$). Die UV-Detektion erfolgte bei $\lambda$ = 220 nm. Retentionszeiten ($R_t$) sind im Dezimalsystem angegeben (z.B. 1,9 min = 1 min 54 sec) und beziehen sich auf die Detektion im Massenspektrometer. Die Totzeit zwischen Injektion und UV-Detektion (HPLC) betrug 1,65

min, zwischen UV-Detektion und Massendetektion 0,21 min. Die Genauigkeit des Massenspektrometers beträgt ca. ± 0,2 amu.

Geräte für die SPOT-Synthese:

Die automatische SPOT-Synthese erfolgte mit dem Gerät *Autospot Robot ASP 222* (Abimed, Langenfeld, Deutschland) unter Anwendung der Steuersoftware Autospot XL Ver. 2.02. Die benötigten Steuerdateien, in denen Ort und Sequenzen der SPOTs festgelegt wurden, wurden mit den Programmen LISA und DIGEN (beide: Jerini AG, Berlin, Deutschland) angefertigt. Die Waschschritte erfolgten in Edelstahlschalen (Merck Eurolab), die auf einem Wipptisch (Labortechnik Fröbel, Lindau/Bodensee, Deutschland) bewegt wurden. Einzelne Reaktionsstellen ("SPOTs") wurden mit einem Bürolocher ausgestanzt und zur Weiterbehandlung in eine Mikrotiterplatte oder 2,0 ml Eppendorf-Reaktionsgefässe überführt. Thermomixer 5437, Zentrifuge 5475C und Vakuumzentrifuge 5301 der Firma Eppendorf wurden zur Behandlung von ausgestanzten SPOTs in Eppendorf-Gefässen verwendet. Zur Beschleunigung des Lösungsvorganges von Reagenzien für die SPOT-Synthese wurde ein Ultraschallbad verwendet (Sonomatic 300 PC).

Allgemeine Arbeitsvorschrift (AAV) 1: Bestimmung des Derivatisierungsgrades von aminoderivatisierten Zellulosemembranen

Der Derivatisierungsgrad lässt sich zuverlässig durch Messung der UV-Absorption des Dibenzofulven-Piperidin Adduktes C nach Abspaltung der Fmoc-Schutzgruppe einer membrangebundenen Aminosäure bestimmen, wie dies in Fig. 9 schematisch dargestellt ist.

Methode A: Quantifizierung freier Aminofunktionen:

Ein SPOT (0,23 $cm^2$) wird ausgestanzt und in einem 2,0 ml Eppendorf-Reaktionsgefäss mit 50 µl Fmoc-β-Ala-OPfp (0,6 M in DMF) versetzt. Nach 30 Minuten wird die Lösung entfernt und der SPOT mit DMF gewaschen (5 x 1,0 ml). In einem zweiten 1,5 ml Eppendorf-Reaktionsgefäss wird der SPOT mit 1,0 ml Piperidin (20 % in DMF) versetzt. Nach 20 Minuten wird die UV-Absorption der unverdünnten Lösung bei λ = 301 nm bestimmt [Vergleichszelle: Stammlösung Piperidin (20% in DMF)]. Aus der Extinktion (E) wird der Derivatisierungsgrad nach der unten dargestellten Formel berechnet ($\varepsilon_{301}$ = 8100). Bei hohen Derivatisierungsgraden (Extinktion > 1,5) muss die Messlösung verdünnt werden.

$$\text{Derivatisierungsgrad } [\mu\text{mol/cm}^2] = \frac{E \cdot V\,[\text{ml}] \cdot 1000}{8100 \cdot F[\text{cm}^2]} \cdot \frac{\mu\text{mol}}{\text{ml}}$$

mit E = Extinktion bei 301 nm; V = Reagenzvolumen, mit dem der SPOT versetzt wurde; F = Fläche des SPOTs (typischerweise 0,23 $cm^2$).

Methode B: Quantifizierung einer membrangebundenen Fmoc-Aminosäure:

Ein SPOT (0,23 $cm^2$) wird ausgestanzt und in einem 2 ml Eppendorf-Reaktionsgefäss mit 1,0 ml Piperidin (20% in DMF) versetzt. Zur Quantifizierung wird wie unter Methode A beschrieben verfahren.

Allgemeine Arbeitsvorschrift (AAV) 2: Glycin-Ester-derivatisierte Zellulosemembran

Dieses Verfahren liefert eine Fmoc-aminoderivatisierte Zellulosemembran, bei der Fmoc-Glycin über eine basenlabile Esterfunktion an die Oberfläche gebunden ist. In eine leere Instrumentenschale gibt man eine Zellulosemembran und inkubiert diese mit 40 ml einer Lösung aus 2,38 g (8,0 mmol) der zu kuppelnden (= immobilisierenden) Verbindung Fmoc-Gly-OH, 1,27 ml (16,0 mmol) der Base NMI und 1,56 ml (9,6 mmol) des Kupplungsreagenzes DIC in wasserfreiem DMF für drei Stunden. Anschliessend wird die Membran gewaschen (DMF, 3 x 10 min; MeOH, 2 x 5 min; DCM, 1 x 2min; $Et_2O$, 1 x 2 min). In einem erweiterten Waschschritt werden freie Aminogruppen durch Acetylierung blockiert (DMF, 2 x 2 min; DMF/$Ac_2O$/DIPEA [70:10:20 (v/v), 2 x 15 min]), und erneut gewaschen (DMF, 3 x 2 min; MeOH, 2 x 2 min; DCM, 1 x 2 min; $Et_2O$, 1 x 2 min) und anschliessend an der Luft getrocknet. Anhand von drei SPOTs, die nur zu diesem Zweck vorgesehen wurden, wird der Derivatisierungsgrad nach AAV 1B bestimmt.

Allgemeine Arbeitsvorschrift (AAV) 3: Manuelle Peptidsynthese an planaren Oberflächen unter SPOT-Bedingungen nach dem Stand der Technik unter Verwendung von OPfp-Estern.

In Fig. 10 ist schematisch die Durchführung der manuellen Peptidsynthese nach dem Verfahren nach dem Stand der Technik unter Verwendung von OPfp-Estern dargestellt.

Auf eine aminoderivatisierte planare Oberfläche wird mit Bleistift ein 1 x 1 cm Raster aufgezeichnet. Die

Membran wird stets in einer Edelstahl-Instrumentenschale behandelt. Die Waschschritte werden unter Bewegung des Lösungsmittels auf einem Wipptisch durchgeführt. Mittels einer Eppendorf Multi-Step-Pipette wird in die Mitte der Rasterkreuze 2 µl (auf Zellulose) oder 1 µl (auf PP-Membran) einer 0,6 M Aminosäure-Pentafluorphenolesterlösung (d. h. eines zuvor synthetisierten Aktivesters) in NMP gespottet. Das Spotten der Reagenzien wird nach jeweils 20 min zweimal wiederholt. Nach beendeter Kopplung wird der Überschuss durch Waschen der Membran entfernt (DMF, 3 x 2 min; MeOH, 2 x 2 min; DCM, 1 x 2 min; $Et_2O$, 1 x 2 min) und anschliessend an der Luft getrocknet. Nicht acylierte freie Aminogruppen werden durch Baden mit einer 20 %-igen Acetanhydrid-Lösung mit 10 % DIPEA in DMF acetyliert, um den Aufbau von Fehlsequenzen zu vermeiden. Anschliessend wird die Membran erneut gewaschen. Die N-terminale Fmoc-Schutzgruppe der Peptide wird durch Inkubation mit einer 20 % Piperidin-Lösung in DMF für 20 min entfernt. Nach Waschen (DMF, 3 x 2 min; MeOH, 2 x 2 min; DCM, 1 x 2 min; $Et_2O$, 1 x 2 min) und Trocknen an der Luft ist die Membran für die Kopplung der nächsten Aminosäure bereit, so dass die Synthesecyclen solange durchlaufen werden können, bis die gewünschte Peptidsequenz erreicht ist.

Ein Unterbrechen oder Abbrechen der Synthese kann nach der Kopplung der Aminosäuren oder Acetylierung erfolgen.

Allgemeine Arbeitsvorschrift (AAV) 4: Halbautomatische SPOT-Synthese von Peptiden

Die halbautomatische Synthese einer Peptid-Bibliothek erfolgt in Analogie zu der manuellen SPOT-Synthese (AAV 3). Folgende Unterschiede werden dabei jedoch realisiert:

- Die Pipettierschritte werden unter Verwendung eines Pipettierroboters (*Autospot Robot ASP 222)* durchgeführt. Die Steuerdateien, in denen Ort und Sequenzen der SPOTs festgelegt ist, werden mit dem Programm LISA erzeugt. In einer Synthese können gleichzeitig 40 Bausteine verwendet werden. Die Acetylierung mit Acetanhydrid und die Abspaltung der Fmoc-Schutzgruppe erfolgt nicht am Pipettierroboter, sondern durch Inkubation der Membran in einer Metallschale mit der entsprechenden Lösung (AAV 3 ).
- Um die Repositionierung der Membran nach den Waschschritten zu ermöglichen, werden freie Aminogruppen durch Waschen mit einer 0,01%igen Lösung von Bromphenolblau in MeOH angefärbt und ausgewählte SPOTs (z.B. Ecken der Membran) mit Bleistift markiert.

Allgemeine Arbeitsvorschrift (AAV) 5: Abspaltung der Syntheseprodukte von den verschiedenen Linkersystemen

Im Rahmen dieser Vorschrift wird beschrieben, wie ausgehend von einer Oberfläche, an die über eine Linkerstruktur eine Reihe von Verbindungen konsekutiv immobilisiert wurde, und somit ein Syntheseprodukt erhalten wurde, das Syntheseprodukt durch Abspalten von der Linkerstruktur freigesetzt wird.

Zur Seitenkettenschutzgruppen-Abspaltung wird das Trägermaterial, hier die Membran, auf der gemäß AAV 3 oder AAV 4 Peptide synthetisiert wurden, mit TFA behandelt [$TFA/H_2O/TIPS$ [95/3/2 (v/v)] unter Zusatz von Phenol (1 g/100ml), 2 x 15 min; $TFA/CH_2Cl_2/H_2O/TIPS$ [50/45/3/2 (v/v)] unter Zusatz von Phenol (1 g/100ml), 1 x 2 h]. Die verwendete Instrumentenschale wird dabei nicht geschüttelt. Im Anschluss wird die Membran gewaschen ($CH_2Cl_2$, 2 x 5 min; DMF, 3 x 2 min; MeOH, 2 x 2 min; $Et_2O$, 1 x 2 min) und an der Luft getrocknet.

Die über eine Esterbindung an die Cellulose gebundenen Peptide werden durch Methylamin in einem Exsikkator innerhalb von 14 h oder durch 30minütige Behandlung mit 0.1 M $TEA/H_2O$ abgespalten.

Die Bereiche der Membran, auf denen ein Peptid synthetisiert wurde ("Spots"), werden ausgestanzt und können in einem geeigneten Lösungsmittel abgelöst für Assays in Lösung eingesetzt oder analysiert (HPLC, HPLC-MS) werden.

Die adsorbierten Syntheseprodukte werden dafür mit Acetonitril (50 % in $H_2O$ mit 0,1 % TFA, 100 µl) abgelöst und analysiert (HPLC, HPLC-MS, 10 µl Injektionsvolumen).

**Beispiel 2: Vergleich zwischen Voraktivierungen der zu immobilisierenden Verbindung auf der Oberfläche eines Trägermaterials und in Lösung mit HBTU (1 eq.) und DIPEA (2 eq.)**

Im Rahmen dieses Beispiels wurden verschiedene Peptide zum einen unter Anwendung des erfindungsgemäßen Verfahrens, d. h. durch Voraktivieren der jeweils zu immobilisierenden Verbindung (d.h. der jeweiligen Aminosäure) auf der Oberfläche eines Trägermaterials, genauer einer Membran, dargestellt in den Figuren 11A bis 11E als a) und durch Voraktivierung der jeweils zu immobilisierenden Verbindung (d.h. der jeweiligen Aminosäure) in Lösung, dargestellt in den Figuren 11A bis 11E als b) synthetisiert.

a) Voraktivierung auf der Membran:

Die in Fig. 11A-E aufgeführten Peptide wurden gemäß folgendem Protokoll synthetisiert: Das Kupplungsreagenz HBTU wird in einer Konzentration von 0.3 M in Acetonitril gelöst. Je 1.2 µl dieser Lösung spottet man mit Hilfe eines automatischen Spotters *(Abimed ASP 222)* auf die Reaktionsfläche einer Glycinester-modifizierten Zellulosemembran. Nach Verdampfen des Lösungsmitteles (ca. 20 min) wird der Vorgang wiederholt. Wieder läßt man das Acetonitril verdampfen und spottet dann zweimal im Abstand von ca. 30 min eine Lösung der zu kuppelnden Verbindung Fmoc-AS-OH (0.6 M) und der Base DIPEA (1.2 M) in NMP (1.2 µl). Nach ca. 45minütiger Reaktionszeit wäscht man die gesamte Membran 5x mit DMF, 3x mit Methanol, acetyliert gemäß AAV 3 und spaltet die Fmoc-Schutzgruppe gemäß AAV 3 ab. Der gesamte Vorgang wird mit der jeweils benötigten Aminosäure wiederholt, bis das gewünschte Peptid synthetisiert ist. Nach erneuter Acetylierung und Abspaltung der Seitenschutzgruppen mit TFA (AAV 5) wird das Peptid von der Zellulose abgespalten (AAV 5) und per HPLC analysiert. Die erhaltenen Chromatogramme sind in Fig. 11 A-E dargestellt. Der Pfeil zeigt hierbei das gewünschte Produkt.

b) Voraktivierung in Lösung:

Die in Fig. 11A-E aufgeführten Peptide wurden gemäß folgendem Protokoll synthetisiert: Eine Lösung des Kupplungsreagenzes HBTU (0.6 M) in NMP und eine Lösung der zu kuppelnden Verbindung Fmoc-AS-OH (0.6 M) in NMP werden im Volumenverhältnis 1:1 gemischt und mit 2 eq. der Base DIPEA versetzt. Nach ca. 45-minütigem Stehenlassen spottet man 1.2 µl dieser Lösung mit Hilfe eines automatischen Spotters (*Abimed ASP 222*) auf die Reaktionsfläche einer Glycinester-modifizierten Zellulosemembran. Diesen Vorgang wiederholt man nach ca. 45 min, läßt weitere 45 min stehen, wäscht die gesamte Membran 5x mit DMF, 3x mit Methanol, acetyliert gemäß AAV 3 und spaltet die Fmoc-Schutzgruppe gemäß AAV 3 ab. Der gesamte Vorgang wird mit der jeweils benötigten Aminosäure wiederholt, bis das gewünschte Peptid synthetisiert ist. Nach erneuter Acetylierung und Abspaltung der Seitenschutzgruppen mit TFA (AAV 5) wird das Peptid von der Zellulose abgespalten (AAV 5) und per HPLC analysiert. Die erhaltenen Chromatogramme sind in Fig. 11A-E dargestellt. Der Pfeil zeigt hierbei das gewünschte Produkt.

Es zeigt sich, dass durch Voraktivierung der zu immobilisierenden Verbindungen auf der Membran (und dabei speziell durch Spotten des Kupplungsreagenzes HBTU und anschließendem Spotten eines Gemisches aus der zu kuppelnden Verbindung Fmoc-AS-OH und der Base DIPEA) unter Anwendung des erfindungsgemäßen Verfahrens höhere Reinheiten der synthetisierten Peptide erreicht werden können, als durch Voraktivierung in Lösung. Eine mögliche Erklärung für den Effekt ist das Auftreten einer sehr reaktiven, aber kurzlebigen Aktivspecies.

**Beispiel 3: Vergleich zwischen Voraktivierungen der zu immobilisierenden Verbindung auf einer Oberfläche eines Trägermaterials und in Lösung mit HBTU (1 eq.) und DIPEA (2 eq.)**

Im Rahmen dieses Beispiels wurden verschiedene Peptide zum einen unter Anwendung des erfindungsgemäßen Verfahrens, d. h. durch Voraktivieren der jeweils zu immobilisierenden Verbindung (d.h. der jeweiligen Aminosäure) auf der Oberfläche eines Trägermaterials, genauer einer Membran, dargestellt in den Figuren 12A bis 12C als a), und zum anderen durch Voraktivierung der jeweils zu immobilisierenden Verbindung (d.h. der jeweiligen Aminosäure) in Lösung, dargestellt in den Figuren 12A bis 12C als b).

a) Voraktivierung auf der Membran:

Die in Fig. 12A-C aufgeführten Peptide wurden gemäß folgendem Protokoll synthetisiert: Eine Glycinester-modifizierte Zellulosemembran (52 $cm^2$) wird mit 1 ml einer gesättigten Lösung des Kupplungsreagenzes HBTU in Acetonitril behandelt. Nach Verdampfen des Lösungsmittels (ca. 20 min) spottet man mit Hilfe eines automatischen Spotters *(Abimed ASP* 222) 1.2 µl einer Lösung der zu kuppelnden Verbindung Fmoc-AS-OH (0.6 M) und der Base DIPEA (1.2 M) in NMP auf die Reaktionsfläche der Membran. Nach 15 min wird dieselbe Lösung nochmal auf die Reaktionsfläche aufgebracht und weitere 15 min gewartet. Man wäscht die gesamte Membran 5x mit DMF, 3x mit Methanol und spaltet die Fmoc-Schutzgruppe gemäß AAV 3 ab. Der gesamte Vorgang wird mit der jeweils benötigten Aminosäure wiederholt, bis das gewünschte Peptid synthetisiert ist. Man spaltet das Peptid mit TEA/Wasser von der Zellulose ab (AAV 5) und analysiert per HPLC-MS. Die erhaltenen Chromatogramme sind in Fig. 12A-C dargestellt. Der Pfeil zeigt hierbei das gewünschte Produkt.

b) Voraktivierung in Lösung:

Die in Fig. 12A-C aufgeführten Peptide wurden gemäß folgendem Protokoll synthetisiert: Eine Lösung des Kupplungsreagenzes HBTU (0.6 M) in NMP und eine Lösung der zu kuppelnden Verbindung Fmoc-AS-OH (0.6 M) in

NMP werden im Volumenverhältnis 1:1 gemischt und mit 2 eq. Der Base DIPEA versetzt. Nach ca. 5minütigem Schütteln spottet man 1.2 µl dieser Lösung mit Hilfe eines automatischen Spotters (*Abimed ASP 222*) auf die Reaktionsfläche einer Glycinester-modifizierten Zellulosemembran. Diesen Vorgang wiederholt man nach ca. 15 min, lässt weitere 15 min stehen, wäscht die gesamte Membran 5x mit DMF, 3x mit Methanol, acetyliert gemäß AAV 3 und spaltet die Fmoc-Schutzgruppe gemäß AAV 3 ab. Der gesamte Vorgang wird mit der jeweils benötigten Aminosäure wiederholt, bis das gewünschte Peptid synthetisiert ist. Das Peptid wird von der Zellulose abgespalten (AAV 5) und per HPLC analysiert. Die erhaltenen Chromatogramme sind in Fig. 12A-C dargestellt. Der Pfeil zeigt hierbei das gewünschte Produkt.

Es zeigt sich, dass durch Voraktivierung der zu immobilisierenden Verbindung auf der Membran (und dabei speziell durch Baden mit einer Lösung des Kupplungsreagenzes HBTU und anschließendem Spotten eines Gemisches aus der immobilisierenden oder zu kuppelnden Verbindung Fmoc-AS-OH und der Base DIPEA) unter Anwendung des erfindungsgemäßen Verfahrens höhere Reinheiten der synthetisierten Peptide erreicht werden können, als durch Voraktivierung in Lösung. Eine mögliche Erklärung für den Effekt ist das Auftreten einer sehr reaktiven, aber kurzlebigen Aktivspecies.

**Beispiel 4: Einfluß der Voraktivierungszeit auf die Kupplungszeit bei Voraktivierungen auf der Membran und in Lösung mit HBTU (1 eq.) und DIPEA (2 eq.)**

Das Ergebnis dieses Beispiels ist in Fig. 13 dargestellt, wobei hier konkret der Anteil an gewünschtem Hauptprodukt RRFG bei der Kopplung von R auf RFG bei verschiedenen Voraktivierungszeiten dargestellt ist. Genauer erstreckt sich das Voraktivierungszeitintervall von 5 Min bis 7,5 h. Mit b) ist dabei das Ergebnis unter Anwendung des erfindungsgemäßen Verfahrens, d. h. Voraktivierung auf der Membran, bezeichnet, wohingegen a) die Voraktivierung in Lösung gemäß den Verfahren nach dem Stand der Technik bezeichnet

a) Voraktivierung auf der Membran:

Eine Glycinester-modifizierte Zellulosemembran (52 $cm^2$), auf der gemäß AAV-5 die Peptidsequenz RF-G synthetisiert wurde, wird mit 1 ml einer gesättigten Lösung des Kupplungsreagenzes HBTU in Acetonitril behandelt. Nach Verdampfen des Lösungsmittels (ca. 20 min) und Ablauf der gewünschten Wartezeit (5 min bis 7.5 h) spottet man auf die Reaktionsfläche 1.2 µl einer Lösung der zu immobilisierenden bzw. zu kuppelnden Verbindung Fmoc-AS-OH (0.6 M) und der Base DIPEA (1.2 M) in NMP, welche ebenfalls für die Dauer der "Wartezeit" bei Raumtemperatur an der Luft stehengelassen wurde. Nach 15 min wird dieselbe Lösung nochmal auf die Reaktionsfläche aufgebracht und weitere 15 min gewartet. Man wäscht die gesamte Membran 5x mit DMF, 3x mit Methanol und spaltet die Fmoc-Schutzgruppe gemäß AAV 3 ab. Man spaltet das Peptid mit TEA/Wasser von der Zellulose ab (AAV 5) und analysiert per HPLC-MS. Die erhaltenen Chromatogramme ergeben den Anteil an gewünschtem Produkt RRFG. Als Nebenprodukt wurde das Edukt RFG identifiziert.

b) Voraktivierung in Lösung:

Eine Lösung des Kupplungsreagenzes HBTU (0.6 M) in NMP und eine Lösung der zu immobilisierenden oder zu kuppelnden Verbindung Fmoc-AS-OH (0.6 M) in NMP werden im Volumenverhältnis 1:1 gemischt und mit 2 eq. der Base DIPEA versetzt. Nach Ablauf der gewünschten Voraktivierungszeit (5 min bis 7.5 h) spottet man 1.2 µl dieser Lösung auf die Reaktionsfläche einer Glycinester-modifizierten Zellulosemembran, auf der gemäß AAV-5 die Peptidsequenz RF-G synthetisiert wurde. Diesen Vorgang wiederholt man nach ca. 15 min, lässt weitere 15 min stehen, wäscht die gesamte Membran 5x mit DMF, 3x mit Methanol, acetyliert gemäß AAV 3 und spaltet die Fmoc-Schutzgruppe gemäß AAV 3 ab. Das Peptid wird von der Zellulose abgespalten (AAV 5) und per HPLC analysiert. Die erhaltenen Chromatogramme ergeben den Anteil an gewünschtem Produkt RRFG. Als Nebenprodukt wurde das Edukt RFG identifiziert.

Die erfindungsgemäße Vorbehandlung der Membran mit HBTU hat deutliche Vorteile gegenüber der konventionellen Methode der Voraktivierung in Lösung. So erhält man schon bei kurzer Voraktivierungszeit (5 min) mit Methode b) ca. 5 % mehr gewünschtes Produkt als mit Methode a).

Besonders auffällig wird der Unterschied der Aktivierungsmethoden aber bei längeren Aktivierungszeiten (ca. 4-7.5 Stunden). Diese Aktivierungszeiten treten dann auf, wenn sehr viele Peptide (z.B. 600) simultan auf einer planaren Membran synthetisiert werden. Mit Hilfe der bisher verwendeten Methode a) erhält man nach 4 Stunden nur noch ca. die Hälfte, nach 8 Stunden sogar nur noch ca. ein Zehntel der mit kurzer Voraktivierungszeit erreichbaren Produktausbeute.

Das erfindungsgemäße Verfahren b) führt zu Ausbeuten, die nahezu unabhängig von der Voraktivierungszeit sind. Selbst nach einer sehr langen Voraktivierungszeit von 7,5 Stunden erhält man das gewünschte Produkt RRFG in einer guten Ausbeute von 45 %, was ca. der zehnfachen Menge der Ausbeute nach Methode a) entspricht.

Die Ursache für die guten Ausbeuten nach Methode b) liegen wahrscheinlich in der Aufbewahrung der Reagenzien. Sowohl festes HBTU auf der Membran als auch Fmoc-AS-OH mit DIPEA in NMP zeigen eine hohe Stabilität, welche die der empfindlichen Aktivspecies X7 deutlich übertrifft.

**Beispiel 5: Vergleich der Syntheseausbeute bei Voraktivierungen auf einer Oberfläche eines Trägermaterials mit HBTU (1 eq.) und DIPEA (2 eq.) und vorsynthetisierten OPfp-Estern**

Das Ergebnis dieses Vergleichs ist in Tabelle 1 dargestellt, wobei Methode a) Voraktivierung auf einer Oberfläche eines Trägermaterials, genauer einer Membran, gemäß dem erfindungsgemäßen Verfahren und Methode b) Synthese mit vorsynthetisierten OPfp-Estern gemäß dem Verfahren nach dem Stand der Technik (AAV 3-5) bezeichnet.

Tabelle 1:

| AS-Sequenz | Methode b) (OPfp-Ester) | Methode a) (HBTU) |
|---|---|---|
| | | |
| Ac-yshqienpfp-G | 24% | 62% |
| Ac-rfgdipqqky-G | 70% | 68% |
| Ac-edfphqqais-G | 53% | 85% |
| Ac-nypinarlvd-G | 43% | 75% |
| Ac-ilaqsvqrsq-G | 81% | 81% |
| Ac-pkvlhkveas-G | 85% | 63% |
| Ac-ikghgvgegl-G | 48% | 60% |
| Ac-vgdgfhnlka-G | 41% | 69% |
| Ac-regkaykrls-G | 62% | 57% |
| Ac-yiieqansiv-G | 0% | 0% |
| | **Ø 51%** | **Ø 59%** |

Es wird deutlich, dass durch Voraktivierung auf der Membran (speziell dem Spotten eines Gemisches aus der zu kuppelnden Verbindung Fmoc-AS-OH und des Kupplungsreagenzes HBTU sowie dem anschließenden Spotten der Base DIPEA) höhere Reinheiten der synthetisierten Peptide erreicht werden können, als durch die Verwendung vorsynthetisierter Pentafluorphenylester.

Die Ergebnisse wurden dabei unter Anwendung der folgenden Verfahren erhalten:

a) Voraktivierung auf der Membran:

Die in Tabelle 1 aufgeführten Peptide wurden gemäß folgendem Protokoll synthetisiert: Das Kupplungsreagenz HBTU und die jeweils zu kuppelnde Verbindung Fmoc-AS-OH werden in einer Konzentration von je 0.5 M in DMF gelöst. Je 1.2 µl dieser Lösung spottet man mit Hilfe eines automatischen Spotters (*Abimed ASP 222*) auf die Reaktionsfläche einer Glycinester-modifizierten Zellulosemembran. Nach 10-minütigem Warten und einminütigem Fönen mit einem handelsüblichen Haartrockner spottet man darauf eine Lösung der Base DIPEA (0.9 M) in NMP (1.2 µl). Man läßt die Membran 30 min liegen, fönt für 1-2 min und wiederholt den gesamten Vorgang. Dann wäscht man die gesamte Membran 5x mit DMF, 3x mit Methanol, acetyliert gemäß AAV 3 und spaltet die Fmoc-Schutzgruppe gemäß AAV 3 ab. Der gesamte Vorgang wird mit der jeweils benötigten Aminosäure wiederholt, bis das gewünschte Peptid synthetisiert ist. Nach erneuter Acetylierung und Abspaltung der Seitenschutzgruppen mit TFA (AAV 5) wird das Peptid von der Zellulose abgespalten (AAV 5) und per HPLC analysiert. Die erhaltenen Reinheiten der Peptide sind in Tabelle 1 dargestellt.

b) Synthese mit vorsynthetisierten OPfp-Estern:

Die in Tabelle 1 aufgeführten Peptide wurden gemäß folgendem Protokoll synthetisiert: Eine Lösung von kommerziell erhältlichen Fmoc-AS-OPfp (0.6 M) in NMP (1.2 µl) spottet man mit Hilfe eines automatischen Spotters (*Abimed ASP 222*) auf die Reaktionsfläche einer Glycinester-modifizierten Zellulosemembran. Diesen Vorgang wiederholt man nach ca. 45 min, lässt weitere 45 min stehen, wäscht die gesamte Membran 5x mit DMF, 3x mit Methanol, acetyliert gemäß AAV 3 und spaltet die Fmoc-Schutzgruppe gemäß AAV 3 ab. Der gesamte Vorgang wird mit der jeweils benötigten Aminosäure wiederholt, bis das gewünschte Peptid synthetisiert ist. Nach erneuter Acetylierung und Abspaltung der Seitenschutzgruppen mit TFA (AAV 5) wird das Peptid von der Zellulose abgespalten (AAV 5) und per HPLC analysiert. Die erhaltenen Reinheiten der Peptide sind in Tabelle 1 dargestellt.

**Beispiel 6: Vergleich der Reinheit des Syntheseproduktes bei Voraktivierungen auf einer Oberfläche eines Trägermaterials mit HBTU (1 eq.) und DIPEA (3 eq.) und Voraktivierung der zu immobilisierenden Verbindung in Lösung**

Das Ergebnis dieses Vergleichs ist in Tabelle 2 dargestellt als HPLC-Reinheit der verscheidenden synthetisierten Aminosäuresequenzen, wobei a) die Voraktivierung auf der Membran und b) die Voraktivierung in Lösung bezeichnet.

Tabelle 2:

| AS-Sequenz | Methode b) | Methode a) |
|---|---|---|
| | (Vorakt. In Lösung) | (Vorakt. auf der Membran) |
| | | |
| H-Ile-Ser(OP(OH)OBzl)-Thr($^t$Bu)-Phe-Gly-OH | 38% | 63% |
| H-Ile-Thr(OP(OH)OBzl)-Thr($^t$Bu)-Phe-Gly-OH | 44% | 57% |
| H-Ile-Tyr(OP(OH)OBzl)-Thr($^t$Bu)-Phe-Gly-OH | 53% | 78% |
| H-Ile-Tyr((OMDPSE)$_2$)-Thr($^t$Bu)-Phe-Gly-OH | 29% | 63% |

Aus den in Tabelle 2 gezeigten Daten wird ersichtlich, dass durch Voraktivierung auf der Membran (speziell dem Spotten eines Gemisches aus der zu kuppelnden Verbindung Fmoc-AS-OH und des Kupplungsreagenzes HBTU sowie dem anschließenden Spotten der Base DIPEA) höhere Reinheiten der synthetisierten Peptide erreicht werden können, als durch ein Verfahren, bei dem die einzelnen, für den Aufbau des Peptids zu immobilisierenden Aminosäuren durch Voraktivierung in Lösung bereitgestellt wurden.

a) Voraktivierung auf der Membran:

Die in Tabelle 2 aufgeführten Peptide wurden gemäß dem folgenden Protokoll synthetisiert: Das Kupplungsreagenz HBTU und die jeweils zu kuppelnde Verbindung Fmoc-AS-OH werden in einer Konzentration von je 0.5 M in DMF gelöst. Je 1.2 µl dieser Lösung spottet man mit Hilfe eines automatischen Spotters (*Abimed ASP 222*) auf die Reaktionsfläche einer Glycinester-modifizierten Zellulosemembran, auf der gemäß AAV-5 die Peptidsequenz TF-G synthetisiert wurde. Nach 10minütigem Warten und einminütigem Fönen mit einem handelsüblichen Haartrockner spottet man darauf eine Lösung der Base DIPEA (1.35 M) in NMP (1.2 µl). Man lässt die Membran 30 min liegen, fönt für 1-2 min und wiederholt den gesamten Vorgang. Dann wäscht man die gesamte Membran 5x mit DMF, 3x mit Methanol, acetyliert gemäß AAV 3 und spaltet die Fmoc-Schutzgruppe gemäß AAV 3 ab. Der gesamte Vorgang wird mit der jeweils benötigten Aminosäure wiederholt, bis das gewünschte Peptid synthetisiert ist. Nach erneuter Acetylierung wird das Peptid von der Zellulose abgespalten (AAV 5) und per HPLC analysiert. Die erhaltenen Reinheiten der Peptide sind in Tabelle 2 dargestellt.

b) Voraktivierung in Lösung:

Die in Tabelle 2 aufgeführten Peptide wurden gemäß folgendem Protokoll synthetisiert: Eine Lösung von HBTU (0.6 M) in NMP und eine Lösung von Fmoc-AS-OH (0.6 M) in NMP werden im Volumenverhältnis 1:1 gemischt

und mit 3 eq. DIPEA versetzt. Nach ca. 45-minütigem Stehenlassen spottet man 1.2 µl dieser Lösung mit Hilfe eines automatischen Spotters (*Abimed ASP 222*) auf die Reaktionsfläche einer Glycinester-modifizierten Zellulosemembran. Diesen Vorgang wiederholt man nach ca. 45 min, läßt weitere 45 min stehen, wäscht die gesamte Membran 5x mit DMF, 3x mit Methanol, acetyliert gemäß AAV 3 und spaltet die Fmoc-Schutzgruppe gemäß AAV 3 ab. Der gesamte Vorgang wird mit der jeweils benötigten Aminosäure wiederholt, bis das gewünschte Peptid synthetisiert ist. Nach erneuter Acetylierung wird das Peptid von der Zellulose abgespalten (AAV 5) und per HPLC analysiert. Die erhaltenen Reinheiten der Peptide sind in Tabelle 2 dargestellt.

**Beispiel 7: Einfluss verschiedener Lösungsmittel auf die Kupplungseffizienz bei Voraktivierung der zu immobilisierenden Verbindung auf der Oberfläche**

Im Rahmen dieses Beispiels wurde der Einfluss erschiedener Lösungsmittel im Rahmen des erfindungsgemäßen Verfahrens untersucht.

Das Kupplungsreagenz HBTU wird in der maximal möglichen Konzentration im jeweiligen Lösungsmittel gelöst. Je 1.2 µl dieser Lösung spottet man auf die Reaktionsfläche einer Glycinester-modifizierten Zellulosemembran. Nach Verdampfen des Lösungsmittels (ca. 20 min) wird der Vorgang wiederholt. Wieder läßt man das Acetonitril verdampfen und spottet dann zweimal im Abstand von ca. 30 min eine Lösung der zu immobilisierenden oder zu kuppelnden Verbindung Fmoc-Gly-OH (0.6 M) und der Base DIPEA (1.2 M) in NMP (1.2 µl). Nach ca. 45minütiger Reaktionszeit wäscht man die gesamte Membran 5x mit DMF, 3x mit Methanol, 2x mit Dichlormethan und bestimmt die Ausbeute der Kupplung der Aminosäure durch photometrische Quantifizierung des membrangebundenen Fmoc.

Das Ergebnis ist in Tabelle 3 dargestellt als Ausbeute der Kupplung von Fmoc-Gly-OH auf eine Glycinester-modifizierte Zellulosemembran unter verschiedenen Bedingungen.

Tabelle 3:

| Lösungsmittel | Konzentration an HBTU [mM] | Ausbeute [%][a] |
|---|---|---|
| Acetonitril | 0,39 | 98 |
| 1,2-Dibromethan | < 0,05 | 40 |
| Dimethylacetamid | 0,4 | 83 |
| Dimethylacetamid + 5%LiCl | 0,1 | 23 |
| *N,N*- Dimethylformamid | 0,64 | 66 |
| Dimethylsulfoxid (vorgetrocknet) | 0,65 | 78 |
| Dimethylsulfoxid | 0,6 | 76 |
| *N*- Methylpyrrolidon | 0,5 | 63 |

[a] lt. photometrischer Quantifizierung des membrangebundenem Fmoc (Ausgangsbeladung der Glycinester-modifizierten Cellulosemembran: 892 ± 5 nmol/$cm^2$)

Die in Tabelle 3 dargestellten Ergebnisse zeigen, dass eine Vielzahl von Lösungsmitteln geeignet ist, das Kupplungsreagenz HBTU effektiv auf die Membran zu bringen. Besonders günstig sind die Lösungsmittel Acetonitril, DMA und DMSO.

**Beispiel 8: NMR-Untersuchungen zur Voraktivierung von Fmoc-Ala-OH mit HBTU in Anwesenheit oder Abwesenheit von Base**

Im Rahmen dieses Beispiels wurde wie folgt vorgegangen:

Die Carbonsäure Fmoc-Ala-OH und das Kupplungsreagenz HBTU werden je 0.45 M in DMF-d7 (0.8 ml) gelöst und die Lösung in ein verschlossenes NMR-Rohr überführt. Nach 15 min und eintägigem Stehenlassen bei Raumtemperatur nimmt man jeweils ein $^{13}$C-NMR-Spektrum auf, das in den Figs 14A - B dargestellt ist. Man gibt anschließend die Base DIPEA (0.90 M) zu und misst ein $^{13}$C-NMR-Spektrum nach 10 min sowie einem Tag (Fig. 14 C - D).

Das Ergebnis ist in Fig. 14 dargestellt, die in den Teilen A - D Ausschnitte von $^{13}$C-NMR-Spektren (180-150 ppm) von Fmoc-Ala-OH, HBTU (je 0,45 M in DMF-d7) und optional DIPEA (0.9 M) zeigt. Dabei sind die vorstehend beschriebenen Reaktionsbedingungen bei den verschiedenen Teilen von Fig. 14 wie folgt realisiert:

A: 15 min stehen bei RT

B: 1 Tag stehen bei RT
C: 1 Tag stehen bei RT, +DIPEA (0.9 M), 10 min stehen bei RT
D: 1 Tag stehen bei RT, +DIPEA (0.9 M), 1 Tag stehen bei RT

Die NMR-Spektren der Carbonsäure Fmoc-Ala-OH und des Kupplungsreagenzes HBTU in DMF-d7 zeigen, dass die Verbindungen nicht miteinander reagieren, da ausschließlich Signale für das COOH der Carbonsäure und das "positiv geladene C-Atom" des HBTU zu erkennen sind (Fig. 14A). Selbst nach einem Tag bildet sich aus HBTU und Fmoc-Ala-OH keine Aktivspecies (Fig. 14B). Die Tatsache, dass die Verbindungen nicht miteinander reagieren, ist der Grund dafür, warum die Lösungen so erstaunlich stabil sind (vgl. Beispiel 9).

Eine Aktivierung der Aminosäure erhält man erst, wenn man eine Base zugibt, wobei die Aktivierung mit DIPEA vollständig ist, da die Signale für das COOH und das positiv geladene C-Atom nach DIPEA-Zugabe verschwinden und ein Signal für das C=O des Aktivesters entsteht (Fig. 14C). Der Aktivester zersetzt sich im folgenden wieder und nach einem Tag liegt nur noch freie Carbonsäure vor (Fig. 14D).

Dieses Beispiel zeigt eindrucksvoll die deutlich höhere Stabilität der Lösung von nicht aktivierter Carbonsäure (Fmoc-Ala-OH und HBTU in DMF) gegenüber der Lösung von aktivierter Carbonsäure (Fmoc-Ala-OH, HBTU und DIPEA in DMF).

**Beispiel 9: Einfluss verschiedener Lagerungsbedingungen auf die Aktivität von Lösungen aus verschiedenen Aminosäuren und dem Kupplungsreagenz HBTU**

Es werden je 100 µl von Lösungen verschiedener Fmoc-AS-OH und dem Kupplungsreagenz HBTU (je 0.45 M) in wasserfreiem DMF (DMF, puriss., absolut, über Molekularsieb, $H_2O$ ≤0.01 % der Fa. Sigma-Aldrich Chemie GmbH) hergestellt. Man lagert die Lösungen unter verschiedenen Bedingungen:

1) in einem 1 ml-Plastikgefäß mit geöffnetem Deckel für 4 Stunden, RT,

2) in einem 1 ml-Plastikgefäß mit geöffnetem Deckel für 16 Stunden, RT,

3) in einem 1 ml-Plastikgefäß mit geschlossenem Deckel für 6 Tage, -20°C.

Anschließend werden je 2 µl der entsprechenden Lösung zu 45 µl einer Lösung von H-Gly-OEt.HCl (0.1 M) und DIPEA (0.3 M) in NMP gegeben. Nach 90-minütigen Stehenlassen analysiert man ein Aliquot der Lösung per HPLC.

Bei der Zusammengabe der beiden Lösungen findet eine Kupplung der Fmoc-AS-OH auf die freie Aminogruppe des H-Gly-OEt statt. Wenn sich unter den Lagerungsbedingungen kein HBTU zersetzt hat, läuft die Kupplung vollständig ab, und im HPLC-Spektrum ist nur die Verbindung Fmoc-AS-Gly-OEt nachweisbar. Wenn aber ein Teil des HBTU zu inaktiven Verbindungen abreagiert ist, so zeigt das HPLC-Spektrum neben dem gewünschten Kupplungsprodukt Fmoc-AS-Gly-OEt auch freie Aminosäure Fmoc-AS-OH.

Der Anteil an gewünschtem Kupplungsprodukt an den Peakflächen im HPLC wird als Aktivität oder Restaktivität der jeweiligen Lösung von Fmoc-AS-OH, HBTU (und. evtl. DIPEA) definiert.

Das Ergebnis ist in Fig. 15 dargestellt, wobei Fig. 15 die Aktivität von Lösungen aus verschiedenen Aminosäuren und dem Kupplungsreagens HPTU bei verschiedenen Lagerungsbedingungen zeigt. Fig. 15A zeigt dabei Fmoc-AS-OH, HBTU (je 0,45 M in DMF) und Fig. 15B Fmoc-AS-OH, HBTU (je 0,45 M in DMF), DIPEA (1,2 M).

Aus den Figuren ist ersichtlich, dass Gemische aus HBTU und Aminosäure erstaunlich stabil sind. Nach vierstündiger offener Lagerung (dunkle Balken) kuppelt noch ca. 90 % der Aminosäuren auf den Glycin-Ethylester, und selbst nach einem Tag an der Luft (helle Balken) ist immer noch ca. 60 % der Lösung aktiv. Die einzige größere Ausnahme hiervon stellt His dar.

Dies belegt, dass die Lösungen erheblich stabiler sind, als wenn die Aminosäuren mit HBTU und DIPEA in Lösung voraktiviert werden (Fig. 15B). Außerdem kann man auf der Grundlage dieser Ergebnisse berechnen, dass nach einem halben Tag die Lösungen zu ca. 80 % aktiv sind, was für gute Kupplungen ausreichend ist. Dies bedeutet, dass in einem automatisierten Prozess die Lösungen morgens und abends erneuert werden können, und die Lösungen dann stabil genug sind, um 12 Stunden lang für das Kontaktieren verwendet werden zu können. Die grundsätzliche Eignung zur Automatisierung ist im Hinblick auf hohe Synthesekapazität ein extrem wichtiger Aspekt der erfindungsgemäßen Verfahren.

**Verwendete Abkürzungen**

| | |
|---|---|
| Abb. | Abbildung |
| AAV | Allgemeine Arbeitsvorschrift |
| Ac | Acetyl |
| DCM | Dichlormethan |
| DIC | Diisopropylcarbodiimid |
| DMF | *N,N*-Dimethylformamid |
| DMSO | Dimethylsulfoxid |
| Fmoc | 9-Fluorenylmethyloxycarbonyl |
| h | Stunde(n) |
| HOBt | 1-Hydroxybenzotriazol |
| HPLC | high-pressure liquid chromatography |
| Me | Methyl |
| min | Minute(n) |
| NMI | *N*-Methylimidazol |
| NMP | *N*-Methylpyrrolidon |
| NMR | nuclear magnetic resonance |
| Pfp | Pentafluorphenyl |
| Pip | Piperidin |
| TFA | Trifluoressigsäure |

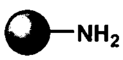 aminoderivatisiertes Syntheseharz

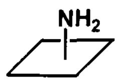 aminoderivatisierte planare Oberfläche

Für proteinogenen Aminosäuren wurde der Ein- und Drei-Buchstaben-Code verwendet:

| 1-Buchstaben-Code | 3-Buchstaben-Code | Aminosäure | 1-Buchstaben-Code | 3-Buchstaben-Code | Aminosäure |
|---|---|---|---|---|---|
| A | Ala | Alanin | M | Met | Methionin |
| C | Cys | Cystein | N | Asn | Asparagin |
| D | Asp | Asparaginsäure | P | Pro | Prolin |
| E | Glu | Glutaminsäure | Q | Gln | Glutamin |
| F | Phe | Phenylalanin | R | Arg | Arginin |
| G | Gly | Glycin | S | Ser | Serin |
| H | His | Histidin | T | Thr | Threonin |
| I | Ile | Isoleucin | V | Val | Valin |
| K | Lys | Lysin | W | Trp | Tryptophan |
| L | Leu | Leucin | Y | Tyr | Tyrosin |

Die in der vorangehenden Beschreibung, den Ansprüchen und den Zeichnungen offenbarten Merkmale der Erfindung können sowohl einzeln als auch in beliebiger Kombination zur Verwirklichung der Erfindung in ihren verschiedenen Ausführungsformen wesentlich sein.

## Patentansprüche

1. Verfahren zur Ausbildung einer chemischen Bindung zwischen einer ersten, auf einer insbesondere planaren Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung und einem Kupplungsreagenz und optional einem Zuschlagstoff sowie optional mit einer Base kontaktiert und umgesetzt wird, **dadurch gekennzeichnet, dass**

   die Schritte
   - Kontaktieren der Oberfläche mit dem Kupplungsreagenz und
   - Kontaktieren der Oberfläche mit der zu immobilisierenden Verbindung

   getrennt, insbesondere zeitlich voneinander getrennt erfolgen.

2. Verfahren nach Anspruch 1, **dadurch gekennzeichnet, dass** dem Kupplungsreagenz und/oder der Verbindung mindestens ein Zuschlagstoff zugesetzt wird.

3. Verfahren nach Anspruch 1 oder 2, **dadurch gekennzeichnet, dass** der Verbindung und/oder dem Kupplungsreagenz die Base zugesetzt ist.

4. Verfahren nach Anspruch 1 oder 2, **dadurch gekennzeichnet, dass** die Base dargestellt wird durch die immobilisierte funktionelle Gruppe.

5. Verfahren nach einem der vorangehenden Ansprüche, weiter umfassend den Schritt
   Kontaktieren der Oberfläche mit einem Zuschlagstoff, wobei dieser Schritt getrennt, insbesondere zeitlich getrennt von den Schritten Kontaktieren der Oberfläche mit dem Kopplungsreagenz und Kontaktieren der Oberfläche mit der zu immobilisierenden Verbindung erfolgt.

6. Verfahren nach einem der vorangehenden Ansprüche **dadurch gekennzeichnet, dass** nach zumindest einem der Schritte

   - Kontaktieren der Oberfläche mit dem Kupplungsreagenz,
   - Kontaktieren der Oberfläche mit der zu immobilisierenden Verbindung,
   - Kontaktieren der Oberfläche mit dem Zuschlagstoff, oder
   - Kontaktieren der Oberfläche mit der Base

   die so behandelte Oberfläche getrocknet wird.

7. Verfahren nach einem der Ansprüche 1 bis 6, **dadurch gekennzeichnet, dass** die Ausbildung der chemischen Bindung bei Anwesenheit sowohl der Verbindung als auch des Kupplungsreagenz und der Base sowie optional des Zuschlagstoffes erfolgt.

8. Verfahren nach einem der Ansprüche 1 bis 7, **dadurch gekennzeichnet, dass** nach der Ausbildung der chemischen Bindung die Oberfläche mit einem Lösungsmittel gewaschen wird.

9. Verfahren zur Ausbildung einer chemischen Bindung zwischen einer ersten, auf einer insbesondere planaren Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung, einem Kupplungsreagenz und optional einem Zuschlagstoff kontaktiert und umgesetzt wird, **dadurch gekennzeichnet, dass** die folgenden Schritte zeitlich getrennt voneinander durchgeführt werden:

   a) Kontaktieren der Oberfläche mit dem Kupplungsreagenz; und
   b) Kontaktieren der Oberfläche mit der zu immobilisierenden Verbindung,

   wobei die immobilisierte funktionelle Gruppe eine Base ist.

10. Verfahren zur Ausbildung einer chemischen Bindung zwischen einer ersten, auf einer insbesondere planaren Oberfläche immobilisierten funktionellen Gruppe und einer zweiten funktionellen Gruppe, wobei die zweite funktionelle

Gruppe Teil einer zu immobilisierenden Verbindung ist, wobei die Oberfläche bereitgestellt wird und mit der darauf zu immobilisierenden Verbindung, einem Kupplungsreagenz und einer Base und optional einem Zuschlagstoff kontaktiert und umgesetzt wird, **dadurch gekennzeichnet, dass** die folgenden Schritte zeitlich getrennt voneinander durchgeführt werden:

a) Kontaktieren der Oberfläche mit dem Kupplungsreagenz; und
b) Kontaktieren der Oberfläche mit der zu immobilisierenden Verbindung und der Base.

**11.** Verfahren zur Ausbildung einer chemischen Bindung zwischen einer ersten, auf einer insbesondere planaren Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung, einem Kupplungsreagenz und einer Base sowie optional einem Zuschlagstoff kontaktiert und umgesetzt wird, **dadurch gekennzeichnet, dass** die folgenden Schritte zeitlich getrennt voneinander durchgeführt werden:

a) Kontaktieren der Oberfläche mit dem Kupplungsreagenz und der zu immobilisierenden Verbindung und
b) Kontaktieren der Oberfläche mit der Base.

**12.** Verfahren nach einem der Ansprüche 9 bis 11, **dadurch gekennzeichnet, dass** das Kontaktieren der Oberfläche an einer definierten Stelle der Oberfläche erfolgt.

**13.** Verfahren nach einem der Ansprüche 9 bis 12, **dadurch gekennzeichnet, dass** das Kontaktieren gemäß Schritt a) und Schritt b) an der im Wesentlichen gleichen Stelle erfolgt.

**14.** Verfahren nach einem der Ansprüche 9 bis 12, **dadurch gekennzeichnet, dass** das Kontaktieren gemäß Schritt a) auf einem größeren Teil der Oberfläche erfolgt als das Kontaktieren gemäß Schritt b).

**15.** Verfahren zur parallelen Ausbildung einer chemischen Bindung an einer Mehrzahl von Stellen auf einer Oberfläche, bevorzugterweise einer planaren Oberfläche, zwischen einer ersten, auf den Stellen der Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung, einem Kupplungsreagenz und einer Base und optional einem Zuschlagstoff kontaktiert und umgesetzt wird, **dadurch gekennzeichnet, dass** die folgenden Schritte zeitlich getrennt voneinander durchgeführt werden:

a) Kontaktieren der Oberfläche mit dem Kupplungsreagenz und der zu immobilisierenden Verbindung an mindestens einer definierten Stelle der Oberfläche,
b) Mindestens einmaliges Wiederholen von Schritt a), wobei bei der Wiederholung von Schritt a) das Kontaktieren an mindestens einer weiteren definierten Stelle der Oberfläche erfolgt, und
c) Kontaktieren der Oberfläche mit der Base, bevorzugterweise an den definierten Stellen der Oberfläche.

**16.** Verfahren zur parallelen Ausbildung einer chemischen Bindung an einer Mehrzahl von Stellen auf einer Oberfläche, bevorzugterweise einer planaren Oberfläche, zwischen einer ersten, auf den Stellen der Oberfläche immobilisierten funktionellen Gruppe und einer zweiten funktionellen Gruppe, wobei die zweite funktionelle Gruppe Teil einer zu immobilisierenden Verbindung ist, wobei die Oberfläche bereitgestellt wird und mit der darauf zu immobilisierenden Verbindung, einem Kupplungsreagenz und einer Base und optional einem Zuschlagstoff kontaktiert und umgesetzt wird, **dadurch gekennzeichnet, dass** die folgenden Schritte zeitlich getrennt voneinander durchgeführt werden:

a) Kontaktieren der Oberfläche mit dem Kupplungsreagenz und der zu immobilisierenden Verbindung an mindestens einer definierten Stelle der Oberfläche,
b) Kontaktieren der Oberfläche mit der Base, bevorzugterweise an der mindestens einen definierten Stelle der Oberfläche gemäß Schritt a);
c) Mindestens einmaliges Wiederholen von Schritt a) und Schritt b), wobei bei der Wiederholung von Schritt a) das Kontaktieren an einer weiteren definierten Stelle der Oberfläche erfolgt.

**17.** Verfahren nach Anspruch 15 oder 16, **dadurch gekennzeichnet, dass** das Kontaktieren der Oberfläche mit der Base an der im Wesentlichen gleichen Stelle erfolgt wie das Kontaktieren der Oberfläche mit dem Kupplungsreagenz und der zu immobilisierenden Verbindung.

**18.** Verfahren nach einem der Ansprüche 15 bis 17, **dadurch gekennzeichnet, dass** bei der Wiederholung von Schritt a) die zu immobilisierende Verbindung die gleiche oder eine andere zu immobilisierende Verbindung ist verglichen mit derjenigen des vorhergehenden Schrittes.

**19.** Verfahren nach einem der Ansprüche 9 bis 18, **dadurch gekennzeichnet, dass** dem Kupplungsreagenz und/oder der Verbindung mindestens ein Zuschlagstoff zugesetzt ist/wird.

**20.** Verfahren nach Anspruch 19, weiter umfassend den Schritt
Kontaktieren der Oberfläche mit einem Zuschlagstoff, wobei dieser Schritt getrennt, insbesondere zeitlich getrennt von dem Schritt Kontaktieren der Oberfläche mit dem Kupplungsreagenz und/oder dem Schritt Kontaktieren der Oberfläche mit dem Kupplungsreagenz und der zu immobilisierenden Verbindung erfolgt.

**21.** Verfahren nach einem der Ansprüche 9 bis 20 **dadurch gekennzeichnet, dass** nach zumindest einem der Schritte

- Kontaktieren der Oberfläche mit dem Kupplungsreagenz,
- Kontaktieren der Oberfläche mit dem Kupplungsreagenz und der zu immobilisierenden Verbindung,
- Kontaktieren der Oberfläche mit der Base,
- Kontaktieren der Oberfläche mit der zu immobilisierenden Verbindung und der Base oder
- Kontaktieren der Oberfläche mit einem Zuschlagstoff

die so behandelte Oberfläche getrocknet wird.

**22.** Verfahren nach einem der Ansprüche 9 bis 21, **dadurch gekennzeichnet, dass** die Ausbildung der chemischen Bindung bei Anwesenheit sowohl der Verbindung als auch des Kupplungsreagenz und optional des Zuschlagstoffes und optional der Base erfolgt.

**23.** Verfahren nach einem der Ansprüche 9 bis 22, **dadurch gekennzeichnet, dass** nach der Ausbildung der chemischen Bindung die Oberfläche mit einem Lösungsmittel gewaschen wird.

**24.** Verfahren nach einem der Ansprüche 9 bis 23, **dadurch gekennzeichnet, dass** das Kupplungsreagenz, der Zuschlagstoff, die Base und/oder die Verbindung jeweils in einer Lösung vorliegt.

**25.** Verfahren nach einem der Ansprüche 1 bis 24, **dadurch gekennzeichnet, dass** es sich bei der ausgebildeten Bindung um eine Bindung handelt, die ausgewählt ist aus der Gruppe, die Carbonsäureamid-Bindung, Carbonsäureester-Bindung, Carbonsäureanhydrid-Bindung, Sulfonsäureamid-Bindung, Phosphorsäureester-Bindung, Phosphonsäureester-Bindung, Phosphorigsäureester-Bindung und Phosphorsäureamid-Bindung umfasst.

**26.** Verfahren nach einem der Ansprüche 1 bis 25, **dadurch gekennzeichnet, dass** das Kupplungsreagenz ausgewählt ist aus der Gruppe, die Carbodiimide, Aminium- und Phosphoniumsalze umfasst, bevorzugterweise ausgewählt ist aus der Gruppe, die HBTU, TBTU und PyBOP umfasst.

**27.** Verfahren nach einem der vorangehenden Ansprüche, **dadurch gekennzeichnet, dass** das Kupplungsreagenz in einem Lösungsmittel vorliegt und das Lösungsmittel bevorzugterweise ausgewählt ist aus der Gruppe, die N, N-Dimethylformamid, 1-Methyl-2-pyrrolidinon, N,N-Dimethylacetamid, Dimethylsulfoxid, Tetrahydrofuran, Dioxan, Acetonitril, Methanol, Ethylacetat, 2,2,2-Trifluorethanol, 1,1,1,3,3,3-Hexafluor-2-Propanol, 1,2-Dibromethan, Dichlormethan, Diethylether und Aceton umfasst.

**28.** Verfahren nach einem der Ansprüche 1 bis 27, **dadurch gekennzeichnet, dass** der Zuschlagstoff ausgewählt ist aus der Gruppe, die Benzotriazole, Benzotriazine, Phenole und organische und anorganische Salze umfasst, bevorzugterweise der Zuschlagstoff ausgewählt ist aus der Gruppe, die HOBt, HOAt, HOOBt, HOPfp, $NaClO_4$ und KSCN umfasst.

**29.** Verfahren nach einem der vorangehenden Ansprüche, **dadurch gekennzeichnet, dass** der Zuschlagstoff in einem Lösungsmittel vorliegt und das Lösungsmittel bevorzugterweise ausgewählt ist aus der Gruppe, die N,N-Dimethylformamid, 1-Methyl-2-pyrrolidinon, N,N-Dimethylacetamid, Dimethylsulfoxid, Tetrahydrofuran, Dioxan, Acetonitril, Methanol, Ethylacetat, 2,2,2-Trifluorethanol, 1,1,1,3,3,3-Hexafluor-2-Propanol, 1,2-Dibromethan, Dichlormethan,

Diethylether und Aceton umfasst.

**30.** Verfahren nach einem der Ansprüche 1 bis 29, **dadurch gekennzeichnet, dass** die zu immobilisierende Verbindung ausgewählt ist aus der Gruppe, die Carbonsäuren, Carbonsäureester, Sulfonsäuren, Phosphorsäureester, Phosphonsäureester, Phosphorigsäureester, Phosphoramidite, primäre Amine, sekundäre Amine und Alkohole umfasst, und die Verbindung bevorzugterweise ausgewählt ist aus der Gruppe, die Carbonsäuren, primäre Amine und sekundäre Amine umfasst.

**31.** Verfahren nach einem der vorangehenden Ansprüche, **dadurch gekennzeichnet, dass** die Verbindung in einem Lösungsmittel vorliegt und das Lösungsmittel bevorzugterweise ausgewählt ist aus der Gruppe, die N,N-Dimethylformamid, 1-Methyl-2-pyrrolidinon, N,N-Dimethylacetamid, Dimethylsulfoxid, Tetrahydrofuran, Dioxan, Acetonitril, Methanol, Ethylacetat, 2,2,2-Trifluorethanol, 1,1,1,3,3,3-Hexafluor-2-Propanol, 1,2-Dibromethan, Dichlormethan, Diethylether und Aceton umfasst.

**32.** Verfahren nach einem der Ansprüche 1 bis 31, **dadurch gekennzeichnet, dass** die Base ausgewählt ist aus der Gruppe, die tertiäre Amine, Pyridine, Imidazole und organische Salze umfasst, bevorzugterweise die Base ausgewählt ist aus der Gruppe, die DIPEA, Collidin, DMAP, NMI, NMM, Pyridin und KOBt umfasst.

**33.** Verfahren nach einem der Ansprüche 1 bis 32, **dadurch gekennzeichnet, dass** die Base in einem Lösungsmittel vorliegt, wobei bevorzugterweise das Lösungsmittel ausgewählt ist aus der Gruppe, die N,N-Dimethylformamid, 1-Methyl-2-pyrrolidinon, N,N-Dimethylacetamid, Dimethylsulfoxid, Tetrahydrofuran, Dioxan, Acetonitril, Methanol, Ethylacetat, 2,2,2-Trifluorethanol, 1,1,1,3,3,3-Hexafluor-2-Propanol, 1,2-Dibromethan, Dichlormethan, Diethylether und Aceton umfasst.

**34.** Verfahren nach einem der Ansprüche 1 bis 33, **dadurch gekennzeichnet, dass** die immobilisierte funktionelle Gruppe ausgewählt ist aus der Gruppe, die Carbonsäuren, Carbonsäureester, Sulfonsäure, Phosphorsäureester, Phosphonsäureester, Phosphorigsäureester, Phosphoramidite, primäre Amine, sekundäre Amine und Alkohole umfasst und die bevorzugterweise die immobilisierte funktionelle Gruppe ausgewählt ist aus der Gruppe, die Carbonsäuren, primäre Amine und sekundäre Amine umfasst.

**35.** Verfahren nach einem der Ansprüche 1 bis 34, **dadurch gekennzeichnet, dass** das Kontaktieren mittels Kontaktieren mit einer Kapillare erfolgt.

**36.** Verfahren nach einem der Ansprüche 1 bis 35, **dadurch gekennzeichnet, dass** das Kontaktieren durch Baden der Oberfläche in einer der Lösungen erfolgt.

**37.** Verfahren nach einem der Ansprüche 1 bis 36, **dadurch gekennzeichnet, dass** das Kontaktieren durch Aufsprühen, bevorzugterweise einer das jeweilige Reagenz enthaltenden Lösung erfolgt.

**38.** Verfahren nach einem der Ansprüche 1 bis 37, **dadurch gekennzeichnet, dass** das Kontaktieren durch Aufstempeln erfolgt.

**39.** Oberfläche mit einer chemischen Bindung zwischen einer ersten funktionellen Gruppe und einer zweiten funktionellen Gruppe, wobei die zweite funktionelle Gruppe Teil einer auf der Oberfläche immobilisierten Verbindung ist, herstellbar durch ein Verfahren nach einem der Ansprüche 1 bis 38.

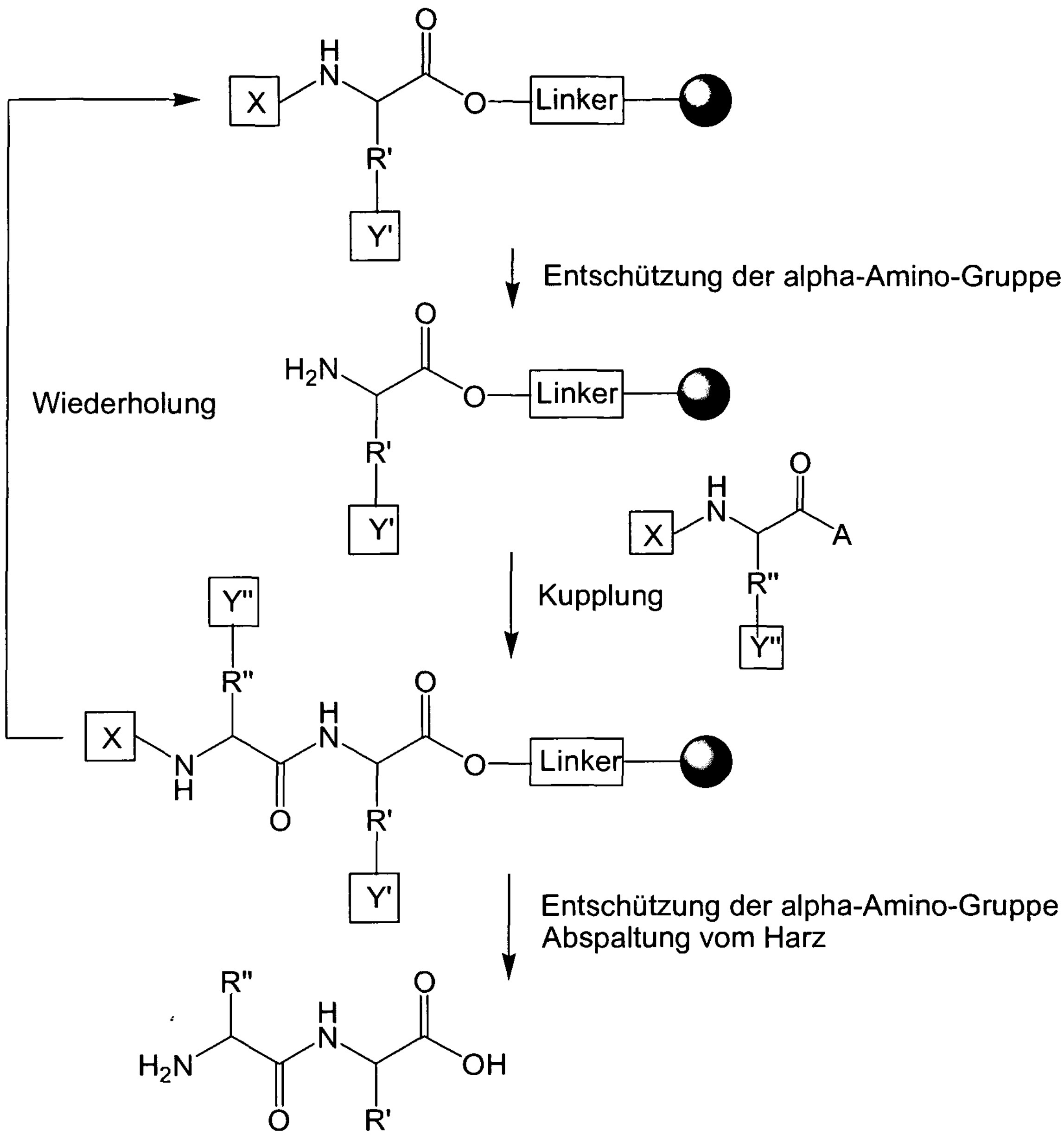
Entschützung der alpha-Amino-Gruppe
Wiederholung
Kupplung
Entschützung der alpha-Amino-Gruppe
Abspaltung vom Harz

Fig. 1

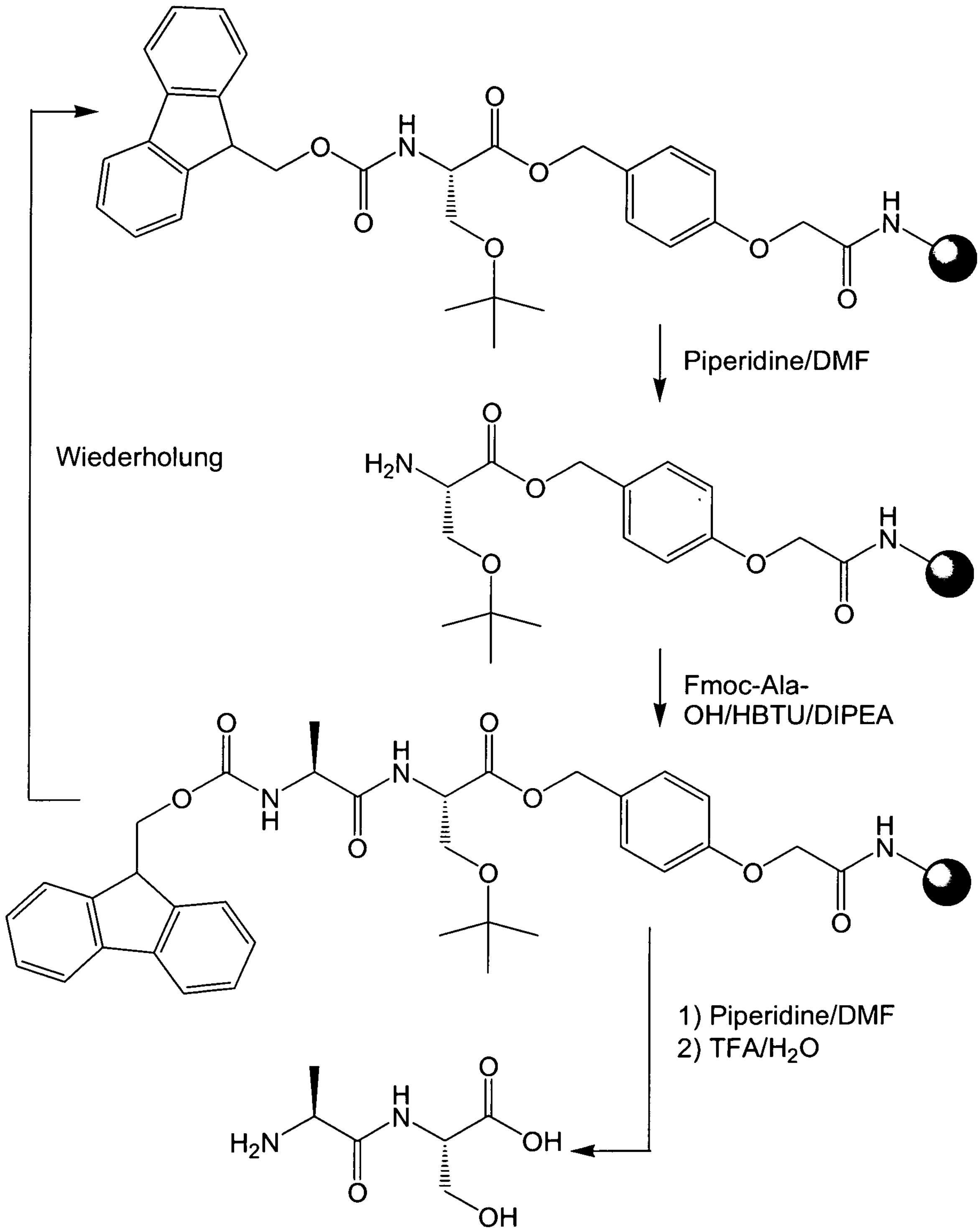
Piperidine/DMF
Wiederholung
Fmoc-Ala-
OH/HBTU/DIPEA
1) Piperidine/DMF
2) TFA/H2O

Fig. 2

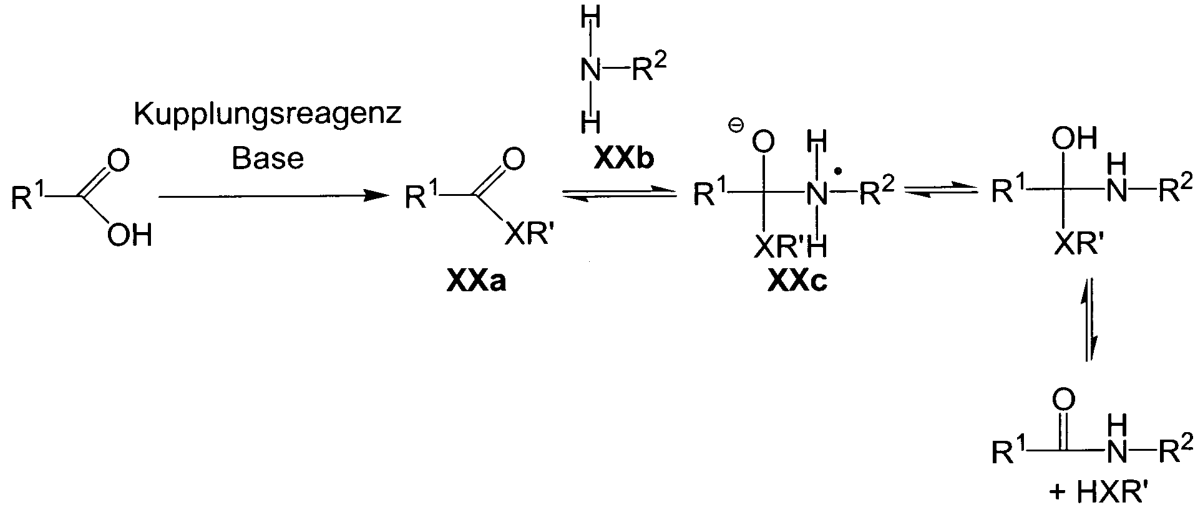


XXb

XXa

XXc

Fig. 3

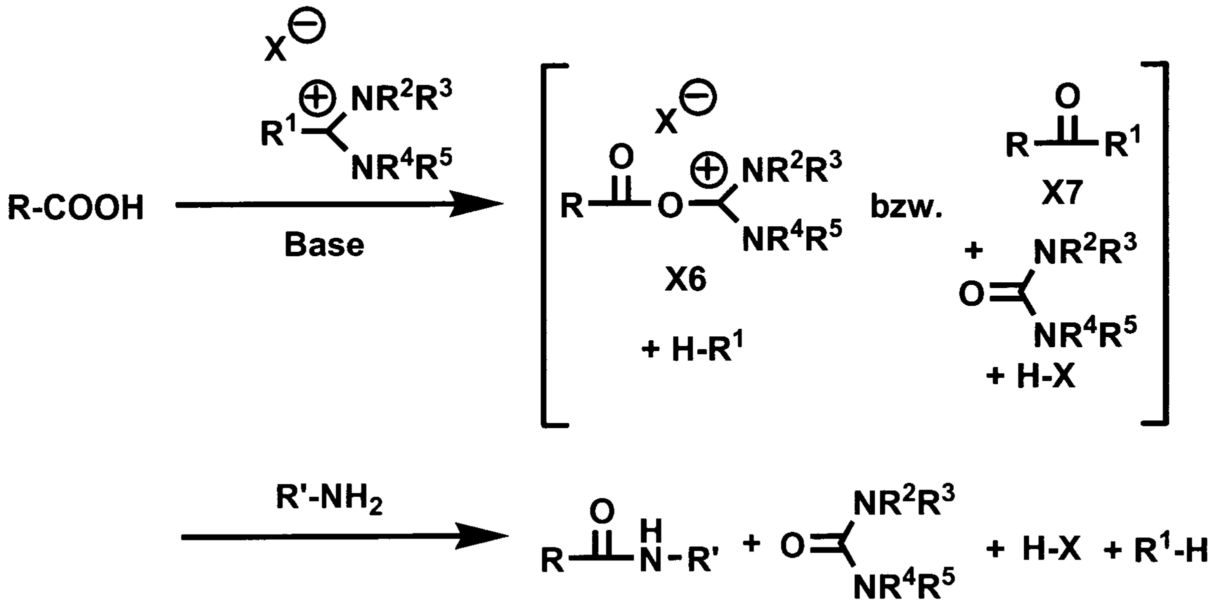


HBTU: $R^1$ = 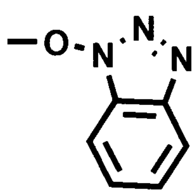 bzw. 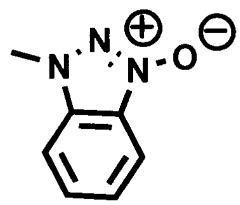

$X^- = PF_6^-$

Fig. 4

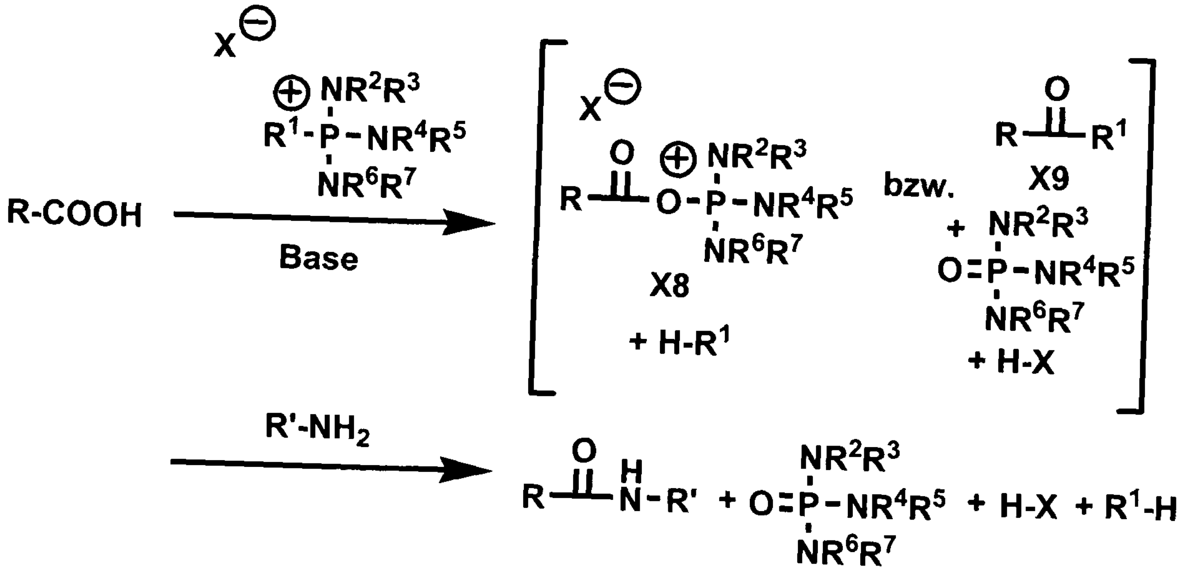


PyBOP: $R^1$ = 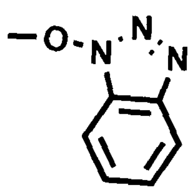 bzw. 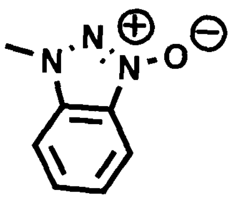

$X^- = PF_6^-$

Fig. 5

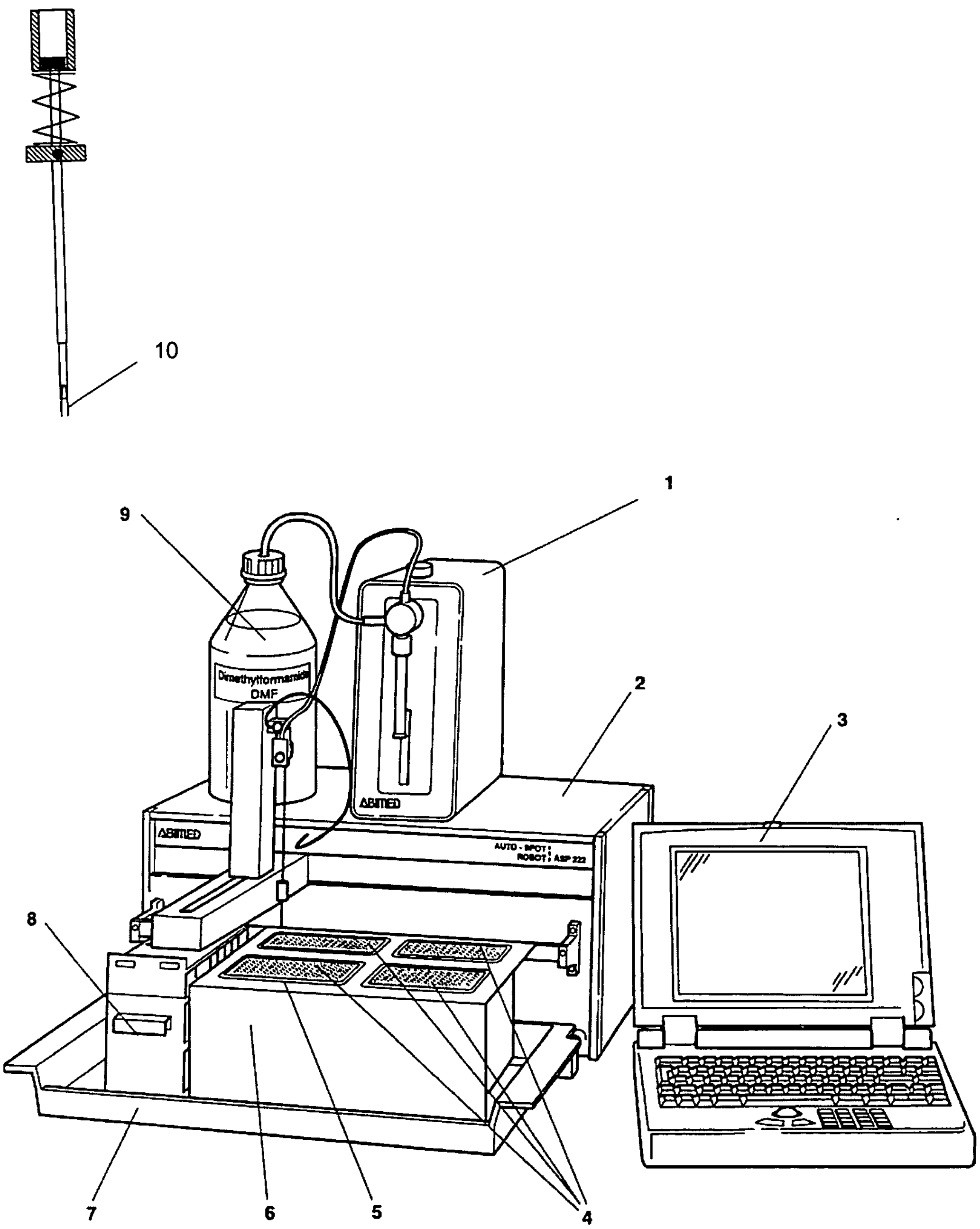


| | |
|---|---|
| 1 Dilutor 401 (oder 402) | 5 Rahmen für Membranen |
| 2 Laborroboter 222 | 6 Arbeitsbereich |
| 3 IBM-kompatibler PC | 7 Schale |
| 4 Synthesemembranen | 8 Ständer für Aminosäurederivate |
| | 9 Spüllösung |
| | 10 Nadel |

Fig. 6

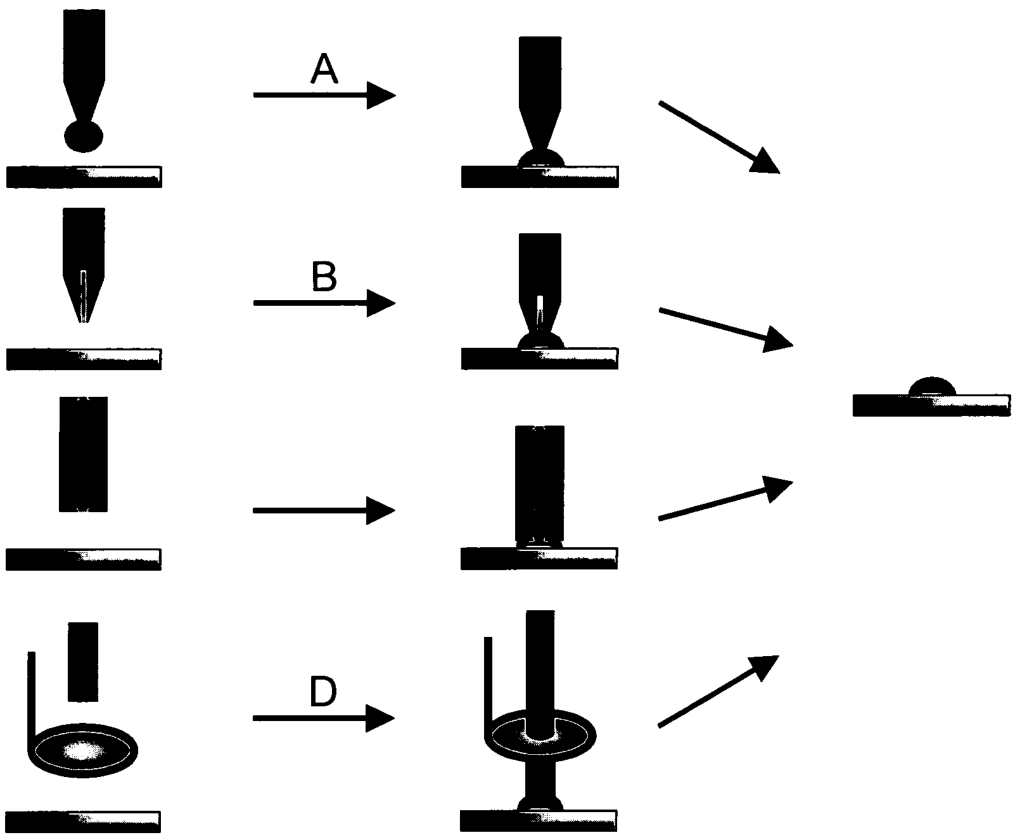


Fig. 7

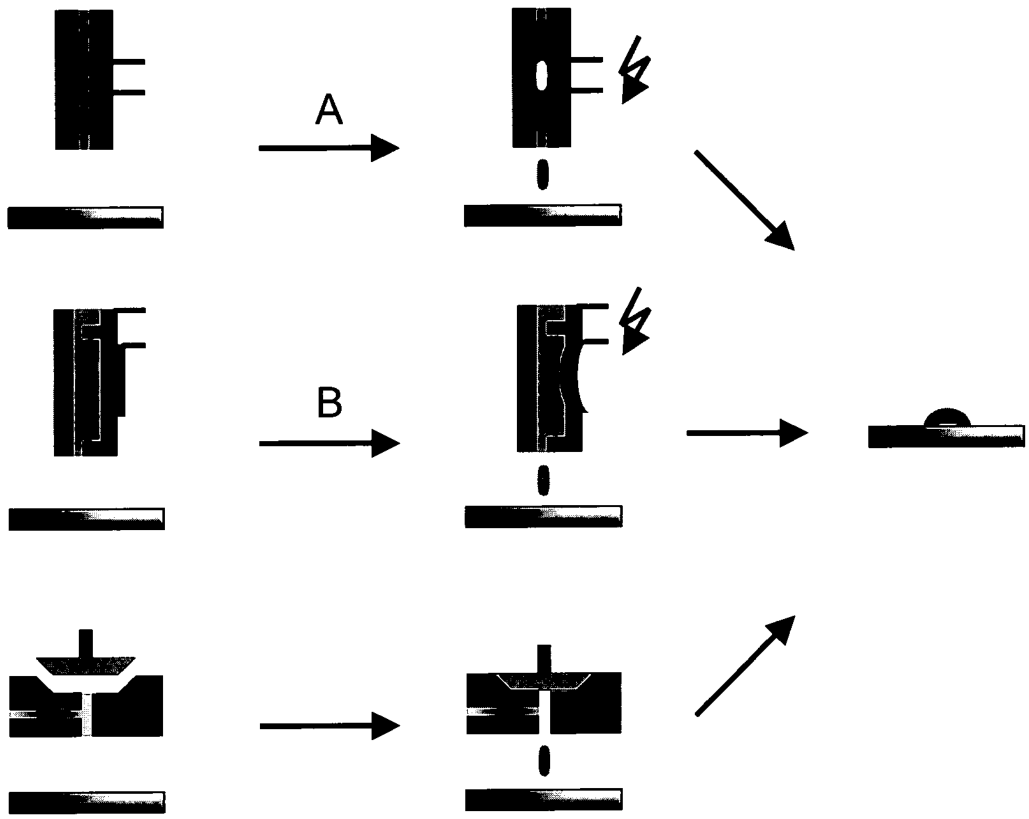


Fig. 8

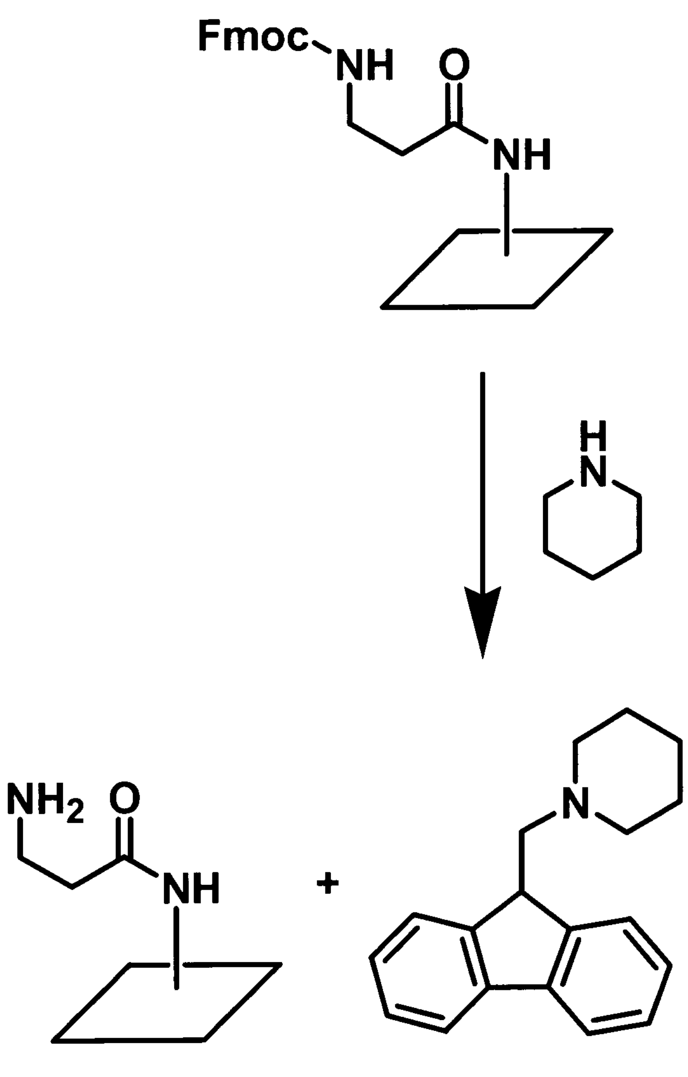

Fig. 9

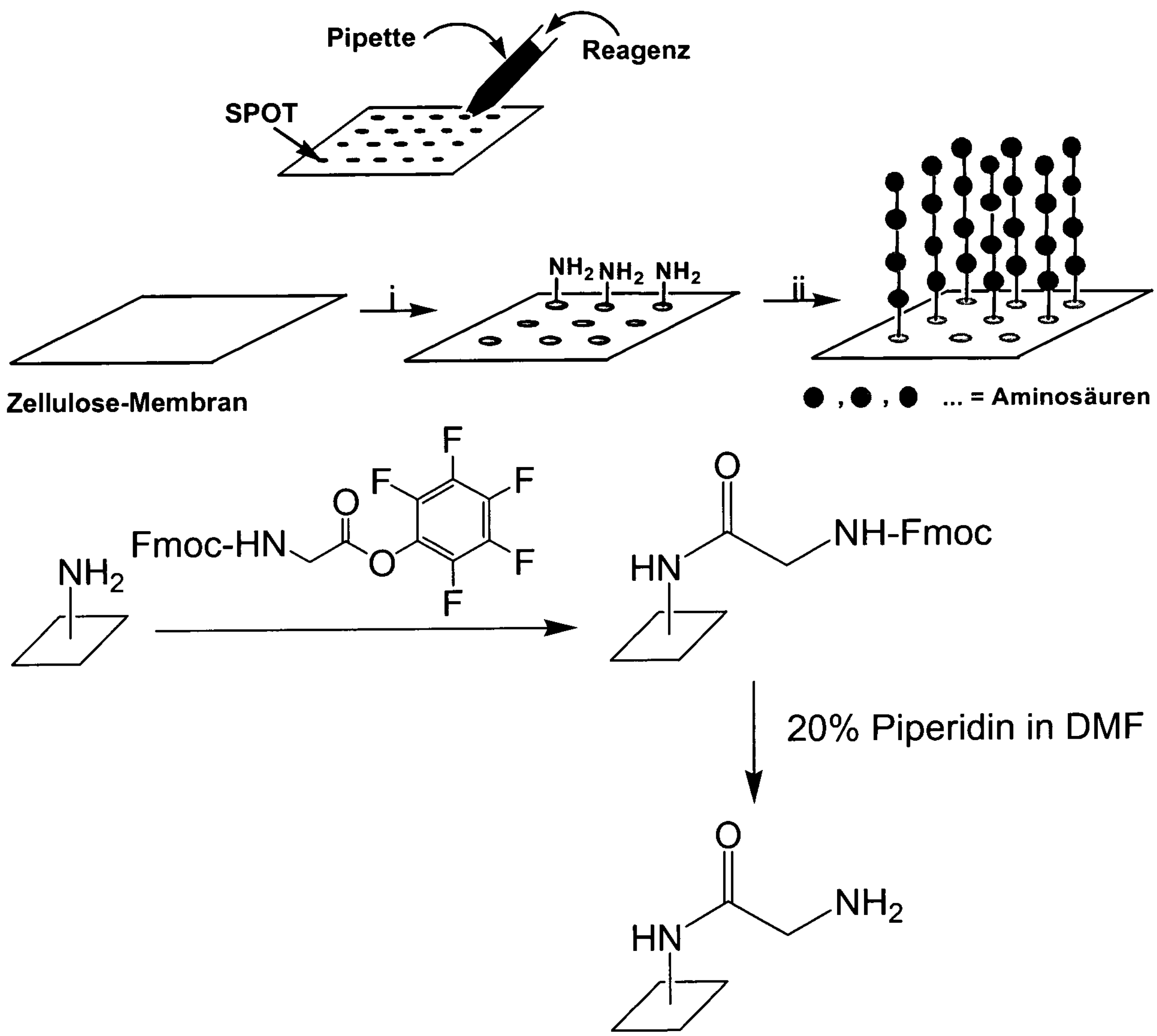
Pipette
Reagenz
SPOT
i
ii
Zellulose-Membran
... = Aminosäuren
20% Piperidin in DMF

Fig. 10

Fig. 11A: Ac-LQQRIRAEREKERG-OH

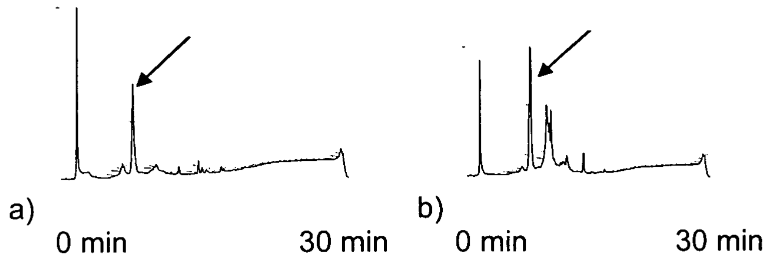


Fig. 11B: Ac-PTLVTTLTYGVQG-OH

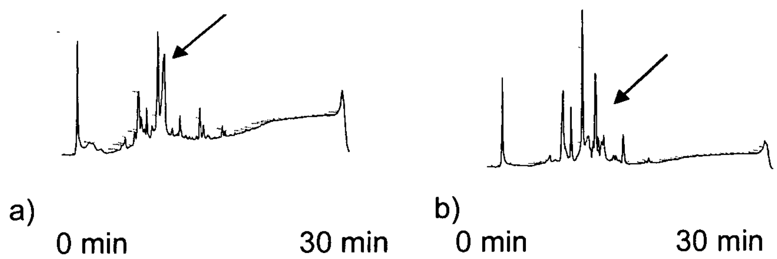


Fig. 11C: Ac-BVRRLRRLTAREAAG-OH

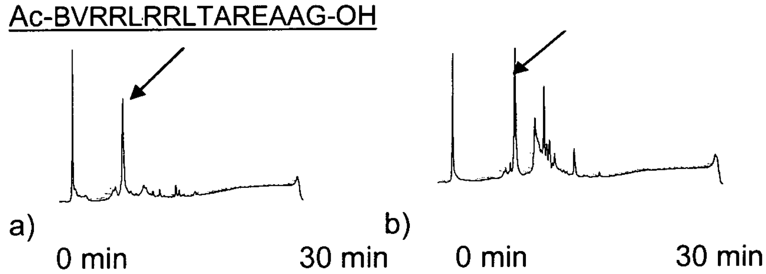


Fig. 11D: Ac-VQAAIDYING-OH

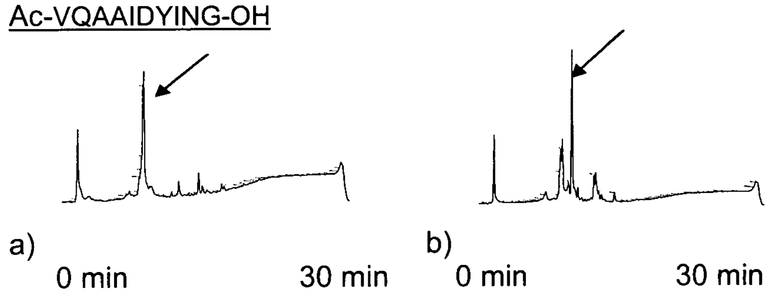


Fig. 11E: Ac-AIIAAAVKFG-OH

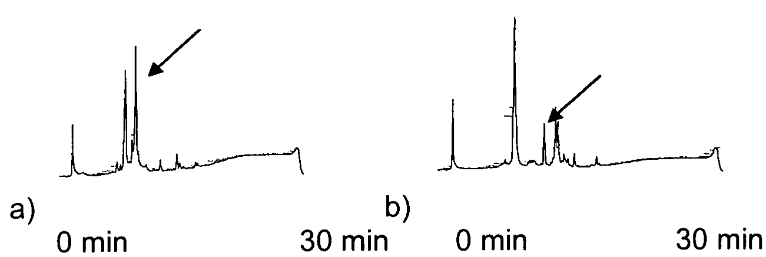


Fig. 11

Fig. 12A: H-IRFG-OH

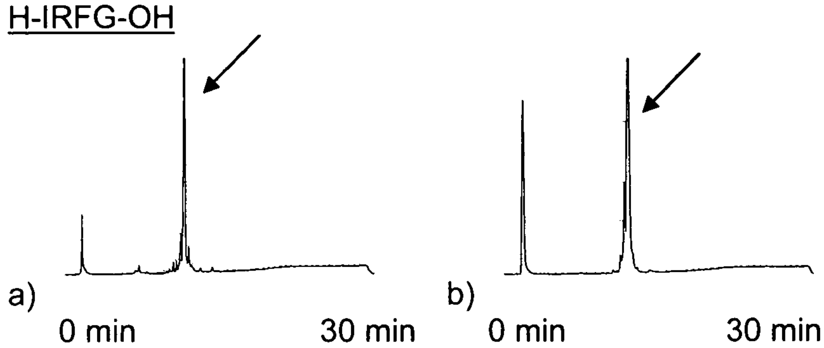


Fig. 12B: H-TTFG-OH

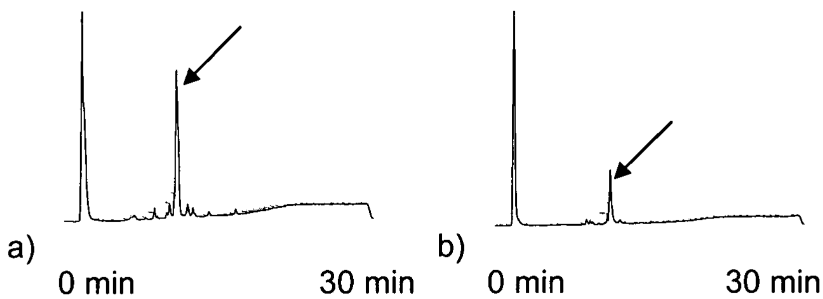


Fig. 12C: H-RTFG-OH

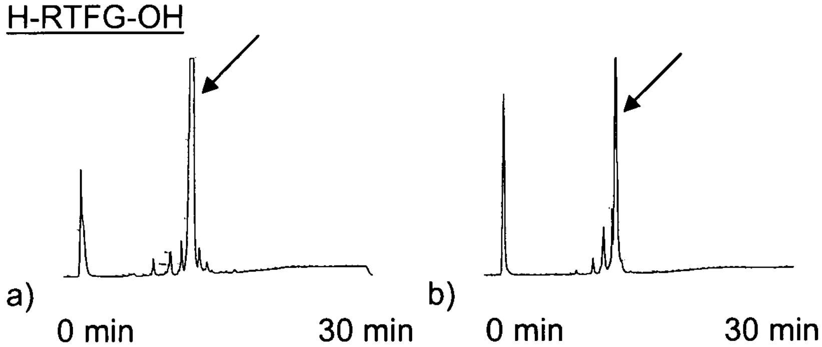


Fig. 12

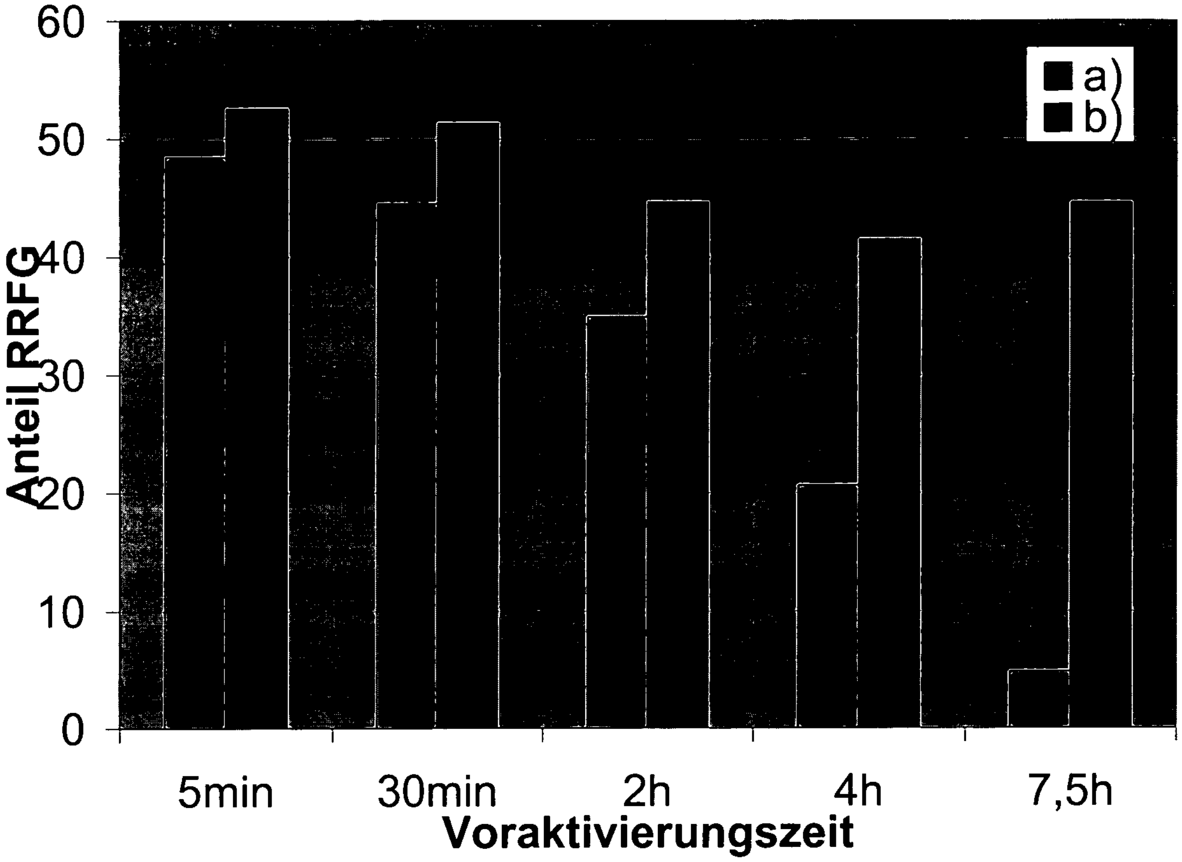
60
50
40
30
20
10
0
Anteil RRFG
a)
b)
5min
30min
2h
4h
7,5h
Voraktivierungszeit

Fig. 13

Fig. 14A:

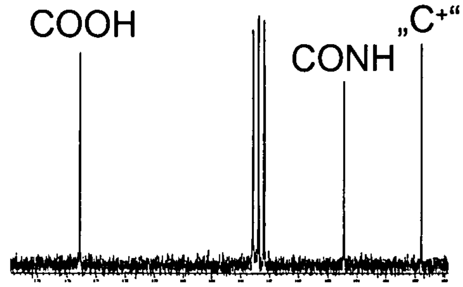


Fig. 14B:

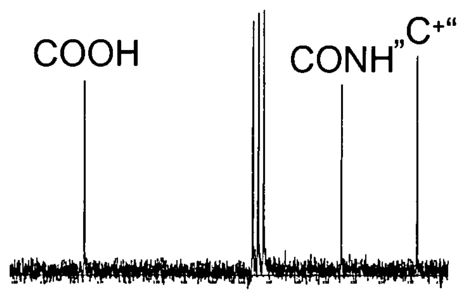


Fig. 14C:

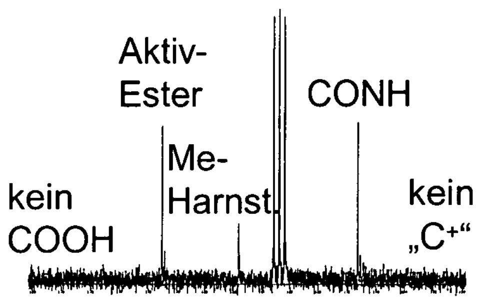


Fig. 14D:

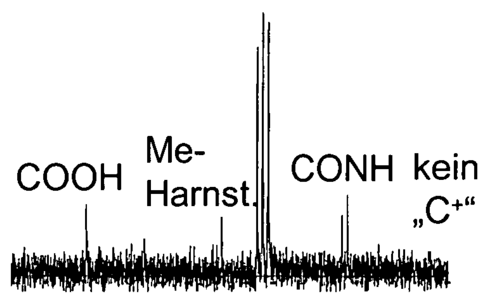


Fig. 14

Fig. 15A:

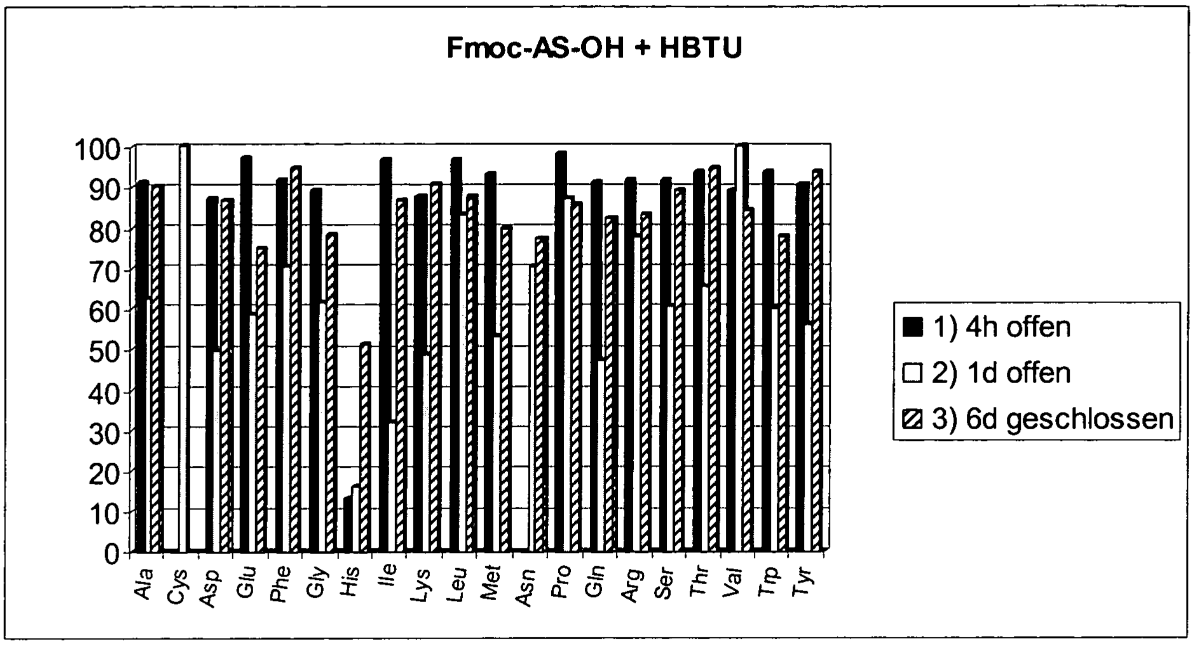


Fig. 15B:

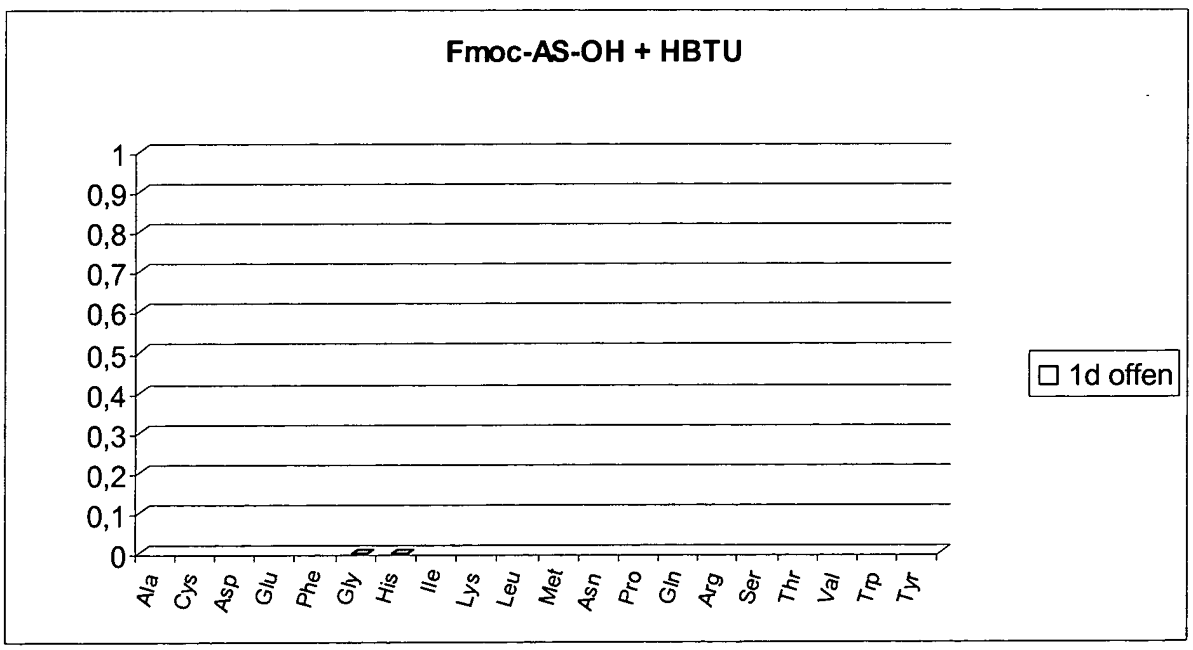


Fig. 15

Europäisches Patentamt

**EUROPÄISCHER TEILRECHERCHENBERICHT**

der nach Regel 45 des Europäischen Patentübereinkommens für das weitere Verfahren als europäischer Recherchenbericht gilt

Nummer der Anmeldung
EP 03 00 2404

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
| --- | --- | --- | --- |
| X | EP 0 134 041 A (AKAD WISSENSCHAFTEN DDR) 13. März 1985 (1985-03-13) | 1,3,5, 7-11, 21-25, 30-34,39 | C07K1/04<br>B01J19/00<br>C07B61/00 |
| Y | * Ansprüche 4-18; Beispiele * | 1-39 | |
| Y | WO 92 04366 A (BIOTECHNOLOG FORSCHUNG GMBH) 19. März 1992 (1992-03-19)<br>* das ganze Dokument * | 1-39 | |
| X | WO 94 15948 A (BOYD VICTORIA LEE ;YUAN PAU MIAU (US); APPLIED BIOSYSTEMS (US)) 21. Juli 1994 (1994-07-21) | 1,3,5, 7-11, 21-25, 30-34,39 | |
| Y | * Ansprüche 1,2,4-11,13-18,20-25; Beispiele 1-10 * | 1-39 | |
| X | US 5 874 569 A (ELSNER HENRIK ET AL) 23. Februar 1999 (1999-02-23)<br>* das ganze Dokument * | 1,3,5, 7-11, 21-25, 30-34,39 | RECHERCHIERTE SACHGEBIETE (Int.Cl.7)<br>C07K<br>B01J<br>C07B |

-/--

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
| --- | --- | --- |
| MÜNCHEN | 1. August 2003 | Döpfer, K-P |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C09)

Europäisches Patentamt

UNVOLLSTÄNDIGE RECHERCHE
ERGÄNZUNGSBLATT C

Nummer der Anmeldung
EP 03 00 2404

Unvollständig recherchierte Ansprüche:
1-39

Grund für die Beschränkung der Recherche:

Die geltenden Patentansprüche 1-39 beziehen sich auf eine unverhältnismäßig große Zahl möglicher Produkte bzw. Verfahren. In der Tat umfassen sie so viele Wahlmöglichkeiten, dass sie im Sinne von Art. 84 EPÜ in einem solche Maße unklar oder zu weitläufig gefasst erscheinen, als daß sie eine sinnvolle Recherche ermöglichten. Die Ansprüche sind derart weit gefaßt, daß nahezu jedes bekannte Verfahren zur Immobilisierung von Entitäten jeglicher Art auf aktivierte Festphasen eingeschlossen wird. Zudem werden allgemeine Begriffe (wie z.B. Zuschlagstoffe) verwendet, die den Anspruchsumfang als unklar erscheinen lassen. Das eigentliche Verfahren, nämlich die zeitlich versetzte Anwendung von Kupplungs- und Hilfsreagenzien zur Erhöhung der Ausbeute und Reinheit festphasensynthetisierter Peptide und anderer Biomolekule (z.B. Nukleinsäuren) wird durch die Formulierung der Ansprüche nicht klar zum Ausdruck gebracht. Die Recherche wurde daher auf die Immobilisierung von Aminosäuren oder Peptiden auf aktivierte Träger beschränkt, wobei die eigentliche Erfindung, wie oben erwähnt, mit recherchiert wurde. Die Vielzahl der anderen möglichen Kombinationen wurde nicht recherchiert.

Europäisches Patentamt

# EUROPÄISCHER TEILRECHERCHENBERICHT

Nummer der Anmeldung
EP 03 00 2404

| EINSCHLÄGIGE DOKUMENTE | | | KLASSIFIKATION DER ANMELDUNG (Int.Cl.7) |
|---|---|---|---|
| Kategorie | Kennzeichnung des Dokuments mit Angabe, soweit erforderlich der maßgeblichen Teile | Betrifft Anspruch | |
| A | WO 02 053606 A (KATTI SETURAM BANDHACHARY ;HAQ WAHAJUL (IN); COUNCIL SCIENT IND RE) 11. Juli 2002 (2002-07-11)<br>* das ganze Dokument *<br>--- | 1-39 | |
| A | US 6 277 957 B1 (HUDSON DEREK ET AL) 21. August 2001 (2001-08-21)<br>* das ganze Dokument *<br>----- | 1-39 | |
| | | | RECHERCHIERTE SACHGEBIETE (Int.Cl.7) |

EPO FORM 1503 03.82 (P04C12)

## ANHANG ZUM EUROPÄISCHEN RECHERCHENBERICHT ÜBER DIE EUROPÄISCHE PATENTANMELDUNG NR. EP 03 00 2404

In diesem Anhang sind die Mitglieder der Patentfamilien der im obengenannten europäischen Recherchenbericht angeführten *Patentdokumente angegeben.*
Die Angaben über die Familienmitglieder entsprechen dem Stand der Datei des Europäischen Patentamts am
Diese Angaben dienen nur zur Unterrichtung und erfolgen ohne Gewähr.

01-08-2003

| Im Recherchenbericht angeführtes Patentdokument | Datum der Veröffentlichung | Mitglied(er) der Patentfamilie | Datum der Veröffentlichung |
|---|---|---|---|
| EP 0134041 A | 13-03-1985 | DD 219481 A1 | 06-03-1985 |
| | | DD 217215 A1 | 09-01-1985 |
| | | DD 218886 A1 | 20-02-1985 |
| | | DD 220969 A1 | 10-04-1985 |
| | | DD 219490 A1 | 06-03-1985 |
| | | AT 33386 T | 15-04-1988 |
| | | DE 3470309 D1 | 11-05-1988 |
| | | EP 0134041 A1 | 13-03-1985 |
| | | HU 38615 A2 | 30-06-1986 |
| | | HU 191715 B | 30-03-1987 |
| | | JP 6122672 A | 06-05-1994 |
| | | JP 60072862 A | 24-04-1985 |
| | | US 4714768 A | 22-12-1987 |
| | | YU 144584 A1 | 30-06-1987 |
| | | HU 40410 A2 | 28-12-1986 |
| | | HU 40139 A2 | 28-11-1986 |
| WO 9204366 A | 19-03-1992 | DE 4027675 A1 | 12-03-1992 |
| | | AU 702234 B2 | 18-02-1999 |
| | | AU 4206396 A | 26-04-1996 |
| | | AU 8390391 A | 30-03-1992 |
| | | CA 2090485 A1 | 01-03-1992 |
| | | DE 59109168 D1 | 23-12-1999 |
| | | WO 9204366 A1 | 19-03-1992 |
| | | EP 0651762 A1 | 10-05-1995 |
| | | JP 6500539 T | 20-01-1994 |
| | | KR 203961 B1 | 15-06-1999 |
| | | NO 930613 A | 19-04-1993 |
| | | US 5830637 A | 03-11-1998 |
| | | US 6040423 A | 21-03-2000 |
| WO 9415948 A | 21-07-1994 | US 5602207 A | 11-02-1997 |
| | | AU 6083794 A | 15-08-1994 |
| | | EP 0677058 A1 | 18-10-1995 |
| | | JP 8509461 T | 08-10-1996 |
| | | WO 9415948 A1 | 21-07-1994 |
| | | US 5717075 A | 10-02-1998 |
| US 5874569 A | 23-02-1999 | AU 4698693 A | 03-03-1994 |
| | | CA 2142011 A1 | 17-02-1994 |
| | | WO 9403530 A1 | 17-02-1994 |
| | | EP 0654061 A1 | 24-05-1995 |
| | | JP 8503066 T | 02-04-1996 |
| | | US 6262256 B1 | 17-07-2001 |
| WO 02053606 A | 11-07-2002 | US 2002147297 A1 | 10-10-2002 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR. EP 03 00 2404

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-08-2003

| Im Recherchenbericht angeführtes Patentdokument | Datum der Veröffentlichung | Mitglied(er) der Patentfamilie | Datum der Veröffentlichung |
|---|---|---|---|
| WO 02053606 A | | WO 02053606 A1 | 11-07-2002 |
| | | EP 1263800 A1 | 11-12-2002 |
| US 6277957 B1 | 21-08-2001 | KEINE | |

EPO FORM P0461

Für nähere Einzelheiten zu diesem Anhang : siehe Amtsblatt des Europäischen Patentamts, Nr.12/82